# EUROPEAN PATENT APPLICATION

(11) **EP 4 834 903 A1**
(43) Date of publication of application: **04.11.2026**
(21) Application number: 25173326.7
(22) Date of filing: 29.04.2025
(51) Int. Cl.: C07D 471/10, C07D 498/10, A61P 37/00, A61P 37/06, A61K 31/438

(54) **SPIROCYCLIC PIPERIDINE ANALOGUES AS CCR8 AGONISTS**

(71) Applicant: Katholieke Universiteit Leuven, 3000 Leuven (BE)
(72) Inventor: Dehaen, Wim, 3000 Leuven (BE); Schols, Dominique, 3000 Leuven (BE)

(57) **Abstract**

The invention relates to Spirocyclic piperidine analogues and their used in CCR8 mediated disease such as graft-versus-host disease (GVHD), multiple sclerosis, inflammatory bowel disease, Crohn's disease, rheumatoid arthritis, psoriasis and diabetes.

## Description

### Introduction

The human CC chemokine receptor 8, discovered in 1997, is a cell surface receptor that belongs to the G protein-coupled receptor (GPCR) family. The endogenous ligands for the chemokine receptors are called chemokines, which is shorthand for chemoattractant cyto*kine,* a family of small proteins that directs the trafficking of immune cells during normal immune function, but is also involved in many pathological disorders. Various chemokines, such as CCL1, CCL8, CCL16, CCL18, are known to interact with CCR8. However, CCL1 exclusively binds CCR8, and hence the CCL1-CCR8 axis is the most extensively studied one (J. Exp. Med. 2013 Sep 23;210(10):1889-98).

CCR8 is highly expressed in the thymus, and the spleen, is also abundant in some NK and T cell lines and in the subset of T-helper-2 (Th2) (J. Immunol. 1998 Jul 15;161(2):547-51). CCR8 expression is upregulated on Th2 cells upon activation. Activated Th2 cells produce the cytokines IL-4, IL-5, and IL-13, which are important mediators of inflammation and airway hyper-reactivity in bronchial asthma, and CCR8 deficiency has been shown to ameliorate lung inflammation and airway function in the mouse.

More recently, it has been demonstrated that CCR8 is also expressed in tumor-resident regulatory T (Treg) cells, but is poorly expressed in tumor-infiltrating effector T-cells or peripheral Tregs (Immunity 2016 Nov 15;45(5):1122-1134). Treg cells promote cancer progression by suppressing antitumor immune responses of cytotoxic T-lymphocytes within the tumor microenvironment. Since Treg cells are endowed with immunosuppressive properties, they are also crucial in the suppression of autoimmunity.

Overall, these data indicate that CCR8 shows promise as drug target for the treatment of cancer, inflammatory disorders and autoimmune diseases.

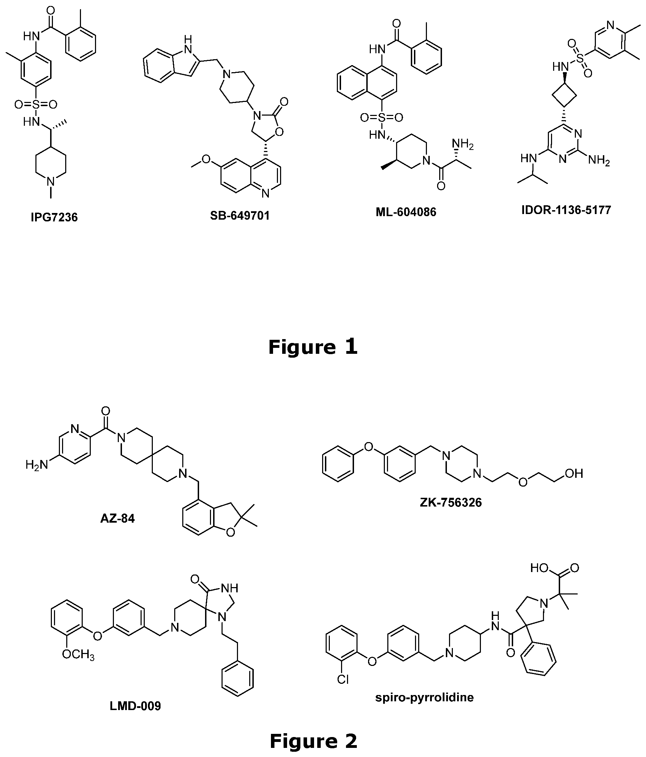

Various anti-human CCR8 antibodies have been developed that show promising activity against a variety of solid cancers. These antibodies induce potent antibody-dependent cellular cytotoxicity (ADCC) and deplete Tregs within the tumor microenvironment leading to tumor growth inhibition. Examples include RO7502175 (Br. J. Pharmacol. 2024 Jul;181(13):2033-2052), BAY 3375968 (Clin. Exp. Med. 2024 Jun 10;24(1):122) and S-531011 (Mol. Cancer Ther. 2023 Jul 7;22(9):1063-1072). IPG7236 is an example of a potent and selective small molecule CCR8 antagonist (J. Med. Chem. 2023 Apr 13;66(7):4548-4564) (Fig. 1). It shows anti-tumor efficacy when evaluated in a mouse preclinical breast cancer xenograft model and is currently being evaluated in phase II clinical trials. *N*-substituted *N*-substituted-5-aryl-oxazolidinones (Bioorg. Med. Chem. Lett. 2007 Mar 15;17(6):1722-5) have also been studied because of their CCR8 antagonistic properties. SB-649701 (Fig. 1) is a potent CCR8 antagonist, lacks activity against other GPCRs (including other chemokine receptors) and does not inhibit CYP isoenzymes or the hERG ionchannel. *In vivo* pharmacokinetic experiments in mice revealed moderate clearance and modest oral bioavailability (18%).
ML-604086 (Fig. 1) is an optimized CCR8 antagonist from the naphthalene sulfonamide series. It was evaluated in a primate model of asthma (Thorax 2013 Jun;68(6):506-12). Although more than >98% target inhibition by ML604086 on peripheral T cells was achieved, this did not result in improvements of lung inflammation and lung function, suggesting a dispensable role of CCR8 in airway inflammation. Very recently, the pyrimidine analogue IDOR-1136-5177 was disclosed that showed low nM CCR8 antagonism and showed antitumoral effect in 3D patient-derived organoid models as an *in vitro* model of colon cancer (ChemMedChem 2025 Feb 23:e202400968).

CCR8 agonism has also been studied as therapeutic strategy. Targeting CCR8 through a stabilized CCL1-Ig fusion protein (CCL1-Ig) improved Treg proliferation and suppressed disease in an experimental autoimmune encephalomyelitis (EAE) model, which is a murine model of multiple sclerosis (Proc. Natl. Acad. Sci. USA 2017 Jun 6;114(23):6086-6091). Similarly, stimulation of the CCL1-CCR8 signaling axis protected the gut from acute intestinal damage in a mouse model of inflammatory bowel disease *(*Front. Immunol. 2021 Feb 5:11:609400).

Various small molecule CCR8 agonists have been developed as well (Figure 2). A series of diazaspiroundecanes was developed as CCR8 agonists. The most attractive congener is AZ-084, showing low nanomolar activity in various CCR8-related assays (Biochem. Pharmacol. 2012 Mar 15;83(6):778-87). In addition, it displays very good selectivity when evaluated against a panel of other chemokine receptors and ionchannels. Moreover, AZ-84 has an attractive *in vivo* pharmacokinetic profile. Phenoxybenzylpiperidine analogues belong to another chemotype that has been pursued for their CCR8 agonistic properties. The prototype is ZK-756326 that shows moderate activity as CCR8 agonist (Mol. Pharmacol. 2006, 69:309-316). Further SAR studies with a focus on structural variation of the ethoxyethanol side chain yielded various analogues with improved CCR8 agonistic activity. For example, the triazaspiro[4.5]decane analogue LMD-009 and the spiro-pyrrolidine analogue both showed low nM CCR8 agonism (Mol. Pharmacol. 2007 Aug;72(2):327-40).

### SUMMARY OF THE INVENTION

The present invention relates to novel spirocyclic compounds as agonists for the human CCR8 receptor and their use as agents for the treatment of autoimmune diseases. It is based on the unexpected finding that certain spirocyclic compounds show unexpected biological properties, in particular having significant human CCR8 agonistic activity. The present invention further provides the use of these triazole analogues as a medicine for the treatment of autoimmune diseases. The auto-immune disorders to be treated by compounds from this invention include both systemic auto-immune diseases such as but not limited to lupus erythematosus, psoriasis, vasculitis, polymyositis, scleroderma, multiple sclerosis, ankylosing spondilytis, rheumatoid arthritis and Sjögren syndrome; auto-immune endocrine disorders such as thyroiditis; and organ-specific auto-immune diseases such as but not limited to Addison disease, hemolytic or pernicious anemia, Goodpasture syndrome, Graves disease, idiopathic thrombocytopenic purpura, insulin-dependent diabetes mellitus, juvenile diabetes, uveitis, Crohn's disease, ulcerative colitis, pemphigus, atopic dermatitis, autoimmune hepatitis, primary biliary cirrhosis, autoimmune pneumonitis, autoimmune carditis, myasthenia gravis, glomerulonephritis and spontaneous infertility. Transplant rejections to be prevented or treated by the pharmaceutical compositions or combined preparations of this invention include the rejection of transplanted or grafted organs or cells (both allografts and xenografts), such as but not limited to host-versus-graft reaction disease. Furthermore, autoimmune-like disorders, such as the graft-versus-host disease (GvHD), can be treated by compounds from this invention.

Numbered statements of the invention are:
1. A compound of formula **I:** 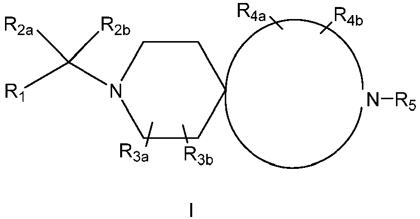 wherein R₁ is selected from: 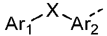 or Ar₃--;
   Ar₁ and Ar₂ are heteroaryl and aryl groups, wherein said heteroaryl or aryl groups are optionally substituted with one or more substituents selected from the group consisting of halogen, C1-7 alkyl, C2-7 alkenyl, C2-7 alkynyl, halo C1-7 alkyl, nitro, hydroxyl, sulfhydryl, amino, C1-7 alkoxy, C3-10 cycloalkoxy, aryloxy, arylalkyloxy, oxyheterocyclic, heterocyclic-substituted alkyloxy, thio C1-7 alkyl, thio C3-10 cycloalkyl, thioaryl, thio-heterocyclic, arylalkylthio, heterocyclic-substituted alkylthio, formyl, carbamoyl, thiocarbamoyl, ureido, thioureido, sulfonamido, hydroxylamino, alkoxy-amino, mercaptoamino, thioalkylamino, acylamino, thioacylamino, cyano, carboxylic acid or esters or thioesters or halides or anhydrides or amides thereof, thiocarboxylic acid or esters or thioesters or halides or anhydrides or amides thereof, alkylamino, cycloalkylamino, alkenylamino, cyclo-alkenylamino, alkynylamino, arylamino, arylalkylamino, hydroxyalkylamino, mercaptoalkylamino, heterocyclic amino;
   X is O, NH, S, or CH₂;
   Ar₃ is a heteroaryl or aryl group, wherein said heteroaryl or aryl group is optionally substituted with one or more substituents selected from the group consisting of halogen, C1-7 alkyl, C1-7 alkoxy, halo C1-7 alkyl, nitro, hydroxyl, amino, and
   Ar₃ is optionally fused with a saturated or partly unsaturated heterocyclic ring with at least one oxygen in said heterocyclic ring; wherein said heterocyclic ring is optionally substituted with one or more substituents selected from the group consisting of halogen, C1-7 alkyl or is optionally spirofused with a C3-10 cycloalkyl;
   R₂ₐ and R_{2b} are independently selected from H and C1-7 alkyl;
   R₃ₐ and R_{3b} are independently selected from the group consisting of hydrogen, C1-7 alkyl and a carbonyl group 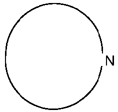 schematically represents a saturated or partly unsaturated heterocyclic ring with at least one nitrogen in said heterocyclic ring; optionally, this heterocyclic ring might also contain one or more oxygen atoms;
   R₄ₐ and R_{4b} are independently selected from the group consisting of hydrogen, C1-7 alkyl or a carbonyl group;
   R₅ is selected from the group consisting of H, C1-7 alkyl or (CR₇ₐR_{7b})ₙ-Ar₄;
      wherein n is 0-2;
      wherein R₇ₐ and R_{7b} are independently selected from H and C1-7 alkyl;
      wherein Ar₄ is a heteroaryl and aryl group, and wherein said heteroaryl or aryl
   groups are optionally substituted with one or more substituents selected from the group consisting of halogen, C1-7 alkyl, C3-10 cycloalkyl, halo C1-7 alkyl, nitro, hydroxyl, cyano, amino, C1-7 alkoxy, C3-10 cycloalkoxy, cyano, carboxylic acid or esters;
   and/or a pharmaceutical acceptable addition salt thereof and/or a stereoisomer thereof and/or a solvate thereof.
2. The compound according to statement 1, wherein Ar₁, Ar₂, Ar₃ and Ar₄ are each independently selected from the group consisting of an unsubstituted heteroaryl and an aryl group, wherein said aryl group is optionally substituted with one or more substituents selected from the group consisting of halogen and C1-7 alkoxy.
3. The compound according to statement 1 or 2, wherein X is O.
4. The compound according to any of statements 1 to 3, wherein R₂ₐ and R_{2b} are both H.
5. The compound according to any of statements 1 to 4, wherein R₄ₐ and R_{4b} are independently selected from the group consisting of hydrogen, a methyl and a carbonyl group.
6. The compound according to any of statements 1 to 4, wherein R₄ₐ is hydrogen and R_{4b} is a carbonyl group.
7. The compound according to any of statements 1 to 6, wherein 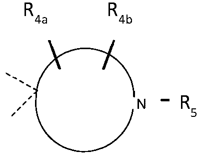 is preferentially 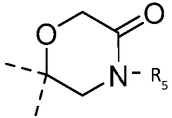
8. The compound according to any of statements 1 to 7, wherein R₅ is selected from (CR₇ₐR_{7b})ₙ--Ar₄, wherein preferentially n is 1 or 2, R₇ₐ is hydrogen and R_{7b} is methyl and in which Ar₄ is preferentially a phenyl group substituted with a halogen.
9. The compound according to any of statements 1 to 8, wherein R₅ is selected from (CR₇ₐR_{7b})ₙ--Ar₄, wherein n is 1, R₇ₐ is hydrogen and R_{7b} is methyl and in which Ar₄ is a phenyl group substituted with a halogen.
10. A compound according to any one of the statements 1 to 9, for use as a medicament.
11. A compound according to any one of the statements 1 to 9, for use in the treatment or prevention of a CCR8 mediated disease.
12. The compound for use in the treatment or prevention according to statement 11 wherein the CCR8 mediated disease is an autoimmune disease.
13. The compound for use in the treatment or prevention according to statement 11 wherein the CCR8 mediated disease is selected from the group consisting of graft-versus-host disease (GVHD), multiple sclerosis, inflammatory bowel disease, Crohn's disease, rheumatoid arthritis, psoriasis and diabetes.
14. The compound for use in the treatment or prevention according to any of statement 11 to 13, wherein the compound is selected from the group consisting of:
   3-benzyl-8-(3-(2-methoxyphenoxy)benzyl)-1-oxa-3,8-diazaspiro[4.5]decan-2-one 8-(3-(2-methoxyphenoxy)benzyl)-3-phenethyl-1-oxa-3,8-diazaspiro[4.5]decan-2-one 3-benzyl-8-(3-(2-methoxyphenoxy)benzyl)-1,3,8-triazaspiro[4.5]decane-2,4-dione 8-(3-(2-methoxyphenoxy)benzyl)-3-phenethyl-1,3,8-triazaspiro[4.5]decane-2,4-dione 1-benzyl-8-(3-(2-methoxyphenoxy)benzyl)-1,8-diazaspiro[4.5]decan-2-one
   8-(3-(2-methoxyphenoxy)benzyl)-1-phenethyl-1,8-diazaspiro[4.5]decan-2-one 2-benzyl-8-(3-(2-methoxyphenoxy)benzyl)-2,8-diazaspiro[4.5]decan-1-one 8-(3-(2-methoxyphenoxy)benzyl)-2-phenethyl-2,8-diazaspiro[4.5]decan-1-one 1-benzyl-8-(3-(2-methoxyphenoxy)benzyl)-1,8-diazaspiro[4.5]decane 8-(3-(2-methoxyphenoxy)benzyl)-1-phenethyl-1,8-diazaspiro[4.5]decane 2-benzyl-8-(3-(2-methoxyphenoxy)benzyl)-2,8-diazaspiro[4.5]decane 8-(3-(2-methoxyphenoxy)benzyl)-2-phenethyl-2,8-diazaspiro[4.5]decane 8-(3-(2-methoxyphenoxy)benzyl)-4-phenyl-1,4,8-triazaspiro[4.5]decan-2-one 4-benzyl-8-(3-(2-methoxyphenoxy)benzyl)-1,4,8-triazaspiro[4.5]decan-2-one 8-(3-(2-methoxyphenoxy)benzyl)-4-phenethyl-1,4,8-triazaspiro[4.5]decan-2-one 8-(3-(2-methoxyphenoxy)benzyl)-4-phenyl-1,2,4,8-tetraazaspiro[4.5]decan-3-one 4-benzyl-8-(3-(2-methoxyphenoxy)benzyl)-1,2,4,8-tetraazaspiro[4.5]decan-3-one 1-benzyl-8-(2-isobutoxybenzyl)-1,8-diazaspiro[4.5]decan-2-one
   4-benzyl-9-(3-(2-methoxyphenoxy)benzyl)-1-oxa-4,9-diazaspiro[5.5]undecan-3-one 9-(3-(2-methoxyphenoxy)benzyl)-4-phenethyl-1-oxa-4,9-diazaspiro[5.5]undecan-3-one
   4-benzyl-9-(3-(2-methoxyphenoxy)benzyl)-1-oxa-4,9-diazaspiro[5.5]undecane 9-(3-(2-methoxyphenoxy)benzyl)-4-phenethyl-1-oxa-4,9-diazaspiro[5.5]undecane 4-(4-fluorobenzyl)-9-(3-(2-methoxyphenoxy)benzyl)-1-oxa-4,9-diazaspiro[5.5]undecan-3-one
   4-(3-fluorobenzyl)-9-(3-(2-methoxyphenoxy)benzyl)-1-oxa-4,9-diazaspiro[5.5]undecan-3-one
   4-(2-fluorobenzyl)-9-(3-(2-methoxyphenoxy)benzyl)-1-oxa-4,9-diazaspiro[5.5]undecan-3-one
   4-(4-chlorobenzyl)-9-(3-(2-methoxyphenoxy)benzyl)-1-oxa-4,9-diazaspiro[5.5]undecan-3-one
   4-(3-chlorobenzyl)-9-(3-(2-methoxyphenoxy)benzyl)-1-oxa-4,9-diazaspiro[5.5]undecan-3-one
   4-(2-chlorobenzyl)-9-(3-(2-methoxyphenoxy)benzyl)-1-oxa-4,9-diazaspiro[5.5]undecan-3-one
   4-((9-(3-(2-methoxyphenoxy)benzyl)-3-oxo-1-oxa-4,9-diazaspiro[5.5]undecan-4-yl)methyl)benzonitrile
   3-((9-(3-(2-methoxyphenoxy)benzyl)-3-oxo-1-oxa-4,9-diazaspiro[5.5]undecan-4-yl)methyl)benzonitrile
   2-((9-(3-(2-methoxyphenoxy)benzyl)-3-oxo-1-oxa-4,9-diazaspiro[5.5]undecan-4-yl)methyl)benzonitrile
   9-(3-(2-methoxyphenoxy)benzyl)-4-(pyridin-2-ylmethyl)-1-oxa-4,9-diazaspiro[5.5]undecan-3-one
   1-benzyl-9-(3-(2-methoxyphenoxy)benzyl)-4-oxa-1,9-diazaspiro[5.5]undecan-2-one 4-benzyl-9-(3-(2-methoxyphenoxy)benzyl)-2-methyl-1-oxa-4,9-diazaspiro[5.5]undecan-3-one
   4-(2-fluorophenyl)-9-(3-(2-methoxyphenoxy)benzyl)-1-oxa-4,9-diazaspiro[5.5]undecan-3-one
   (*S*)-4-(1-(2-fluorophenyl)ethyl)-9-(3-(2-methoxyphenoxy)benzyl)-1-oxa-4,9-diazaspiro[5.5]undecan-3-one
   (*R*)*-4-(1-(2-fluorophenyl)ethyl)-9-(3-(2-methoxyphenoxy)benzyl)-1-oxa-4,9-*diazaspiro[5.5]undecan-3-one
   4-benzyl-9-((3,3-dimethyl-2,3-dihydrobenzofuran-4-yl)methyl)-1-oxa-4,9-diazaspiro[5.5]undecan-3-one
   4-benzyl-9-((2,2-dimethyl-2,3-dihydrobenzofuran-4-yl)methyl)-1-oxa-4,9-diazaspiro[5.5]undecan-3-one
   4-benzyl-9-((2,2-dimethyl-2,3-dihydrobenzofuran-7-yl)methyl)-1-oxa-4,9-diazaspiro[5.5]undecan-3-one
   4-benzyl-9-((2,2-dimethyl-2H-chromen-8-yl)methyl)-1-oxa-4,9-diazaspiro[5.5]undecan-3-one
   9-(benzofuran-7-ylmethyl)-4-benzyl-1-oxa-4,9-diazaspiro[5.5]undecan-3-one 4-benzyl-9-((2,3-dihydrobenzofuran-7-yl)methyl)-1-oxa-4,9-diazaspiro[5.5]undecan-3-one
   4-benzyl-9-(chroman-8-ylmethyl)-1-oxa-4,9-diazaspiro[5.5]undecan-3-one 9-(benzo[d][1,3]dioxol-4-ylmethyl)-4-benzyl-1-oxa-4,9-diazaspiro[5.5]undecan-3-one 4-benzyl-9-((2,3-dihydrobenzo[b][1,4]dioxin-5-yl)methyl)-1-oxa-4,9-diazaspiro[5.5]undecan-3-one
   4-benzyl-9-((3,4-dihydro-2*H*-benzo[b][1,4]dioxepin-6-yl)methyl)-1-oxa-4,9-diazaspiro[5.5]undecan-3-one
   4-benzyl-9-((2,2-dimethylbenzo[d][1,3]dioxol-4-yl)methyl)-1-oxa-4,9-diazaspiro[5.5]undecan-3-one
   4-benzyl-9-((2,2-difluorobenzo[d][1,3]dioxol-4-yl)methyl)-1-oxa-4,9-diazaspiro[5.5]undecan-3-one
   9-((2,2-dimethyl-2,3-dihydrobenzofuran-4-yl)methyl)-4-(2-fluorophenyl)-1-oxa-4,9-diazaspiro[5.5]undecan-3-one
   9-((2,2-dimethyl-2,3-dihydrobenzofuran-4-yl)methyl)-4-(2-fluorobenzyl)-1-oxa-4,9-diazaspiro[5.5]undecan-3-one
   9-((2,2-dimethyl-2,3-dihydrobenzofuran-4-yl)methyl)-4-(pyridin-2-ylmethyl)-1-oxa-4,9-diazaspiro[5.5]undecan-3-one
   (*R*)-9-((2,2-dimethyl-2,3-dihydrobenzofuran-4-yl)methyl)-4-(1-(2-fluorophenyl)ethyl)-1-oxa-4,9-diazaspiro[5.5]undecan-3-one
   (*S*)-9-((2,2-dimethyl-2,3-dihydrobenzofuran-4-yl)methyl)-4-(1-(2-fluorophenyl)ethyl)-1-oxa-4,9-diazaspiro[5.5]undecan-3-one
   (*S*)-4-(1-(2,4-difluorophenyl)ethyl)-9-((2,2-dimethyl-2,3-dihydrobenzofuran-4-yl)methyl)-1-oxa-4,9-diazaspiro[5.5]undecan-3-one
   (*S*)-9-((6-chloro-2,2-dimethyl-2,3-dihydrobenzofuran-4-yl)methyl)-4-(1-(2-fluorophenyl)ethyl)-1-oxa-4,9-diazaspiro[5.5]undecan-3-one
   (*S*)-9-((6-fluoro-2,2-dimethyl-2,3-dihydrobenzofuran-4-yl)methyl)-4-(1-(2-fluorophenyl)ethyl)-1-oxa-4,9-diazaspiro[5.5]undecan-3-one
   (*S*)-9-((2,2-dimethyl-2,3-dihydrofuro[2,3-c]pyridin-4-yl)methyl)-4-(1-(2-fluorophenyl)ethyl)-1-oxa-4,9-diazaspiro[5.5]undecan-3-one
   (*S*)-9-((3*H*-spiro[benzofuran-2,1'-cyclopropan]-4-yl)methyl)-4-(1-(2-fluorophenyl)ethyl)-1-oxa-4,9-diazaspiro[5.5]undecan-3-one
15. A pharmaceutical composition comprising a compound according to any one of the statements 1 to 9, and a pharmaceutically acceptable carrier.

### BRIEF DESCRIPTION OF THE FIGURES

**Figure 1****.** Small molecule CCR8 antagonists.
**Figure 2****.** Small molecule CCR8 agonists.

### DETAILED DESCRIPTION OF THE INVENTION

One embodiment of the present invention concerns a compound of formula I according to the invention wherein R₁ is 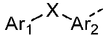 and wherein R₂ₐ, R_{2b}, R₃ₐ, R_{3b} R₄ₐ, R_{4b}, R₅, X, Ar₄ and 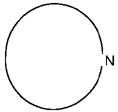 have any of the values as described herein and wherein Ar₁ and Ar₂ are heteroaryl and aryl groups, wherein said heteroaryl or aryl groups are optionally substituted with one or more substituents selected from the group consisting of halogen, C1-7 alkyl, C2-7 alkenyl, C2-7 alkynyl, halo C1-7 alkyl, nitro, hydroxyl, sulfhydryl, amino, C1-7 alkoxy, C3-10 cycloalkoxy, aryloxy, arylalkyloxy, oxyheterocyclic, heterocyclic-substituted alkyloxy, thio C1-7 alkyl, thio C3-10 cycloalkyl, thioaryl, thio-heterocyclic, arylalkylthio, heterocyclic-substituted alkylthio, formyl, carbamoyl, thiocarbamoyl, ureido, thioureido, sulfonamido, hydroxylamino, alkoxy-amino, mercaptoamino, thioalkylamino, acylamino, thioacylamino, cyano, carboxylic acid or esters or thioesters or halides or anhydrides or amides thereof, thiocarboxylic acid or esters or thioesters or halides or anhydrides or amides thereof, alkylamino, cycloalkylamino, alkenylamino, cyclo-alkenylamino, alkynylamino, arylamino, arylalkylamino, hydroxyalkylamino, mercaptoalkylamino, heterocyclic amino; preferably said Ar₁ and Ar₂ are aryl groups independently selected from the group consisting of an unsubstituted heteroaryl and an aryl group, wherein said aryl group is optionally substituted with one or more substituents selected from the group consisting of halogen, C1-7 alkyl, halo C1-7 alkyl, C1-7 alkoxy, hydroxyl and cyano. In certain embodiments thereof, said Ar₁ is an aryl group, optionally substituted with one or more substituents selected from the group consisting of halogen, C1-7 alkyl, halo C1-7 alkyl, C1-7 alkoxy, hydroxyl and cyano; preferably said aryl is a C6 aryl. In certain embodiments thereof, said Ar₂ is an aryl group, optionally substituted with one or more substituents selected from the group consisting of halogen, C1-7 alkyl, halo C1-7 alkyl, C1-7 alkoxy, hydroxyl and cyano; preferably said aryl is a C6 aryl. In certain other embodiments thereof, said Ar₂ is a C6 aryl which is substituted with one C1-7 alkoxy, preferably a methoxy.

Another embodiment of the present invention concerns a compound of formula I according to the invention wherein R₁ is 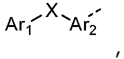 and wherein R₂ₐ, R_{2b}, R₃ₐ, R_{3b} R₄ₐ, R_{4b}, R₅, Ar₁, Ar₂, Ar₄ and 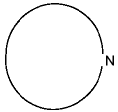 have any of the values as described herein and wherein X is O, NH, S, or CH₂, preferably X is O (oxygen).

Another embodiment of the present invention concerns a compound of formula I according to the invention wherein R₁ is Ar₃, R₂ₐ, R_{2b}, R₃ₐ, R_{3b} R₄ₐ, R_{4b}, R₅, X, Ar₄ and 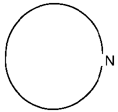 have any of the values as described herein and wherein said Ar₃ is a heteroaryl or aryl group, wherein said heteroaryl or aryl group is optionally substituted with one or more substituents selected from the group consisting of halogen, C1-7 alkyl, C1-7 alkoxy, halo C1-7 alkyl, nitro, hydroxyl, amino, and Ar₃ is optionally fused with a saturated or partly unsaturated heterocyclic ring with at least one oxygen in said heterocyclic ring; wherein said heterocyclic ring is optionally substituted with one or more substituents selected from the group consisting of halogen, C1-7 alkyl or is optionally spirofused with a C3-10 cycloalkyl; preferably said Ar₃ is fused with a saturated or partly unsaturated heterocyclic 5- or 6-ring with at least one oxygen in said heterocyclic ring; wherein said heterocyclic ring is optionally substituted with one or more substituents selected from the group consisting of halogen and C1-7 alkyl or said heterocyclic ring is optionally spirofused with a C3-10 cycloalkyl. In certain embodiments thereof, said Ar₃ is fused with a saturated or partly unsaturated heterocyclic 5- or 6-ring with one or two oxygen atoms in said heterocyclic ring, wherein said heterocyclic ring is optionally substituted with one or two C1-7 alkyl, more preferably two C1-3 alkyl and even more preferably two methyl substituted at the same carbon-atom of said heterocyclic ring. In certain embodiments thereof said Ar₃ is a C₆ aryl group optionally substituted with one C1-3 alkyl or one halogen. In certain embodiments thereof, said Ar₃ is an unsubstituted C₆ aryl group.

In another embodiment of the present invention concerns a compound of formula I according to the invention wherein R₁ is Ar₃ , R₂ₐ, R_{2b}, R₃ₐ, R_{3b} R₄ₐ, R_{4b}, R₅, X, Ar₄ and 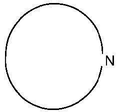 have any of the values as described herein and wherein said Ar₃ is a C₆ aryl group optionally substituted with one or more substituents selected from the group consisting of halogen, C1-7 alkyl, C1-7 alkoxy, halo C1-7 alkyl, nitro, hydroxyl, amino; preferably said C₆ aryl group is substituted with one substituent selected from the group consisting of halogen, C1-7 alkyl, C1-7 alkoxy, more preferably said one substituent is a C1-7 alkoxy.

Another embodiment of the present invention concerns a compound of formula I according to the invention wherein R₁, R₃ₐ, R_{3b} R₄ₐ, R_{4b}, R₅, X, Ar₁, Ar₂, Ar₃, Ar₄ and 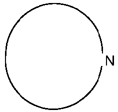 have any of the values as described herein and wherein R₂ₐ and R_{2b} are independently selected from H or C1-7 alkyl, preferably R₂ₐ and R_{2b} are independently selected from H or C1-3 alkyl, more preferably R₂ₐ and R_{2b} are both hydrogen.

Another embodiment of the present invention concerns a compound of formula I according to the invention wherein R₁, R₂ₐ, R_{2b}, R₄ₐ, R_{4b}, R₅, X, Ar₁, Ar₂, Ar₃, Ar₄ and 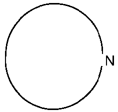 have any of the values as described herein and wherein R₃ₐ and R_{3b} are independently selected from the group consisting of hydrogen, C1-3 alkyl and a carbonyl group; preferably R₃ₐ and R_{3b} are both hydrogen.

Another embodiment of the present invention concerns a compound of formula I according to the invention wherein R₁, R₂ₐ, R_{2b}, R₃ₐ, R_{3b}, R₅, X, Ar₁, Ar₂, Ar₃, Ar₄ and 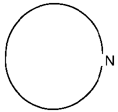 have any of the values as described herein and wherein R₄ₐ and R_{4b} are independently selected from the group consisting of hydrogen, C1-7 alkyl and a carbonyl group; preferably said R₄ₐ and R_{4b} are independently selected from the group consisting of hydrogen, C1-3 alkyl and a carbonyl group; more preferably said R₄ₐ and R_{4b} are independently selected from the group consisting of hydrogen, methyl and a carbonyl group; even more preferably said R₄ₐ is a carbonyl group and said R_{4b} is a hydrogen or a methyl.

Another embodiment of the present invention concerns a compound of formula I according to the invention wherein R₁, R₂ₐ, R_{2b}, R₃ₐ, R_{3b} R₄ₐ, R_{4b}, R₅, X, Ar₁, Ar₂, Ar₃ and Ar₄ have any of the values as described herein and wherein 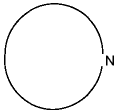 schematically represents a saturated or partly unsaturated heterocyclic ring with at least one nitrogen in said heterocyclic ring. In certain embodiments thereof, said heterocyclic ring is a 5- or 6-ring with one or two nitrogen and one oxygen as heteroatoms in said 5- or 6-ring. In certain embodiments thereof, said heterocyclic ring is a 5- or 6-ring with one nitrogen and one oxygen as heteroatoms in said 5- or 6-ring. In other embodiments thereof, said heterocyclic ring is a 5-ring with only one nitrogen as heteroatom in said ring. In yet other embodiments thereof, said heterocyclic ring is a 6-ring with one nitrogen and one oxygen as only heteroatoms in said 6-ring.

Another embodiment of the present invention concerns a compound of formula I according to the invention wherein R₁, R₂ₐ, R_{2b}, R₃ₐ, R_{3b} R₄ₐ, R_{4b}, X, Ar₁, Ar₂, Ar₃, and 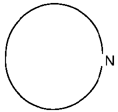 have any of the values as described herein and wherein R₅ is selected from the group consisting of H, C1-7 alkyl or (CR₇ₐR_{7b})ₙ-Ar₄, wherein n is 0-2 and wherein R₇ₐ and R_{7b} are independently selected from H or C1-7 alkyl, and wherein Ar₄ is a heteroaryl and aryl group, wherein said heteroaryl or aryl groups are optionally substituted with one or more substituents selected from the group consisting of halogen, C1-7 alkyl, C3-10 cycloalkyl, halo C1-7 alkyl, nitro, hydroxyl, cyano, amino, C1-7 alkoxy, C3-10 cycloalkoxy, cyano, carboxylic acid or esters. In certain embodiments thereof, said R₅ is (CR₇ₐR_{7b})ₙ-Ar₄, wherein n, R₇ₐ, R_{7b} and Ar₄ have any of the values as described herein. In certain embodiments thereof, said n is 1 or 2, in specific embodiments thereof said n is 1 in other specific embodiments said n is 2. In certain embodiments thereof, said R₇ₐ and R_{7b} are independently selected from H or C1-3 alkyl, preferably said R₇ₐ is hydrogen and said R_{7b} is hydrogen or methyl. In certain embodiments thereof said Ar₄ is a C₆-heteroaryl or a C₆-aryl group, wherein said heteroaryl or aryl group is optionally substituted with one or more, preferably one or two substituents selected from the group consisting of halogen, C1-3 alkyl and cyano. In certain embodiments thereof said Ar₄ is a C₆-aryl group; in more specific embodiments thereof, said C₆-aryl group is unsubstituted; in other specific embodiments thereof, said C₆-aryl group is substituted with one halogen.

The present invention also concerns a compound of formula I, any subgroup thereof, and/or a pharmaceutical acceptable addition salt thereof and/or a stereoisomer thereof and/or a solvate thereof, for use as a medicine.

The present invention also concerns a compound of formula I, any subgroup thereof, and/or a pharmaceutical acceptable addition salt thereof and/or a stereoisomer thereof and/or a solvate thereof, for use as a medicine for the prevention or treatment of a CCR8 mediated disease in an animal. In a specific embodiment thereof said CCR8 mediated disease is an autoimmune disease, more specifically said autoimmune disease is selected from the group consisting of graft-versus-host disease (GVHD), multiple sclerosis, inflammatory bowel disease, Crohn's disease, rheumatoid arthritis, psoriasis and diabetes.

The present invention also concerns a compound of formula I, any subgroup thereof, and/or a pharmaceutical acceptable addition salt thereof and/or a stereoisomer thereof and/or a solvate thereof, for the manufacture of a medicament for the prevention or treatment of an autoimmune disease in an animal. In an embodiment, said animal is a mammal, preferably said mammal is a human being.

The present invention also concerns a pharmaceutical composition comprising a therapeutically effective amount of a compound of formula I, any subgroup thereof, and/or a pharmaceutical acceptable addition salt thereof and/or a stereoisomer thereof and/or a solvate thereof, and one or more pharmaceutically acceptable excipients. In an embodiment, said pharmaceutical composition further comprises one or more biologically active drugs being selected from the group consisting of immunosuppressant and/or immunomodulator drugs.

The present invention also concerns a method of prevention or treatment of an immune disorder in a mammal, comprising the administration of a therapeutically effective amount of a compound of formula I, any subgroup thereof, and/or a pharmaceutical acceptable addition salt thereof and/or a stereoisomer thereof and/or a solvate thereof, optionally in combination with one or more pharmaceutically acceptable excipients. In an embodiment, said immune disorder is an autoimmune disorder.

### DEFINITIONS

When describing the compounds of the invention, the terms used are to be construed in accordance with the following definitions, unless a context dictates otherwise.

As used herein with respect to a substituting radical, and unless otherwise stated, the term " C₁₋₇ alkyl " means straight and branched chain saturated acyclic hydrocarbon monovalent radicals having from 1 to 7 carbon atoms such as, for example, methyl, ethyl, propyl, n-butyl, 1-methylethyl (isopropyl), 2-methylpropyl (isobutyl), 1,1-dimethylethyl (tert-butyl), 2-methylbutyl, n-pentyl, dimethylpropyl, n- hexyl, 2-methylpentyl, 3-methylpentyl, n-heptyl and the like. By analogy, the term " C₁₋₃ alkyl " refers to such radicals having from 1 to 3 carbon atoms, i.e. up to and including propyl. As used herein with respect to a substituting radical, and unless otherwise stated, the term "aryl " designate any mono- or polycyclic aromatic monovalent hydrocarbon radical having from 6 up to 30 carbon atoms such as but not limited to phenyl, naphthyl, anthracenyl, phenantracyl, fluoranthenyl, chrysenyl, pyrenyl, biphenylyl, terphenyl, picenyl, indenyl, biphenyl, indacenyl, benzocyclobutenyl, benzocyclooctenyl and the like, including fused benzo-C₄-β cycloalkyl radicals (the latter being as defined above) such as, for instance, indanyl, tetrahydronaphthyl, fluorenyl and the like, all of the said radicals being optionally substituted with one or more substituents independently selected from the group consisting of halogen, C1-7 alkyl, C2-7 alkenyl, C2-7 alkynyl, halo C1-7 alkyl, nitro, hydroxyl, sulfhydryl, amino, C1-7 alkoxy, C3-10 cycloalkoxy, aryloxy, arylalkyloxy, oxyheterocyclic, heterocyclic-substituted alkyloxy, thio C1-7 alkyl, thio C3-10 cycloalkyl, thioaryl, thio-heterocyclic, arylalkylthio, heterocyclic-substituted alkylthio, formyl, carbamoyl, thiocarbamoyl, ureido, thioureido, sulfonamido, hydroxylamino, alkoxy-amino, mercaptoamino, thioalkylamino, acylamino, thioacylamino, cyano, carboxylic acid or esters or thioesters or halides or anhydrides or amides thereof, thiocarboxylic acid or esters or thioesters or halides or anhydrides or amides thereof, alkylamino, cycloalkylamino, alkenylamino, cyclo-alkenylamino, alkynylamino, arylamino, arylalkylamino, hydroxyalkylamino, mercaptoalkylamino, heterocyclic amino and the like.

As used herein with respect to a substituting radical, and unless otherwise stated, the terms " C₁₋₇ alkoxy ", " C₃₋₁₀ cycloalkoxy ", " aryloxy ", " arylalkyloxy ", " oxyheterocyclic ", " thio C₁₋₇ alkyl ", " thio C₃₋₁₀ cycloalkyl ", " arylthio ", " arylalkylthio " and " thioheterocyclic" refer to substituents wherein a carbon atom of a C₁₋₇ alkyl, respectively a C₃₋₁₀ cycloalkyl, aryl, arylalkyl or heterocyclic radical (each of them such as defined herein), is attached to an oxygen atom or a divalent sulfur atom through a single bond such as, but not limited to, methoxy, ethoxy, propoxy, butoxy, pentoxy, isopropoxy, sec-butoxy, tert-butoxy, isopentoxy, cyclopropyloxy, cyclobutyloxy, cyclopentyloxy, thiomethyl, thioethyl, thiopropyl, thiobutyl, thiopentyl, thiocyclopropyl, thiocyclobutyl, thiocyclopentyl, thiophenyl, phenyloxy, benzyloxy, mercaptobenzyl, cresoxy, and the like.

As used herein with respect to a substituting atom, and unless otherwise stated, the term "halogen" or "halo" means any atom selected from the group consisting of fluorine, chlorine, bromine and iodine.

As used herein with respect to a substituting radical, and unless otherwise stated, the term " halo C1-7 alkyl " means a C1-7 alkyl radical (such as above defined) in which one or more hydrogen atoms are independently replaced by one or more halogens (preferably fluorine, chlorine or bromine), such as but not limited to difluoromethyl, trifluoromethyl, trifluoroethyl, octafluoropentyl, dodecafluoroheptyl, dichloromethyl and the like.

As used herein with respect to a substituting radical, and unless otherwise stated, the term " heterocyclic " means a mono- or polycyclic, saturated or mono-unsaturated or polyunsaturated monovalent hydrocarbon radical having from 2 up to 15 carbon atoms and including one or more heteroatoms in one or more heterocyclic rings, each of said rings having from 3 to 10 atoms (and optionally further including one or more heteroatoms attached to one or more carbon atoms of said ring, for instance in the form of a carbonyl or thiocarbonyl or selenocarbonyl group, and/or to one or more heteroatoms of said ring, for instance in the form of a sulfone, sulfoxide, N-oxide, phosphate, phosphonate or selenium oxide group), each of said heteroatoms being independently selected from the group consisting of nitrogen, oxygen, sulfur, selenium and phosphorus, also including radicals wherein a heterocyclic ring is fused to one or more aromatic hydrocarbon rings for instance in the form of benzo-fused, dibenzo-fused and naphtho-fused heterocyclic radicals; within this definition are included heterocyclic radicals such as, but not limited to, diazepinyl, oxadiazinyl, thiadiazinyl, dithiazinyl, triazolonyl, diazepinonyl, triazepinyl, triazepinonyl, tetrazepinonyl, benzoquinolinyl, benzothiazinyl, benzothiazinonyl, benzoxa-thiinyl, benzodioxinyl, benzodithiinyl, benzoxazepinyl, benzothiazepinyl, benzodiazepine, benzodioxepinyl, benzodithiepinyl, benzoxazocinyl, benzothiazocinyl, benzodiazocinyl, benzoxathiocinyl, benzodioxocinyl, benzotrioxepinyl, benzoxathiazepinyl, benzoxadiazepinyl, benzothia-diazepinyl, benzotriazepinyl, benzoxathiepinyl, benzotriazinonyl, benzoxazolinonyl, azetidinonyl, azaspiroundecyl, dithiaspirodecyl, selenazinyl, selenazolyl, selenophenyl, hypoxanthinyl, azahypoxanthinyl, bipyrazinyl, bipyridinyl, oxazolidinyl, diselenopyrimidinyl, benzodioxocinyl, benzopyrenyl, benzopyranonyl, benzophenazinyl, benzoquinolizinyl, dibenzocarbazolyl, dibenzoacridinyl, dibenzophenazinyl, dibenzothiepinyl, dibenzoxepinyl, dibenzopyranonyl, dibenzoquinoxalinyl, dibenzothiazepinyl, dibenzisoquinolinyl, tetraazaadamantyl, thiatetraazaadamantyl, oxauracil, oxazinyl, dibenzothiophenyl, dibenzofuranyl, oxazolinyl, oxazolonyl, azaindolyl, azolonyl, thiazolinyl, thiazolonyl, thiazolidinyl, thiazanyl, pyrimidonyl, thiopyrimidonyl, thiamorpholinyl, azlactonyl, naphtindazolyl, naphtindolyl, naphtothiazolyl, naphtothioxolyl, naphtoxindolyl, naphto- triazolyl, naphtopyranyl, oxabicycloheptyl, azabenzimidazolyl, azacycloheptyl, azacyclooctyl, azacyclononyl, azabicyclononyl, tetrahydrofuryl, tetrahydropyranyl, tetrahydro-pyronyl, tetrahydroquinoleinyl, tetrahydrothienyl and dioxide thereof, dihydrothienyl dioxide, dioxindolyl, dioxinyl, dioxenyl, dioxazinyl, thioxanyl, thioxolyl, thiourazolyl, thiotriazolyl, thiopyranyl, thiopyronyl, coumarinyl, quinoleinyl, oxyquinoleinyl, quinuclidinyl, xanthinyl, dihydropyranyl, benzodihydrofuryl, benzothiopyronyl, benzothiopyranyl, benzoxazinyl, benzoxazolyl, benzodioxolyl, benzodioxanyl, benzothiadiazolyl, benzotriazinyl, benzothiazolyl, benzoxazolyl, phenothioxinyl, phenothiazolyl, phenothienyl (benzothiofuranyl), phenopyronyl, phenoxazolyl, pyridinyl, dihydropyridinyl, tetrahydropyridinyl, piperidinyl, morpholinyl, thiomorpholinyl, pyrazinyl, pyrimidinyl, pyridazinyl, triazinyl, tetrazinyl, triazolyl, benzotriazolyl, tetrazolyl, imidazolyl, pyrazolyl, thiazolyl, thiadiazolyl, isothiazolyl, oxazolyl, oxadiazolyl, pyrrolyl, furyl, dihydrofutyl, furoyl, hydantoinyl, dioxolanyl, dioxolyl, dithianyl, dithienyl, dithiinyl, thienyl, indolyl, indazolyl, benzofutyl, quinolyl, quinazolinyl, quinoxalinyl, carbazolyl, phenoxazinyl, phenothiazinyl, xanthenyl, purinyl, benzothienyl, naphtothienyl, thianthrenyl, pyranyl, pyronyl, benzopyronyl, isobenzofuranyl, chromenyl, phenoxathiinyl, indolizinyl, quinolizinyl, isoquinolyl, phthalazinyl, naphthiridinyl, cinnolinyl, pteridinyl, carbolinyl, acridinyl, perimidinyl, phenanthrolinyl, phenazinyl, phenothiazinyl, imidazolinyl, imidazolidinyl, benzimidazolyl, pyrazolinyl, pyrazolidinyl, pyrrolinyl, pyrrolidinyl, piperazinyl, uridinyl, thymidinyl, cytidinyl, azirinyl, aziridinyl, diazirinyl, diaziridinyl, oxiranyl, oxaziridinyl, dioxiranyl, thiiranyl, azetyl, dihydroazetyl, azetidinyl, oxetyl, oxetanyl, oxetanonyl, homopiperazinyl, homopiperidinyl, thietyl, thietanyl, diazabicyclooctyl, diazetyl, diaziridinonyl, diaziridinethionyl, . chromanyl, chromanonyl, thiochromanyl, thiochromanonyl, thiochromenyl, benzofuranyl, benzisothiazolyl, benzocarbazolyl, benzochromonyl, benzisoalloxazinyl, benzocoumarinyl, thiocoumarinyl, pheno- metoxazinyl, phenoparoxazinyl, phentriazinyl, thiodiazinyl, thiodiazolyl, indoxyl, thioindoxyl, benzodiazinyl (e.g. phtalazinyl), phtalidyl, phtalimidinyl, phtalazonyl, alloxazinyl, dibenzopyronyl (i.e. xanthonyl), xanthionyl, isatyl, isopyrazolyl, isopyrazolonyl, urazolyl, urazinyl, uretinyl, uretidinyl, succinyl, succinimido, benzylsultimyl, benzylsultamyl and the like, including all possible isomeric forms thereof, wherein each carbon atom of said heterocyclic ring may furthermore be independently substituted with a substituent selected from the group consisting of halogen, nitro, C₁₋₇ alkyl (optionally containing one or more functions or radicals selected from the group consisting of carbonyl (oxo), alcohol (hydroxyl), ether (alkoxy), acetal, amino, imino, oximino, alkyloximino, amino-acid, cyano, carboxylic acid ester or amide, nitro, thio C₁₋₇ alkyl, thio C₃₋₁₀ cycloalkyl, C₁₋₇ alkylamino, cycloalkylamino, alkenylamino, cycloalkenylamino, alkynylamino, arylamino, arylalkyl- amino, hydroxylalkylamino, mercaptoalkylamino, heterocyclic-substituted alkylamino, heterocyclic amino, heterocyclic-substituted arylamino, hydrazino, alkylhydrazino, phenylhydrazino, sulfonyl, sulfonamido and halogen), C₃₋₇ alkenyl, C₂₋₇ alkynyl, halo C₁₋₇ alkyl, C₃₋₁₀ cycloalkyl, aryl, arylalkyl, alkylaryl, alkylacyl, arylacyl, hydroxyl, amino, C₁₋₇ alkylamino, cycloalkylamino, alkenylamino, cycloalkenylamino, alkynylamino, arylamino, arylalkylamino, hydroxyalkylamino, mercaptoalkylamino, heterocyclic- substituted alkylamino, heterocyclic amino, heterocyclic-substituted arylamino, hydrazino, alkylhydrazino, phenylhydrazino, sulfhydryl, C₁₋₇ alkoxy, C₃₋₁₀ cycloalkoxy, aryloxy, arylalkyloxy, oxyheterocyclic, heterocyclic-substituted alkyloxy, thio C₁₋₇ alkyl, thio C₃₋₁₀ cycloalkyl, thioaryl, thioheterocyclic, arylalkylthio, heterocyclic-substituted alkylthio, formyl, hydroxylamino, cyano, carboxylic acid or esters or thioesters or amides thereof, tricarboxylic acid or esters or thioesters or amides thereof; depending upon the number of unsaturations in the 3 to 10 atoms ring, heterocyclic radicals may be sub-divided into heteroaromatic (or "heteroaryl") radicals and non- aromatic heterocyclic radicals.

### Synthesis

### Synthesis of piperidine analogues spirofused with a five-membered heterocycle

Alkylation of the commercially available spirocyclic analogues **1a-b** with benzyl bromide or phenethyl bromide yielded intermediates **2a-d.** The Boc group on the piperidinyl moiety was cleaved off, which was then followed by alkylation of the free nitrogen with 1-(3-(chloromethyl)phenoxy)-2-methoxybenzene affording final compounds **3a-d.** Alternatively, alkylation of the commercially available starting materials **4a-b** by treatment with 1-(3-(chloromethyl)phenoxy)-2-methoxybenzene was performed as the first reaction yielding intermediates **5a-b,** from which the nitrogen of the pyrrolidin-2-one moiety was then alkylated with benzyl bromide and phenethyl bromide affording final compounds **6a-d,** respectively. Reduction of the lactam moiety of the pyrrolidin-2-ones **6a-d** by treatment with lithium aluminium hydride (LAH) yielded the pyrrolidines **7a-d.**

Alkylation of piperidin-4-one **8** with 1-(3-(chloromethyl)phenoxy)-2-methoxybenzene yielded intermediate **9.** The final compounds **10a-c** were obtained by condensation with various 2-amino-acetamides using 10-camphorsulfonic acid as the catalyst. Treatment of intermediate **9** with hydroxylamine-O-sulfonic acid and ammonia in methanol yielded diaziridine **11,** which was then reacted with different isocyanates[1][2], yielding the final compounds **12a-b** in moderate yields.

Compound **15** was prepared by two subsequent alkylations (Scheme **1**). Alkylation of the amide groups of compound **13** by treatment with benzyl bromide yielded intermediate **14.** Then, the Boc group was cleaved off and the crude amine was alkylated with 1-(chloromethyl)-2-isobutoxybenzene directly to get access to the final compound **15.**

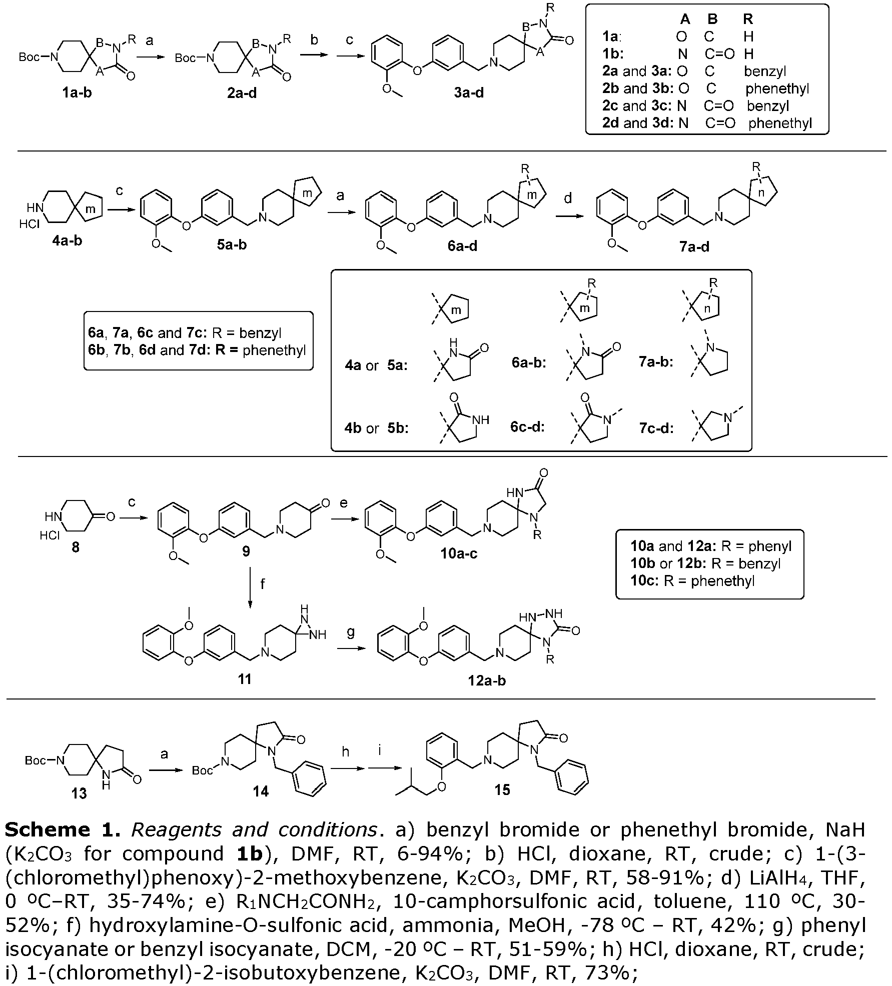

### Synthesis of piperidine analogues spirofused with a morpholine ring

Reaction of the commercially available piperidine analogue **16** with 2-chloroacetyl chloride yielded the corresponding amide as intermediate[3], which upon ring closure under alkaline conditions furnished the morpholin-3-one derivative **17.** The Boc protecting group was removed by acidic treatment. The resulting free amine was alkylated at the nitrogen of the piperidinyl ring, followed by a second alkylation of the lactam moiety yielding final compounds **19a-b.** Finally, LAH-mediated reduction of the morpholin-3-one moiety afforded the morpholine derivatives **20a-b.** Final compounds **21a-j** were obtained by treatment of intermediate **18** with various substituted benzyl bromides under alkaline conditions.

Reduction of the carboxylic acid group of 4-amino-1-Boc-piperidine-4-carboxylic acid **22** by treatment with NaBH₄ and iodine [4] yielded the primary alcohol **23.** Using a similar series of reactions as for the preparation of compound **19a** allowed to synthesize final compound **26** (Scheme **2**).

Compound **29** was synthesized in a similar manner as compound **19a.** Reaction of starting material **16** with 2-chloropropanoyl chloride, followed by an intramolecular ether formation yielded intermediate **27.** Acidic cleavage of the Boc group, followed by two alkylations furnished compound **29.**

Nucleophilic addition of various primary amines to the epoxide of starting material **30** led to the formation of the tertiary alcohols **31a-c**[5]. Coupling with 2-chloroacetyl chloride and subsequent ring closure gave access to intermediates **32a-c.** Finally, Boc deprotection and reaction with 1-(3-(chloromethyl)phenoxy)-2-methoxybenzene yielded the final compounds **33a-c** (Scheme **2**).

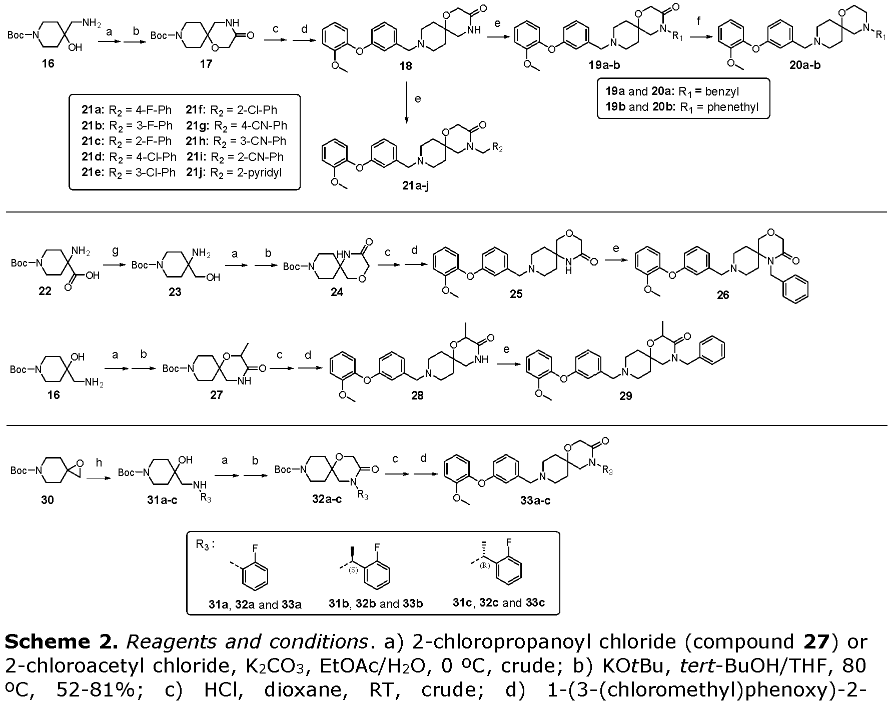

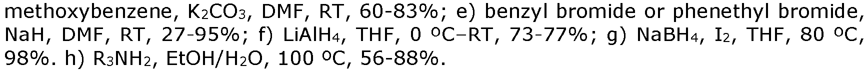

### Synthesis of 1-oxa-4,9-diazaspiro[5.5]undecan-3-one analogues with structural modifications on the piperidinyl nitrogen

Compound **36** was synthesized by two subsequent alkylations (Scheme **3**). Alkylation of compound **17** by reacting with benzyl bromide yielded intermediate **34.** Then, the Boc group was removed by treatment with HCl and the crude amine was alkylated with 1-(chloromethyl)-2-isobutoxybenzene affording final compound **28.**

The phenolic hydroxyl group of 3-hydroxybenzaldehyde **37** reacted with 3-chloro-2-methylprop-1-ene under basic conditions yielding intermediate **38.** A rhodium(III)-catalyzed intramolecular olefin hydroarylation of benzaldehyde **38** with β-alanine as a transient directing group and pivalic acid as additive, yielded the 2,3-dihydrobenzofuran derivative **39**[6]. Starting material **40** was subjected to a Pd(II)-catalyzed C-H activation/C-O cyclization reaction affording dihydrobenzofuran **41**[7]. This was followed by a Br-Li exchange and reaction with DMF to insert an aldehyde functionality yielding intermediate **42**. Benzaldehyde **43** underwent a one-pot Claisen rearrangement at 210 °C using iodine as a catalyst to give dihydrobenzofurane **44** [8]. Salicylaldehyde **45** was coupled with 3-chloro-3-methylbut-1-yne using CuCl₂ as catalyst yielding intermediate **46**. Upon heating at 210 °C in *N,N*-diethylaniline as solvent, a ring closure took place furnishing benzopyrane **47**. Direct lithiation and subsequent formylation with DMF of 4-bromobenzofuran **48** furnished benzofuran-4-carbaldehyde **49.** Reaction of 3-methylcatechol **51** with 2,2-dimethoxypropane under acidic conditions afforded intermediate **52.** The methyl group was brominated with N-bromosuccinimide yielding benzyl bromide **53** [9]. Reduction of the aldehyde group of starting material **54** led to the formation of the primary alcohol, which was chlorinated with thionylchloride affording intermediate **55** was synthesized. Finally, target compounds **56a-I** were obtained by either reductive amination (for intermediates **39, 42, 44, 47, 49** and **50a-e**) or alkylation (for intermediates **53** and **55**) of key intermediate **35.** Straightforward alkylation of the lactam moiety of key intermediate **17** allowed the preparation of intermediates **57b-c.** The intermediate **32a** was synthesized before in Scheme **2.** After removal of the Boc group, reductive amination with aldehyde **42** happened to obtain final compounds **58a-c.** To access intermediate **60,** similar procedures as for the synthesis of compound **55b** were applied, using (*S*)-1-(2,4-difluorophenyl)ethan-1-amine as the nucleophile. Acidic deprotection of the Boc group yielded the piperidine analogue **61a-c.** The aldehyde functionality of dihydrobenzofurane **42** was transformed into a methylene chloride **62** via its reduction to a primary hydroxyl, followed by chlorination. Finally, reaction between benzyl chloride **62** and the piperidine analogues **61a-c,** led to the formation of target compounds **63a-c.**

The addition of magnesium metal to benzyl bromide **64** generated the Grignard reagent, which subsequently nucleophilically attacked acetone to give tertiary alcohol **66b.** Nucleophilic addition of methylmagnesium bromide to the ester moiety of compound **65** yielded intermediate **66a**[10]. A similar methodology as for the synthesis of intermediate **42** allowed to prepare the dihydrobenzofurans **68a-b.**

Treatment of 3,5-dibromo-4-methylpyridine **69** with acetone under strong alkaline conditions by employment of LDA yielded intermediate **70**[11]. An intramolecular Ullmann coupling allowed to isolate intermediate **71.** Using very low temperatures afforded intermediate **72** (-100 °C instead of -40 °C, as being used in the preparation of intermediate **68a**). Final compounds **73a-c** were synthesized by a reductive amination reaction between aldehydes **68a-b/72** and piperidine analogue **32b**. In a 2-step, one-pot reaction, 2-bromo-6-hydroxybenzaldehyde **74** was reacted with ethyl bromoacetate followed by an intramolecular cyclocondensation under high temperature to obtain benzofurane **75**[12]. Then, reduction of the ester moiety was followed by chlorination resulting in the formation of compound **76.** Elimination of the chlorine yielded an exocyclic chlorine as an intermediate **77**[13]. A Simmons-Smith reaction with iodochloromethane and diethylzinc afforded cyclopropyl derivative **78.** Lithiation and formylation furnished intermediate **79.** The aldehyde **79** underwent a similar series of reactions as compound **62** affording benzyl chloride **80.** Finally, the final compound **81** was easily synthesized by alkylation (Scheme **3**).

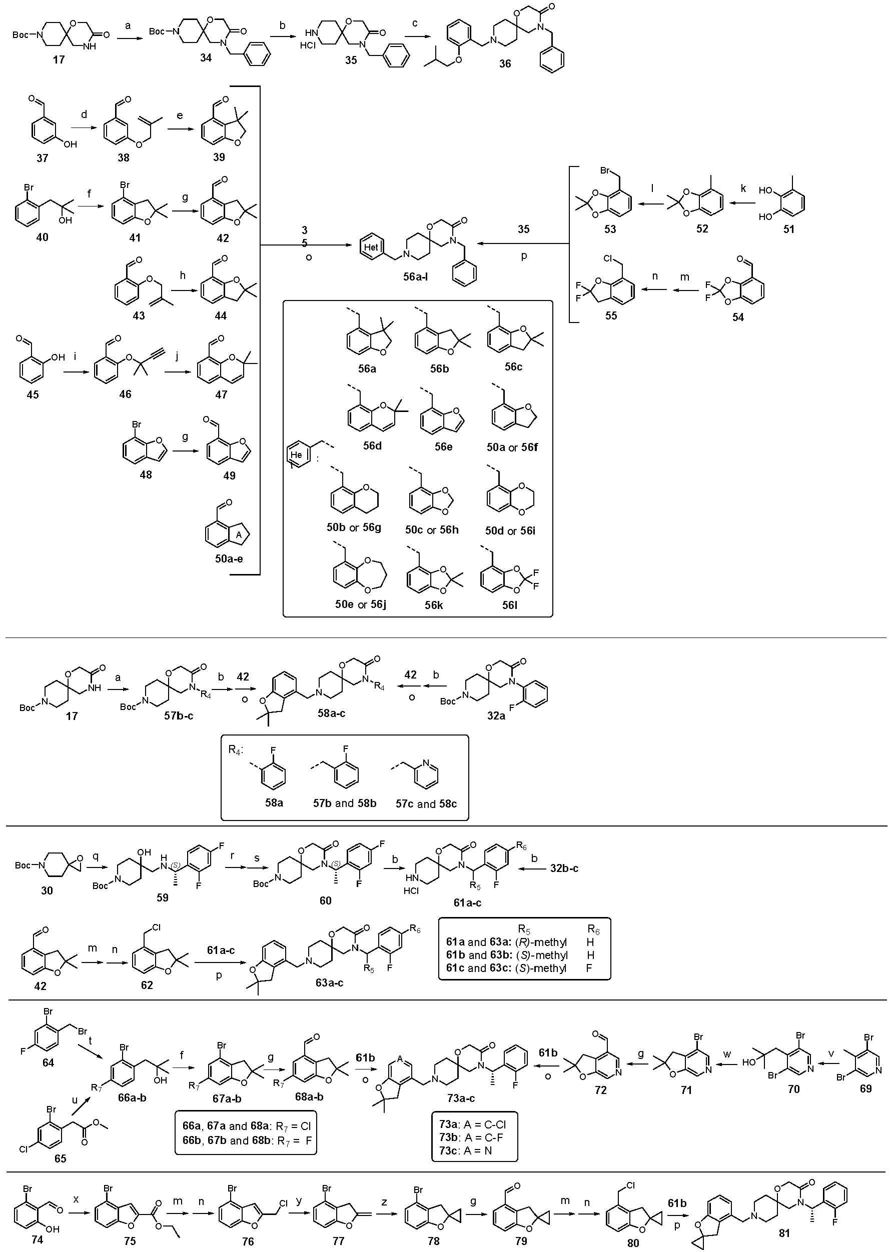

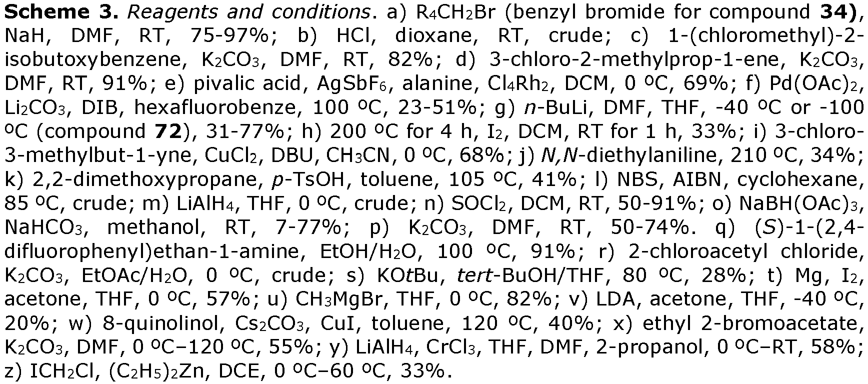

### Experimental section

### General information

### Chemistry

Acros Organics (Geel, Belgium), Merck (Darmstadt, Germany), BLD (Germany), Fluorochem (Hadfield, UK), and TCI Europe (Zwijndrecht, Belgium) provided all chemicals, and chemicals were used as received. Moisture sensitive reactions were carried out under argon atmosphere. Reaction mixtures were stirred magnetically and were run in round-bottom flasks when cooling or room temperature reactions were required. Reactions that were run at elevated temperature (higher than 20 - 30°C) were carried out in sealed screw-capped reaction tubes. The process of the various reactions was monitored by TLC analysis using MilliporeSigma^{™} Silica Gel 60 F254 Coated Aluminum-Backed TLC Sheets or Macherey-Nagel SILPre-coated ALUGRAM^{®} Xtra SIL G/UV₂₅₄ TLC sheets. UV irradiation (254 nm), iodine coated silica or ninhydrin-staining were used to visualize compounds. Compound purification was performed via standard column chromatography. For column chromatography, 70 - 230 mesh silica 60 (Acros, Geel, Belgium) was used as the stationary phase. Solvents were evaporated with a rotary evaporator at 50°C. Samples were dissolved in d₆-DMSO, CDCl₃ or CD₃OD. ¹H NMR spectra were recorded on a Bruker Avance 400 (400 MHz working frequency). Proton-decoupled ¹³C NMR spectra were recorded on a Bruker Avance 400 (101 MHz working frequency). Chemical shifts (δ) were reported in parts per million (ppm) referenced to tetramethylsilane (¹H), or the internal (NMR) solvent signal (¹H and ¹³C) as internal standards. High-resolution mass spectra were acquired on a quadrupole orthogonal acceleration time-of-flight mass spectrometer (Synapt G2 HDMS, Waters, Milford, MA, USA). Samples were infused at 3 µL/min and spectra were obtained in positive ionization mode with a resolution of 15,000 (FWHM-full width at half maximum) using leucine enkephalin as a lock mass. Melting points were determined using a Reichert Thermovar apparatus.

### General procedure 1 (with compound 2a as a representative example)

Compound **1a** (115.3 mg, 0.45 mmol, 1.00 eq) and NaH (19.8, 0.50 mmol, 1.1 eq) were dissolved in DMF (2.0 mL) and the reaction mixture was stirred for 5 minutes at room temperature before the addition of the benzyl bromide (84.7 mg, 0.50 mmol, 1.10 eq). The mixture was stirred at room temperature for 8 h. Then, the reaction mixture was concentrated under reduced pressure affording the crude product. The crude residue was purified by chromatography on silica gel (2% of methanol in dichloromethane) to give compound **2a** as a white solid (133.3 mg, 86% yield).

### General procedure 2 (with compound 3a as a representative example)

The compound **2a** (120.0 mg, 0.35 mmol, 1.00 eq) was dissolved in 1,4-dioxane (2.0 mL) prior to the addition of a hydrogen chloride solution (2.0 mL of a 4.0 M solution in dioxane). This reaction mixture was stirred at room temperature for 6 h. Afterwards, it was concentrated under reduced pressure to give the crude compound 3-benzyl-1-oxa-3,8-diazaspiro[4.5]decan-2-one hydrochloride which was used to do next step without further purification. The crude compound 3-benzyl-1-oxa-3,8-diazaspiro[4.5]decan-2-one hydrochloride (62.2 mg, 0.22 mmol, 1.00 eq) and K₂CO₃ (66.9 mg, 0.48 mmol, 2.20 eq) were dissolved in DMF (2.0 mL) and the reaction mixture was stirred for 5 minutes at room temperature before the addition of the compound 1-(chloromethyl)-3-(2-methoxyphenoxy)benzene (60.2 mg, 0.24 mmol, 1.10 eq). The mixture was stirred at room temperature for 8 h. At last, the reaction mixture was concentrated under reduced pressure affording the crude product. The crude residue was purified by chromatography on silica gel (3% of methanol in dichloromethane) to give compound **3a** as a colorless oil (87.5 mg, 87% yield).

### General procedure 3 (with compound 7a as a representative example)

An oven-dried screw cap test tube containing a magnetic stir-bar and compound **6a** (85.0 mg, 0.19 mmol, 1.00 eq) was evacuated and backfilled with argon three times. Under a counterflow of argon, anhydrous THF (1.0 mL) was added by a syringe. The reaction mixture was cooled down at 0 °C for 10 minutes. Then, LiAlH₄ (0.37 mL of a 1.0 M solution in THF) was added and the reaction mixture was stirred at room temperature for 1.5 h. A saturated aqueous ammonium chloride solution (0.2 mL) was added to quench the reaction. The reaction mixture was concentrated under reduced pressure. The crude residue was purified by chromatography on silica gel (5% methanol in dichloromethane) to give compound **7a** as a yellow oil (61.2 mg, 74% yield).

### General procedure 4 (with compound 10a representative example)

An oven-dried screw cap test tube was added with a magnetic stir-bar, compound **5.9** (37.4 mg, 0.12 mmol, 1.00 eq), 2-(phenylamino)acetamide (19.8 mg, 0.13 mmol, 1.10 eq) and 10-camphorsulfonic acid (30.7 mg, 0.13 mmol, 1.10 eq). The tube was then evacuated and back-filled with argon. The evacuation/backfill sequence was repeated with two additional times. The reaction tube was evacuated and backfilled with argon three times. Under counterflow of argon, 1.0 mL of toluene was added by syringe. After that, the reaction mixture was placed at 110 °C overnight. Then, it was concentrated under reduced pressure affording the crude product. The crude residue was purified by chromatography on silica gel (6% of methanol in dichloromethane) to give compound **10a** as a white solid (27.6 mg, 52% yield).

### General procedure 5 (with compound 17 as a representative example)

A solution of K₂CO₃ (447.8 mg, 3.24 mmol, 2.70 eq) in water (1.5 mL) was added into the solution of compound **16** (276.4 mg, 1.20 mmol, 1.00 eq) in ethyl acetate (3.0 mL). Then, the reaction mixture was stirred at 0 °C for 5 minutes before adding chloroacetyl chloride (183.0 mg, 1.62 mmol, 1.35 eq) dropwise. Afterwards, the reaction mixture was stirred at 0 °C for further 1h. After this, the reaction mixture was washed with brine (10.0 mL) and extracted with ethyl acetate (20.0 mL). The organic layer was collected and dried over magnesium sulfate. Then, it was concentrated under reduced pressure affording the crude product. The potassium t-butoxide (202.0 mg, 1.80 mmol, 1.50 eq) was added into THF (5.0 mL) and t-butanol (2.0 mL). Then, the reaction mixture was stirred at 80 °C for 5 minutes before adding the crude product in THF (3.0 mL) dropwise. After this, the reaction mixture was stirred at 80 °C for further 1h. Then, the reaction mixture was concentrated under reduced pressure affording the crude product. The crude residue was purified by chromatography on silica gel (5% of methanol in dichloromethane) to give compound **17** as a white solid (178.4 mg, 55% yield).

### General procedure 6 (with compound 31a a representative example)

The compound **30** (1.00 g, 4.69 mmol, 1.00 eq) and 2-fluoroaniline (0.52 g, 4.69 mmol, 1.00 eq) were dissolved in ethanol (9.0 mL) and H₂O (1.0 mL). The reaction mixture was stirred at 100 °C for 8 hours. Then, the reaction mixture was concentrated under reduced pressure affording the crude product. The crude residue was purified by chromatography on silica gel (30% of ethyl acetate in petroleum ether) to give compound **31a** as a yellow oil (1.34 g, 88% yield).

### General procedure 7 (with compound 42 as a representative example)

To an oven-dried screw cap test tube with a magnetic stir-bar was added compound **40** (950.0 mg, 4.15 mmol, 1.00 eq), Pd(OAc)₂ (46.5 mg, 0.21 mmol, 0.05 eq), Li₂CO₃ (459.6 mg, 6.22 mmol, 1.50 eq) and iodobenzene diacetate (2003.3 mg, 6.22 mmol, 1.50 eq). The tube was then evacuated and back-filled with argon. This evacuation/ backfill sequence was repeated three times. Under a counterflow of argon, the solvent hexafluorobenzene (10 mL) was added via a syringe. Then, the reaction mixture was stirred for 24 hours at 100 °C. Finally, the reaction mixture was concentrated under reduced pressure affording the crude product. The crude residue was purified by chromatography on silica gel (2% of ethyl acetate in petroleum ether) to give compound **41** as a colorless oil (484.0 mg, 51% yield).

To an oven-dried screw cap test tube containing a magnetic stirring bar was added compound **41** (460.0 mg, 1.00 mmol, 1.00 eq). The reaction tube was evacuated and backfilled with argon three times. Under a counterflow of argon, anhydrous THF (6.0 mL) was added and the reaction mixture was placed at -40 °C for 5 minutes before the addition of n-butyllithium solution (1.20 mL, 2.5 M). Then, the reaction mixture was stirred at -40 °C for 30 minutes before adding DMF (296.1 mg, 4.05 mmol, 2.00 eq). Afterwards, the reaction mixture was stirred at -40 °C for additional 30 minutes and quenched by saturated aqueous ammonium chloride solution (1.0 mL). It was washed with brine (20.0 mL) and extracted with ethyl acetate (40.0 mL). The organic layer was collected and dried over magnesium sulfate. Then, it was concentrated under reduced pressure affording the crude product. The crude residue was purified by chromatography on silica gel (7% of ethyl acetate in petroleum ether) to give compound **42** as a yellow oil (234.2 mg, 66% yield).

### General procedure 8 (with compound 55 a representative example)

An oven-dried screw cap test tube containing a magnetic stir-bar and compound **54** (186.1 mg, 1.00 mmol, 1.00 eq) was evacuated and backfilled with argon three times. Under a counterflow of argon, anhydrous THF (1.5 mL) was added by a syringe. The reaction mixture was cooled down at 0 °C for 10 minutes. Then, LiAlH₄ (2.50 mL of a 1.0 M solution in THF) was added and the reaction mixture was stirred at room temperature for 1.5 h. A saturated aqueous ammonium chloride solution (0.5 mL) was added to quench the reaction. The reaction mixture was concentrated under reduced pressure. It was washed with brine (10.0 mL) and extracted with ethyl acetate (20.0 mL). The organic layer was collected and dried over magnesium sulfate. Then, it was concentrated under reduced pressure affording the crude product which was used for next step without further purification. The crude compound was dissolved into dichloromethane (10.0 mL) and the reaction mixture was stirred for 5 minutes at 0 °C before the addition of the thionyl chloride (594.8 mg, 5.00 mmol, 5.00 eq). The mixture was stirred at room temperature for 2 h. Then, the reaction mixture was washed with brine (10.0 mL) and extracted with dichloromethane (20.0 mL). The organic layer was collected and dried over magnesium sulfate. Then, it was concentrated under reduced pressure affording the crude product. The crude residue was purified by chromatography on silica gel (6% of ethyl acetate in petroleum ether) to give compound **55** as a colorless oil (104.0, 50% yield).

### General procedure 9 (with compound 56a a representative example)

The crude compound **35** (32.0 mg, 0.11 mmol, 1.00 eq), compound **39** (26.6 mg, 0.15 mmol, 1.40 eq) and NaHCO₃ (36.2 mg, 0.26 mmol, 2.40 eq) were dissolved in methanol (2.0 mL), and the reaction mixture was stirred at 0 °C for 5 minutes, followed by the addition of NaBH(OAc)₃ (32.0 mg, 0.15 mmol, 1.40 eq). The mixture was stirred at room temperature for 10 h. The reaction mixture was concentrated under reduced pressure. The crude residue was purified by chromatography on silica gel (3% of methanol in dichloromethane) to give compound **56a** as a yellow oil (20.1 mg, 44% yield).

### The preparation of tert-butyl 3-benzyl-2-oxo-1-oxa-3,8-diazaspiro[4.5]decane-8-carboxylate (compound 2a)

This compound was synthesized via general procedure 1 affording compound **2a** as a white solid, in 86% yield.
¹H NMR (400 MHz, CDCl₃): δ (ppm) 7.38-7.29 (m, 3H), 7.27-7.25 (m, 2H), 4.43 (s, 2H), 3.82-3.79 (m, 2H), 3.31-3.25 (m, 2H), 3.12 (s, 2H), 1.87-1.84 (m, 2H), 1.63-1.56 (m, 2H), 1.44 (s, 9H); ¹³C NMR (101 MHz, CDCl₃) *δ* (ppm) 157.10, 154.53, 135.64, 128.92, 128.05, 128.03, 79.89, 76.79, 54.76, 48.20, 39.77, 35.91, 28.38;

### The preparation of tert-butyl 2,4-dioxo-3-phenethyl-1,3,8-triazaspiro[4.5]decane-8-carboxylate (compound 2b)

This compound was synthesized via general procedure 1 affording compound **2b** as a white solid, in 35% yield.
¹H NMR (400 MHz, CDCl₃): δ (ppm) 7.33-7.31 (m, 2H), 7.25-7.21 (m, 3H), 3.76-3.74 (m, 2H), 3.53 (t, *J* = 7.1 Hz, 2H), 3.27-3.21 (m, 2H), 3.04 (s, 2H), 2.89 (t, *J* = 7.1 Hz, 2H), 1.77-1.73 (m, 2H), 1.58-1.48 (m, 2H), 1.45 (s, 9H); ¹³C NMR (101 MHz, CDCl₃) *δ* (ppm) 156.89, 154.52, 138.26, 128.76, 128.71, 126.75, 79.88, 76.54, 56.01, 45.21, 40.03, 35.84, 34.03, 28.40;

### The preparation of tert-butyl 3-benzyl-2,4-dioxo-1,3,8-triazaspiro[4.5]decane-8-carboxylate (compound 2c)

This compound was synthesized via general procedure 1 affording compound **2c** as a white solid, in 51% yield.
¹H NMR (400 MHz, CDCl₃): δ (ppm) 7.37-7.27 (m, 6H), 4.64 (s, 2H), 4.01-3.99 (m, 2H), 3.19-3.13 (m, 2H), 2.02-1.95 (m, 2H), 1.59-1.56 (m, 2H), 1.47 (s, 9H); ¹³C NMR (101 MHz, CDCl₃) *δ* (ppm) 175.45, 156.97, 154.47, 135.94, 128.72, 128.27, 127.95, 80.18, 59.99, 42.12, 39.72, 33.11, 28.40;

### The preparation of tert-butyl 2,4-dioxo-3-phenethyl-1,3,8-triazaspiro[4.5]decane-8-carboxylate (compound 2d)

This compound was synthesized via general procedure 1 affording compound **2d** as a white solid, in 87% yield.
¹H NMR (400 MHz, CDCl₃): δ (ppm) 7.29-7.18 (m, 6H), 3.97-3.94 (m, 2H), 3.76 (t, *J* = 7.3 Hz, 2H), 3.18-3.11 (m, 2H), 2.95 (t, *J* = 7.3 Hz, 2H), 1.90-1.83 (m, 2H), 1.47 (s, 9H), 1.49-1.44 (m, 2H); ¹³C NMR (101 MHz, CDCl₃) *δ* (ppm) 175.55, 157.03, 154.46, 137.58, 129.01, 128.47, 126.72, 80.14, 59.64, 39.93, 39.41, 33.71, 33.08, 28.41;

### The preparation of 3-benzyl-8-(3-(2-methoxyphenoxy)benzyl)-1-oxa-3,8-diazaspiro[4.5]decan-2-one (compound 3a)

This compound was synthesized via general procedure 2 affording compound **3a** as a colorless oil, in 87% yield.

¹H NMR (400 MHz, CDCl₃): δ (ppm) 7.37-7.30 (m, 3H), 7.27-7.24 (m, 2H), 7.21 (t, *J* = 7.8 Hz, 1H), 7.14-7.10 (m, 1H), 7.01-6.88 (m, 5H), 6.80 (dd, *J* = 2.5, 8.1 Hz, 1H), 4.42 (s, 2H), 3.83 (s, 3H), 3.46 (s, 2H), 3.11 (s, 2H), 2.54-2.50 (m, 4H), 1.91-1.86 (m, 2H), 1.72-1.65 (m, 2H); ¹³C NMR (101 MHz, CDCl₃) *δ* (ppm) 157.86, 157.38, 151.33, 145.14, 140.12, 135.82, 129.26, 128.85, 128.01, 127.94, 124.66, 123.09, 121.06, 120.78, 117.93, 115.86, 112.75, 62.41, 55.93, 54.55, 49.20, 48.18, 36.24; HRMS (ESI-Q-TOF): *m*/*z* [M+H]⁺ calcd. for C₂₈H₃₀N₂O₄: 459.22781; found: 459.2282.

### The preparation of 8-(3-(2-methoxyphenoxy)benzyl)-3-phenethyl-1-oxa-3,8-diazaspiro[4.5]decan-2-one (compound 3b)

This compound was synthesized via general procedure 2 affording compound **3b** as a colorless oil, in 74% yield.

¹H NMR (400 MHz, CDCl₃): δ (ppm) 7.32-7.28 (m, 2H), 7.24-7.19 (m, 4H), 7.15-7.10 (m, 1H), 7.02-6.89 (m, 5H), 6.80 (dd, *J* = 2.5, 8.1 Hz, 1H), 3.84 (s, 3H), 3.51 (t, *J* = 7.2 Hz, 2H), 3.46 (s, 2H), 3.05 (s, 2H), 2.87 (t, *J* = 7.2 Hz, 2H), 2.48-2.46 (m, 4H), 1.82-1.78 (m, 2H), 1.65-1.58 (m, 2H); ¹³C NMR (101 MHz, CDCl₃) *δ* (ppm) 157.86, 157.14, 151.33, 145.15, 140.15, 138.31, 129.26, 128.75, 128.64, 126.65, 124.66, 123.10, 121.06, 120.77, 117.94, 115.85, 112.75, 62.40, 55.94, 55.72, 49.20, 45.20, 36.13, 34.00; HRMS (ESI-Q-TOF): *m*/*z* [M+H]⁺ calcd. for C₂₉H₃₂N₂O₄: 473.24346; found: 473.2436.

### The preparation of 3-benzyl-8-(3-(2-methoxyphenoxy)benzyl)-1,3,8-triazaspiro[4.5]decane-2,4-dione (compound 3c)

This compound was synthesized via general procedure 2 affording compound **3c** as a white solid, in 70% yield.

Mp: 133.5-137.2 °C. ¹H NMR (400 MHz, CDCl₃): δ (ppm) 7.37-7.34 (m, 2H), 7.32-7.20 (m, 4H), 7.15-7.11 (m, 1H), 7.02-7.00 (m, 2H), 6.97-6.95 (m, 2H), 6.93-6.89 (m, 1H), 6.83-6.77 (m, 2H), 4.64 (s, 2H), 3.83 (s, 3H), 3.51 (s, 2H), 2.90-2.86 (m, 2H), 2.20-2.07 (m, 4H), 1.59-1.57 (m, 2H); ¹³C NMR (101 MHz, CDCl₃) *δ* (ppm) 175.98, 157.95, 156.63, 151.36, 145.11, 139.86, 136.14, 129.35, 128.67, 128.31, 127.84, 124.74, 123.02, 121.10, 120.84, 117.83, 115.98, 112.82, 62.60, 60.04, 55.97, 48.98, 42.09, 33.42; HRMS (ESI-Q-TOF): *m*/*z* [M+H]⁺ calcd. for C₂₈H₂₉N₃O₄: 472.22306; found: 472.2232.

### The preparation of 8-(3-(2-methoxyphenoxy)benzyl)-3-phenethyl-1,3,8-triazaspiro[4.5]decane-2,4-dione (compound 3d)

This compound was synthesized via general procedure 2 affording compound **3d** as a white solid, in 58% yield.

Mp: 151.2-153.0 °C. ¹H NMR (400 MHz, CDCl₃): δ (ppm) 7.28-7.18 (m, 6H), 7.15-7.11 (m, 1H), 7.02-6.90 (m, 5H), 6.81 (dd, *J* = 1.8, 8.1 Hz, 1H), 6.25 (br, 1H), 3.84 (s, 3H), 3.75 (t, *J* = 7.4 Hz, 1H), 3.50 (s, 2H), 2.95 (t, *J* = 7.4 Hz, 1H), 2.87-2.84 (m, 2H), 2.15-2.10 (m, 2H), 2.04-1.97 (m, 2H), 1.48-1.45 (m, 2H); ¹³C NMR (101 MHz, CDCl₃) *δ* (ppm) 176.03, 157.95, 156.49, 151.37, 145.09, 139.85, 137.69, 129.35, 129.00, 128.46, 126.65, 124.76, 122.99, 121.10, 120.86, 117.81, 115.95, 112.82, 62.57, 59.65, 55.96, 49.03, 39.44, 33.75, 33.37; HRMS (ESI-Q-TOF): *m*/*z* [M+H]⁺ calcd. for C₂₉H₃₁N₃O₄: 486.23871; found: 486.2393.

### The preparation of 8-(3-(2-methoxybenzyl)benzyl)-1,8-diazaspiro[4.5]decan-2-one (compound 5a)

This compound was synthesized via general procedure 2 affording compound **5a** as a colorless oil, in 91% yield.

¹H NMR (400 MHz, CDCl₃): δ (ppm) 7.22 (t, *J* = 7.8 Hz, 1H), 7.15-7.10 (m, 1H), 7.02-6.93 (m, 4H), 6.93-6.89 (m, 1H), 6.80 (dd, *J* = 2.5, 8.1 Hz, 1H), 6.30 (br, 1H), 3.84 (s, 3H), 3.48 (s, 2H), 2.43-2.39 (m, 4H), 2.38 (t, *J* = 8.1 Hz, 2H), 1.93 (t, *J* = 8.1 Hz, 2H), 1.69 (t, *J* = 5.6 Hz, 4H); ¹³C NMR (101 MHz, CDCl₃) *δ* (ppm) 176.89, 157.84, 151.33, 145.19, 140.13, 129.27, 124.64, 123.10, 121.07, 120.76, 117.99, 115.87, 112.78, 62.60, 57.30, 55.95, 50.32, 37.82, 32.48, 29.59;

### The preparation of 8-(3-(2-methoxybenzyl)benzyl)-2,8-diazaspiro[4.5]decan-1-one (compound 5b)

This compound was synthesized via general procedure 2 affording compound **5b** as a white solid, in 86% yield. ¹H NMR (400 MHz, CDCl₃): δ (ppm) 7.22 (t, *J* = 7.8 Hz, 1H), 7.14-7.10 (m, 1H), 7.05-6.99 (m, 2H), 6.96-6.89 (m, 3H), 6.81 (dd, *J* = 2.5, 8.2 Hz, 1H), 6.56-6.50 (br, 1H), 3.84 (s, 3H), 3.48 (s, 2H), 3.30 (t, *J* = 6.9 Hz, 2H), 2.84-2.81 (m, 2H), 2.12-2.07 (m, 2H), 2.00 (t, *J* = 6.9 Hz, 2H), 1.97-1.91 (m, 2H), 1.43-1.39 (m, 2H); ¹³C NMR (101 MHz, CDCl₃) *δ* (ppm) 182.41, 157.78, 151.31, 145.26, 140.40, 129.20, 124.59, 123.14, 121.07, 120.71, 117.95, 115.87, 112.81, 62.81, 55.96, 49.82, 41.96, 38.75, 32.14, 31.86;

### The preparation of 1-benzyl-8-(3-(2-methoxyphenoxy)benzyl)-1,8-diazaspiro[4.5]decan-2-one (compound 6a)

This compound was synthesized via general procedure 1 affording compound **6a** as a yellow oil, in 65% yield.

¹H NMR (400 MHz, CDCl₃): δ (ppm) 7.30-7.25 (m, 4H), 7.23-7.19 (m, 2H), 7.15-7.11 (m, 1H), 7.01-6.96 (m, 2H), 6.94-6.88 (m, 3H), 6.80 (dd, *J* = 2.4, 8.0 Hz, 1H), 4.44 (s, 2H), 3.81 (s, 3H), 3.44 (s, 2H), 2.78-2.75 (m, 2H), 2.47 (t, *J* = 8.0 Hz, 2H), 2.08-2.01 (m, 2H), 1.95 (t, *J* = 8.0 Hz, 2H), 1.89-1.82 (m, 2H), 1.30-1.27 (m, 2H); ¹³C NMR (101 MHz, CDCl₃) *δ* (ppm) 175.05, 157.87, 151.33, 145.10, 139.90, 138.94, 129.26, 128.38, 127.16, 126.91, 124.69, 123.09, 121.06, 120.80, 117.89, 115.88, 112.75, 62.75, 62.42, 55.92, 50.10, 42.52, 34.70, 29.07, 29.05; HRMS (ESI-Q-TOF): *m*/*z* [M+H]⁺ calcd. for C₂₉H₃₂N₂O₃: 457.24855; found: 457.2481.

### The preparation of 8-(3-(2-methoxyphenoxy)benzyl)-1-phenethyl-1,8-diazaspiro[4.5]decan-2-one (compound 6b)

This compound was synthesized via general procedure 1 affording compound **6b** as a yellow oil, in 6% yield.

¹H NMR (400 MHz, CDCl₃): δ (ppm) 7.32-7.19 (m, 6H), 7.13-7.09 (m, 1H), 7.01-6.89 (m, 5H), 6.81 (dd, *J* = 2.4, 8.0 Hz, 1H), 3.84 (s, 3H), 3.47 (s, 2H), 3.32 (t, *J* = 8.0 Hz, 2H), 2.89-2.81 (m, 4H), 2.37 (t, *J* = 8.0 Hz, 2H), 2.10-2.04 (m, 2H), 1.90-1.81 (m, 4H), 1.31-1.28 (m, 2H); ¹³C NMR (101 MHz, CDCl₃) *δ* (ppm) 174.79, 157.86, 151.32, 145.19, 139.97, 139.19, 129.29, 128.95, 128.43, 126.34, 124.67, 123.16, 121.06, 120.73, 118.05, 115.94, 112.75, 62.51, 62.29, 55.95, 50.16, 41.49, 35.77, 34.75, 29.08, 28.87; HRMS (ESI-Q-TOF): *m*/*z* [M+H]⁺ calcd. for C₃₀H₃₄N₂O₃: 471.26420; found: 471.2637.

### The preparation of 2-benzyl-8-(3-(2-methoxyphenoxy)benzyl)-2,8-diazaspiro[4.5]decan-1-one (compound 6c)

This compound was synthesized via general procedure 1 affording compound **6c** as a yellow oil, in 59% yield.

¹H NMR (400 MHz, CDCl₃): δ (ppm) 7.33-7.27 (m, 3H), 7.24-7.19 (m, 3H), 7.14-7.10 (m, 1H), 7.05-6.99 (m, 2H), 6.97-6.89 (m, 3H), 6.81 (dd, *J* = 2.4, 8.1 Hz, 1H), 4.44 (s, 2H), 3.84 (s, 3H), 3.48 (s, 2H), 3.13 (t*, J* = 7.0 Hz, 2H), 2.85-2.82 (m, 2H), 2.12-1.98 (m, 4H), 1.87 (t, *J* = 7.0 Hz, 2H), 1.41-1.38 (m, 2H); ¹³C NMR (101 MHz, CDCl₃) *δ* (ppm) 178.42, 157.79, 151.33, 145.22, 140.36, 136.68, 129.20, 128.64, 127.99, 127.47, 124.61, 123.16, 121.06, 120.74, 117.95, 115.85, 112.80, 62.88, 55.95, 49.75, 46.63, 43.15, 43.01, 32.45, 29.57; HRMS (ESI-Q-TOF): *m*/*z* [M+H]⁺ calcd. for C₂₉H₃₂N₂O₃: 457.24855; found: 457.2481.

### The preparation of 8-(3-(2-methoxyphenoxy)benzyl)-2-phenethyl-2,8-diazaspiro[4.5]decan-1-one (compound 6d)

This compound was synthesized via general procedure 1 affording compound **6d** as a colorless oil, in 20% yield.

¹H NMR (400 MHz, CDCl₃): δ (ppm) 7.29-7.17 (m, 6H), 7.14-7.10 (m, 1H), 7.04-6.99 (m, 2H), 6.97-6.89 (m, 3H), 6.80 (dd, *J* = 2.4, 8.0 Hz, 1H), 3.84 (s, 3H), 3.53 (t, *J* = 7.2 Hz, 2H), 3.46 (s, 2H), 3.10 (t, *J* = 6.9 Hz, 2H), 2.83 (t, *J* = 7.2 Hz, 2H), 2.80-2.77 (m, 2H), 2.09-2.03 (m, 2H), 1.91-1.84 (m, 2H), 1.80 (t, *J* = 6.9 Hz, 2H), 1.29-1.26 (m, 2H); ¹³C NMR (101 MHz, CDCl₃) *δ* (ppm) 178.27, 157.79, 151.33, 145.23, 140.31, 138.71, 129.21, 128.73, 128.44, 126.41, 124.62, 123.20, 121.07, 120.76, 117.98, 115.88, 112.80, 62.88, 55.96, 49.76, 44.21, 43.80, 42.87, 33.70, 32.37, 29.92; HRMS (ESI-Q-TOF): *m*/*z* [M+H]⁺ calcd. for C₃₀H₃₄N₂O₃: 471.26420; found: 471.2633.

### The preparation of 1-benzyl-8-(3-(2-methoxyphenoxy)benzyl)-1,8-diazaspiro[4.5]decane (compound 7a)

This compound was synthesized via general procedure 3 affording compound **7a** as a yellow oil, in 74% yield.

¹H NMR (400 MHz, CDCl₃): δ (ppm) 7.33-7.28 (m, 3H), 7.24-7.18 (m, 2H), 7.13-7.08 (m, 1H), 7.03-6.95 (m, 4H), 6.93-6.88 (m, 1H), 6.82 (dd, *J* = 2.5, 8.1 Hz, 1H), 3.83 (s, 3H), 3.62 (s, 2H), 3.50 (s, 2H), 2.90-2.87 (m, 2H), 2.64 (t, *J* = 6.5 Hz, 2H), 2.11-2..04 (m, 2H), 1.88-1.81 (m, 2H), 1.72-1.61 (m, 4H), 1.39-1.36 (m, 2H); ¹³C NMR (101 MHz, CDCl₃) *δ* (ppm) 157.78, 151.32, 145.23, 140.76, 140.20, 129.20, 128.55, 128.14, 126.56, 124.59, 123.27, 121.05, 120.76, 118.10, 115.83, 112.74, 62.67, 61.73, 55.92, 52.44, 51.68, 50.56, 34.07, 31.90, 20.83; HRMS (ESI-Q-TOF): *m*/*z* [M+H]⁺ calcd. for C₂₉H₃₄N₂O₂: 443.26928; found: 443.2690.

### The preparation of 8-(3-(2-methoxyphenoxy)benzyl)-1-phenethyl-1,8-diazaspiro[4.5]decane (compound 7b)

This compound was synthesized via general procedure 3 affording compound **7b** as a red oil, in 50% yield.

¹H NMR (400 MHz, CDCl₃): δ (ppm) 7.32-7.28 (m, 2H), 7.24-7.19 (m, 4H), 7.09-7.05 (m, 1H), 6.98-6.87 (m, 5H), 6.80 (dd, *J* = 2.0, 8.0 Hz, 1H), 3.82 (s, 3H), 3.46 (s, 2H), 3.13-2.96 (m, 4H), 2.87-2.84 (m, 4H), 2.08-2.02 (m, 2H), 1.96-1.90 (m, 4H), 1.84-1.78 (m, 2H), 1.54-1.47 (m, 2H); ¹³C NMR (101 MHz, CDCl₃) *δ* (ppm) 157.87, 151.33, 145.13, 139.93, 139.18, 129.28, 128.73, 128.54, 126.46, 124.66, 123.07, 121.06, 120.81, 117.86, 115.88, 112.75, 62.28, 55.94, 50.94, 50.90, 50.31, 50.22, 34.49, 32.93, 30.80, 20.37; HRMS (ESI-Q-TOF): *m*/*z* [M+H]⁺ calcd. for C₃₀H₃₆N₂O₂: 457.28493; found: 457.2846.

### The preparation of 2-benzyl-8-(3-(2-methoxyphenoxy)benzyl)-2,8-diazaspiro[4.5]decane (compound 7c)

This compound was synthesized via general procedure 3 affording compound 7c as a yellow oil, in 54% yield.

¹H NMR (400 MHz, CDCl₃): δ (ppm) 7.35-7.29 (m, 4H), 7.26-7.19 (m, 2H), 7.13-7.09 (m, 1H), 7.02-6.98 (m, 2H), 6.96-6.88 (m, 3H), 6.80 (dd, *J* = 1.8, 8.1 Hz, 1H), 3.83 (s, 3H), 3.63 (s, 2H), 3.44 (s, 2H), 2.62 (t, *J* = 6.8 Hz, 2H), 2.41 (s, 2H), 2.37-2.32 (m, 4H), 1.65-1.58 (m, 6H); ¹³C NMR (101 MHz, CDCl₃) *δ* (ppm) 157.74, 151.30, 145.26, 140.07, 138.46, 129.21, 128.78, 128.28, 127.06, 124.57, 123.36, 121.06, 120.71, 118.22, 115.88, 112.75, 65.49, 62.96, 60.44, 55.94, 53.43, 51.13, 39.90, 37.68, 36.87; HRMS (ESI-Q-TOF): *m*/*z* [M+H]⁺ calcd. for C₂₉H₃₄N₂O₂: 443.26928; found: 443.2685.

### The preparation of 8-(3-(2-methoxyphenoxy)benzyl)-2-phenethyl-2,8-diazaspiro[4.5]decane (compound 7d)

This compound was synthesized via general procedure 3 affording compound **7d** as a red oil, in 35% yield.

¹H NMR (400 MHz, CDCl₃): δ (ppm) 7.30-7.20 (m, 6H), 7.14-7.10 (m, 1H), 7.02-6.99 (m, 2H), 6.95-6.89 (m, 3H), 6.81 (dd, *J* = 1.8, 8.0 Hz, 1H), 3.83 (s, 3H), 3.47 (s, 2H), 2.95-2.86 (m, 6H), 2.66 (s, 2H), 2.42-2.36 (m, 4H), 1.75 (t, *J* = 6.9 Hz, 2H), 1.69-1.61 (m, 4H); ¹³C NMR (101 MHz, CDCl₃) *δ* (ppm) 157.80, 151.31, 145.19, 139.75, 138.93, 129.29, 128.67, 128.55, 126.45, 124.64, 123.34, 121.08, 120.76, 118.20, 115.95, 112.77, 65.22, 62.85, 58.31, 55.95, 53.58, 50.93, 40.00, 37.22, 36.16, 34.24; HRMS (ESI-Q-TOF): *m*/*z* [M+H]⁺ calcd. for C₃₀H₃₆N₂O₂: 457.28493; found: 457.2849.

The preparation of 1-(3-(2-methoxyphenoxy)benzyl)piperidin-4-one (compound **9**) This compound was synthesized via general procedure 2 affording compound **9** as a yellow oil, in 92% yield.
¹H NMR (400 MHz, CDCl₃): δ (ppm) 7.25 (t, *J* = 7.7 Hz, 1H), 7.16-7.11 (m, 1H), 7.04-6.97 (m, 4H), 6.94-6.90 (m, 1H), 6.83 (dd, *J* = 1.9, 8.1 Hz, 1H), 3.84 (s, 3H), 3.59 (s, 2H), 2.73 (t, *J* = 6.0 Hz, 4H), 2.44 (t, *J* = 6.1 Hz, 4H); ¹³C NMR (101 MHz, CDCl₃) *δ* (ppm) 209.28, 158.04, 151.39, 145.08, 140.08, 129.40, 124.78, 122.90, 121.10, 120.89, 117.70, 116.01, 112.79, 61.62, 55.95, 52.88, 41.33;

### The preparation of 8-(3-(2-methoxyphenoxy)benzyl)-4-phenyl-1,4,8-triazaspiro[4.5]decan-2-one (compound 10a)

This compound was synthesized via general procedure 4 affording compound **10a** as a white solid, in 52% yield.

Mp: 179.0-180.8 °C. ¹H NMR (400 MHz, CDCl₃): δ (ppm) 8.42 (br, 1H), 7.30 (t, *J* = 8.0 Hz, 2H), 7.24 (t, *J* = 8.0 Hz, 1H), 7.16-7.12 (m, 1H), 7.03-6.98 (m, 4H), 6.95-6.82 (m, 5H), 3.98 (s, 2H), 3.83 (s, 3H), 3.56 (s, 2H), 2.98-2.95 (m, 2H), 2.79-2.71 (m, 2H), 2.25-2.19 (m, 2H), 1.61-1.57 (m, 2H); ¹³C NMR (101 MHz, CDCl₃) *δ* (ppm) 170.72, 158.00, 151.40, 145.08, 143.31, 140.13, 129.31, 129.28, 124.76, 122.95, 121.10, 120.94, 118.80, 117.57, 115.89, 115.67, 112.76, 77.58, 62.31, 55.94, 51.13, 50.64, 34.54; HRMS (ESI-Q-TOF): *m*/*z* [M+H]⁺ calcd. for C₂₇H₂₉N₃O₃: 444.22815; found: 444.2283.

### The preparation of 4-benzyl-8-(3-(2-methoxyphenoxy)benzyl)-1,4,8-triazaspiro [4.5]decan-2-one (compound 10b)

This compound was synthesized via general procedure 4 affording compound **10b** as a white solid, in 37% yield.

Mp: 126.4-128.2 °C. ¹H NMR (400 MHz, CDCl₃): δ (ppm) 7.57 (br, 1H), 7.35-7.30 (m, 4H), 7.29-7.20 (m, 2H), 7.13-7.09 (m, 1H), 7.00-6.96 (m, 4H), 6.93-6.89 (m, 1H), 6.81 (dd, *J* = 1.7, 8.1 Hz, 1H), 3.83 (s, 3H), 3.73 (s, 2H), 3.51 (s, 2H), 3.20 (s, 2H), 2.96-2.93 (m, 2H), 2.15-2.00 (m, 4H), 1.67-1.64 (m, 2H); ¹³C NMR (101 MHz, CDCl₃) *δ* (ppm) 173.17, 157.90, 151.35, 145.17, 140.07, 138.23, 129.29, 128.48, 128.24, 127.32, 124.66, 123.06, 121.08, 120.83, 117.91, 115.89, 112.78, 76.31, 62.34, 55.96, 53.32, 52.19, 50.68, 33.51; HRMS (ESI-Q-TOF): *m*/*z* [M+H]⁺ calcd. for C₂₈H₃₁N₃O₃: 458.24380; found: 458.2436.

### The preparation of 8-(3-(2-methoxyphenoxy)benzyl)-4-phenethyl-1,4,8-triazaspiro[4.5]decan-2-one (compound 10c)

This compound was synthesized via general procedure 4 affording compound **10c** as a yellow solid, in 30% yield.

Mp: 51.4-53.0 °C. ¹H NMR (400 MHz, CDCl₃): δ (ppm) 7.53 (br, 1H), 7.31-7.26 (m, 2H), 7.23-7.19 (m, 4H), 7.11-7.07 (m, 1H), 6.99-6.95 (m, 4H), 6.92-6.88 (m, 1H), 6.80 (dd, *J* = 1.9*,* 8.0 Hz, 1H), 3.82 (s, 3H), 3.48 (s, 2H), 3.42 (s, 2H), 2.88-2.86 (m, 2H), 2.81 (t, *J* = 6.9 Hz, 2H), 2.74 (t, *J* = 6.9 Hz, 2H), 2.07-2.01 (m, 2H), 1.91-1.83 (m, 2H), 1.47-1.44 (m, 2H); ¹³C NMR (101 MHz, CDCl₃) *δ* (ppm) 173.14, 157.89, 151.34, 145.14, 140.11, 139.75, 129.27, 128.66, 128.36, 126.24, 124.66, 123.01, 121.07, 120.84, 117.83, 115.85, 112.77, 76.48, 62.24, 55.95, 53.54, 50.62, 49.80, 35.49, 33.22; HRMS (ESI-Q-TOF): *m*/*z* [M+H]⁺ calcd. for C₂₉H₃₃N₃O₃: 472.25945; found: 472.2592.

### The preparation of 6-(3-(2-methoxyphenoxy)benzyl)-1,2,6-triazaspiro[2.5]octane (compound 11)

An oven-dried screw cap test tube was added with a magnetic stir-bar and compound **9** (218.0 mg, 0.70 mmol, 1.00 eq). The tube was then evacuated and back-filled with argon. The evacuation/ backfill sequence was repeated with two additional times. The reaction tube was evacuated and backfilled with argon three times. Under counterflow of argon, the solvent anhydrous methanol (3.0 mL) was added. Then, the reaction mixture was stirred for 5 minutes at -78 °C before the addition of the ammonia (7.0 N in methanol, 0.3 mL). Afterwards, the reaction mixture was stirred for 45 minutes at - 78 °C before adding hydroxylamine-O-sulfonic acid (52.2 mg, 0.46 mmol, 0.66 eq) in anhydrous methanol (1 mL) drop by drop. The reaction mixture was stirred at -78 °C for additional 2 h and then stirred at room temperature overnight. At last, the reaction mixture was concentrated under reduced pressure affording the crude product. The crude residue was purified by chromatography on silica gel (12% of methanol in dichloromethane) to give compound **11** as a colorless oil (96.2 mg, 42% yield).

¹H NMR (400 MHz, CDCl₃): δ (ppm) 7.23 (t, *J* = 7.8 Hz, 1H), 7.15-7.11 (m, 1H), 7.03-6.96 (m, 4H), 6.94-6.89 (m, 1H), 6.82 (dd, *J* = 2.1, 8.0 Hz, 1H), 3.84 (s, 3H), 3.52 (s, 2H), 2.63-2.57 (m, 4H), 1.76-1.64 (m, 6H); ¹³C NMR (101 MHz, CDCl₃) *δ* (ppm) 157.90, 151.36, 145.15, 140.20, 129.40, 129.28, 124.68, 123.08, 121.07, 120.82, 117.91, 115.88, 112.76, 62.32, 56.38, 55.94, 51.86, 35.51.

### The preparation of 8-(3-(2-methoxyphenoxy)benzyl)-4-phenyl-1,2,4,8-tetraazaspiro[4.5]decan-3-one (compound 12a)

An oven-dried screw cap test tube was added with a magnetic stir-bar and compound **11** (30.0 mg, 0.09 mmol, 1.00 eq). The tube was then evacuated and back-filled with argon. The evacuation/ backfill sequence was repeated with two additional times. The reaction tube was evacuated and backfilled with argon three times. Under counterflow of argon, the solvent anhydrous dichloromethane (1.5 mL) was added. Then, the reaction mixture was stirred for 5 minutes at -20 °C before the addition of the phenyl isocyanate (12.1 mg, 0.10 mmol, 1.10 eq). The reaction mixture was stirred at -20 °C for additional 1 h and then stirred at room temperature overnight. At last, the reaction mixture was concentrated under reduced pressure affording the crude product. The crude residue was purified by chromatography on silica gel (2% of methanol in dichloromethane) to give compound **12a** as a white solid (24.1 mg, 59% yield).

Mp: 50.6-52.2 °C. ¹H NMR (400 MHz, CDCl₃): δ (ppm) 8.02 (s, 1H), 7.48 (d, *J* = 7.7 Hz, 2H), 7.31 (t, *J* = 7.9 Hz, 2H), 7.22 (t, *J* = 7.8 Hz, 1H), 7.15-7.07 (m, 2H), 7.02-6.89 (m, 5H), 6.81 (dd, *J* = 1.9, 8.1 Hz, 1H), 3.83 (s, 3H), 3.53 (d, *J* = 3.9 Hz, 2H), 2.78-2.72 (m, 2H), 2.63-2.52 (m, 3H), 1.97-1.70 (m, 4H); ¹³C NMR (101 MHz, CDCl₃) *δ* (ppm) 159.70, 157.92, 151.35, 145.14, 140.25, 137.14, 129.30, 129.05, 124.70, 124.15, 123.03, 121.09, 120.83, 119.15, 117.78, 115.94, 112.79, 65.06, 62.15, 55.95, 51.52, 51.15, 36.52, 30.28; HRMS (ESI-Q-TOF): *m*/*z* [M+H]⁺ calcd. for C₂₆H₂₈N₄O₃: 445.22340; found: 445.2225.

### The preparation of 4-benzyl-8-(3-(2-methoxyphenoxy)benzyl)-1,2,4,8-tetraazaspiro[4.5]decan-3-one (compound 12b)

This compound was synthesized via procedure of compound **12a** affording compound **12b** as a white solid, in 51% yield.

Mp: 131.8-133.2 °C. ¹H NMR (400 MHz, CDCl₃): δ (ppm) 7.34-7.26 (m, 5H), 7.22 (t, *J* = 7.9 Hz, 1H), 7.15-7.11 (m, 1H), 7.02-6.89 (m, 5H), 6.82 (dd, *J =* 2.1, 8.0 Hz, 1H), 6.41 (t, *J* = 5.5 Hz, 1H), 4.46-4.34 (m, 2H), 3.83 (s, 3H), 3.52 (s, 2H), 2.76-2.70 (m, 2H), 2.56-2.48 (m, 2H), 2.34 (s, 1H), 1.94-1.63 (m, 4H); ¹³C NMR (101 MHz, CDCl₃) *δ* (ppm) 162.33, 157.91, 151.36, 145.14, 140.34, 138.11, 129.28, 128.69, 127.73, 127.56, 124.69, 123.03, 121.09, 120.83, 117.78, 115.92, 112.78, 64.09, 62.16, 55.94, 51.58, 51.15, 44.22, 36.58, 30.03; HRMS (ESI-Q-TOF): *m*/*z* [M+H]⁺ calcd. for C₂₇H₃₀N₄O₃: 459.23905; found: 459.2386.

### The preparation of tert-butyl 1-benzyl-2-oxo-1,8-diazaspiro[4.5]decane-8-carboxylate (compound 14)

This compound was synthesized via general procedure 1 affording compound **14** as a white solid, in 94% yield.

¹H NMR (400 MHz, CDCl₃): δ (ppm) 7.31-7.21 (m, 5H), 4.43 (s, 2H), 4.08-4.04 (m, 2H), 2.80-2.73 (m, 2H), 2.51 (t, *J* = 8.0 Hz, 2H), 2.01 (t, *J* = 8.0 Hz, 2H), 1.74-1.69 (m, 2H), 1.44 (s, 9H), 1.34-1.30 (m, 2H); ¹³C NMR (101 MHz, CDCl₃) *δ* (ppm) 174.81, 154.61, 138.70, 128.50, 127.21, 127.11, 79.93, 62.69, 42.62, 40.63, 34.51, 28.97, 28.38, 28.29;

### The preparation of 1-benzyl-8-(2-isobutoxybenzyl)-1,8-diazaspiro[4.5]decan-2-one (compound 15)

This compound was synthesized via general procedure 2 affording compound **15** as a colorless oil, in 73% yield.

¹H NMR (400 MHz, CDCl₃): δ (ppm) 7.29-7.25 (m, 5H), 7.22-7.18 (m, 2H), 6.92-6.88 (m, 1H), 6.82 (d, *J* = 7.9 Hz, 1H), 4.44 (s, 2H), 3.71 (d, *J* = 6.4 Hz, 2H), 3.56 (s, 2H), 2.86-2.83 (m, 2H), 2.47 (t, *J* = 8.0 Hz, 2H), 2.17-2.03 (m, 3H), 1.97-1.88 (m, 4H), 1.31-1.28 (m, 2H), 1.03 (d, *J* = 6.7 Hz, 6H); ¹³C NMR (101 MHz, CDCl₃) *δ* (ppm) 175.07, 157.33, 139.01, 130.51, 128.36, 128.13, 127.16, 126.87, 126.12, 120.04, 111.27, 74.35, 62.82, 55.88, 50.14, 42.52, 34.81, 29.10, 29.09, 28.44, 19.40; HRMS (ESI-Q-TOF): *m*/*z* [M+H]⁺ calcd. for C₂₆H₃₄N₂O₂: 407.26928; found: 407.2691.

### The preparation of tert-butyl 3-oxo-1-oxa-4,9-diazaspiro[5.5]undecane-9-carboxylate (compound 17)

This compound was synthesized via general procedure 5 affording compound **17** as a white solid, in 55% yield.
¹H NMR (400 MHz, CDCl₃): δ (ppm) 6.87-6.77 (br, 1H), 4.16 (s, 2H), 3.85-3.83 (m, 2H), 3.24 (d, *J* = 2.6 Hz, 2H), 3.15-3.09 (m, 2H), 1.91-1.85 (m, 2H), 1.56-1.49 (m, 2H), 1.46 (s, 9H);

### The preparation of 9-(3-(2-methoxybenzyl)benzyl)-1-oxa-4,9-diazaspiro[5.5]undecan-3-one (compound 18)

This compound was synthesized via general procedure 2 affording compound **18** as a colorless oil, in 70% yield.
¹H NMR (400 MHz, CDCl₃): δ (ppm) 7.22 (t, *J* = 7.8 Hz, 1H), 7.14-7.10 (m, 1H), 7.01-6.89 (m, 5H), 6.81 (dd, *J* = 1.8, 8.1 Hz, 1H), 6.73-6.71 (br, 1H), 4.14 (s, 2H), 3.84 (s, 3H), 3.49 (s, 2H), 3.22 (d, *J* = 2.6 Hz, 2H), 2.60-2.57 (m, 2H), 2.37-2.31 (m, 2H), 1.91-1.87 (m, 2H), 1.68-1.61 (m, 2H); ¹³C NMR (101 MHz, CDCl₃) *δ* (ppm) 169.12, 157.85, 151.32, 145.21, 140.13, 129.29, 124.64, 123.22, 121.08, 120.75, 118.10, 115.91, 112.77, 68.65, 62.64, 62.20, 55.95, 50.31, 48.65, 31.90;

### The preparation of 4-benzyl-9-(3-(2-methoxyphenoxy)benzyl)-1-oxa-4,9-diazaspiro[5.5]undecan-3-one (compound 19a)

This compound was synthesized via general procedure 1 affording compound **19a** as a colorless oil, in 95% yield.

¹H NMR (400 MHz, CDCl₃): δ (ppm) 7.36-7.29 (m, 3H), 7.26-7.22 (m, 2H), 7.20 (t, *J* = 7.8 Hz, 1H), 7.13-7.09 (m, 1H), 7.00-6.88 (m, 5H), 6.79 (dd, *J* = 2.5, 8.1 Hz, 1H), 4.59 (s, 2H), 4.21 (s, 2H), 3.82 (s, 3H), 3.43 (s, 2H), 3.06 (s, 2H), 2.48-2.45 (m, 2H), 2.31-2.25 (m, 2H), 1.81-1.77 (m, 2H), 1.50-1.43 (m, 2H); ¹³C NMR (101 MHz, CDCl₃) *δ* (ppm) 166.76, 157.82, 151.32, 145.20, 140.25, 136.10, 129.24, 128.74, 128.34, 127.81, 124.62, 123.14, 121.06, 120.74, 118.02, 115.85, 112.76, 69.25, 62.59, 62.38, 55.94, 54.07, 49.50, 48.51, 31.93; HRMS (ESI-Q-TOF): *m*/*z* [M+H]⁺ calcd. for C₂₉H₃₂N₂O₄: 473.24346; found: 473.2426.

### The preparation of 9-(3-(2-methoxyphenoxy)benzyl)-4-phenethyl-1-oxa-4,9-diazaspiro[5.5]undecan-3-one (compound 19b)

This compound was synthesized via general procedure 1 affording compound **19b** as a colorless oil, in 27% yield.

¹H NMR (400 MHz, CDCl₃): δ (ppm) 7.31-7.27 (m, 2H), 7.24-7.20 (m, 4H), 7.14-7.10 (m, 1H), 7.02-6.89 (m, 5H), 6.81 (dd, *J* = 2.4, 7.4 Hz, 1H), 4.10 (s, 2H), 3.84 (s, 3H), 3.61 (t, *J* = 7.5 Hz, 2H), 3.46 (s, 2H), 3.05 (s, 2H), 2.88 (t, *J* = 7.5 Hz, 2H), 2.51-2.48 (m, 4H), 2.32-2.26 (m, 2H), 1.79-1.75 (m, 2H), 1.54-1.47 (m, 2H); ¹³C NMR (101 MHz, CDCl₃) *δ* (ppm) 166.71, 157.82, 151.31, 145.22, 140.17, 138.40, 129.27, 128.80, 128.59, 126.59, 124.62, 123.22, 121.07, 120.72, 118.12, 115.89, 112.77, 69.16, 62.64, 62.33, 55.95, 55.89, 48.56, 48.51, 33.44, 32.01; HRMS (ESI-Q-TOF): *m*/*z* [M+H]⁺ calcd. for C₃₀H₃₄N₂O₄: 487.25911; found: 487.2586.

### The preparation of 4-benzyl-9-(3-(2-methoxyphenoxy)benzyl)-1-oxa-4,9-diazaspiro[5.5]undecane (compound 20a)

This compound was synthesized via general procedure 3 affording compound **20a** as a red oil, in 77% yield.

¹H NMR (400 MHz, CDCl₃): δ (ppm) 7.30-7.28 (m, 4H), 7.25-7.18 (m, 2H), 7.12-7.08 (m, 1H), 7.02-6.93 (m, 4H), 6.92-6.88 (m, 1H), 6.80 (dd, *J* = 2.4, 7.4 Hz, 1H), 3.82 (s, 3H), 3.71 (t, *J* = 4.9 Hz, 2H), 3.46 (s, 2H), 3.42 (s, 2H), 2.50-2.47 (m, 2H), 2.41-2.32 (m, 4H), 2.20 (s, 2H), 1.97-1.94 (m, 2H), 1.58-1.51 (m, 2H); ¹³C NMR (101 MHz, CDCl₃) *δ* (ppm) 157.75, 151.31, 145.24, 140.26, 138.27, 129.21, 128.67, 128.21, 126.99, 124.56, 123.31, 121.05, 120.74, 118.15, 115.84, 112.74, 70.26, 62.99, 62.70, 62.17, 60.54, 55.92, 53.82, 48.85, 32.84; HRMS (ESI-Q-TOF): *m*/*z* [M+H]⁺ calcd. for C₂₉H₃₄N₂O₃: 459.26420; found: 459.2646.

### The preparation of 9-(3-(2-methoxyphenoxy)benzyl)-4-phenethyl-1-oxa-4,9-diazaspiro[5.5]undecane (compound 20b)

This compound was synthesized via general procedure 3 affording compound **20b** as a red oil, in 73% yield.

¹H NMR (400 MHz, CDCl₃): δ (ppm) 7.29-7.25 (m, 2H), 7.24-7.17 (m, 4H), 7.14-7.09 (m, 1H), 7.04-6.95 (m, 4H), 6.93-6.89 (m, 1H), 6.81 (dd, *J* = 1.8, 8.1 Hz, 1H), 3.83 (s, 3H), 3.70 (t, *J* = 4.8 Hz, 2H), 3.49 (s, 2H), 2.75 (t, *J* = 7.8 Hz, 2H), 2.53-2.49 (m, 4H), 2.44-2.33 (m, 4H), 2.28 (s, 2H), 1.94-1.90 (m, 2H), 1.62-1.55 (m, 2H); ¹³C NMR (101 MHz, CDCl₃) *δ* (ppm) 157.77, 151.32, 145.25, 140.39, 140.22, 129.23, 128.72, 128.29, 125.96, 124.57, 123.38, 121.06, 120.75, 118.21, 115.88, 112.75, 70.11, 62.77, 62.46, 60.53, 60.48, 55.94, 53.98, 48.88, 33.21, 32.97; HRMS (ESI-Q-TOF): *m*/*z* [M+H]⁺ calcd. for C₃₀H₃₆N₂O₃: 473.27985; found: 473.2785.

### The preparation of 4-(4-fluorobenzyl)-9-(3-(2-methoxyphenoxy)benzyl)-1-oxa-4,9-diazaspiro[5.5]undecan-3-one (compound 21a)

This compound was synthesized via general procedure 1 affording compound **21a** as a brown oil, in 53% yield.

¹H NMR (400 MHz, CDCl₃): δ (ppm) 7.25-7.19 (m, 3H), 7.14-7.09 (m, 1H), 7.06-6.88 (m, 7H), 6.79 (dd, *J* = 2.5, 8.1 Hz, 1H), 4.55 (s, 2H), 4.20 (s, 2H), 3.83 (s, 3H), 3.45 (s, 2H), 3.05 (s, 2H), 2.50-2.47 (m, 2H), 2.31-2.26 (m, 2H), 1.80-1.77 (m, 2H), 1.50-1.43 (m, 2H); ¹³C NMR (101 MHz, CDCl₃) *δ* (ppm) 166.76, 162.39 (d, ¹*J_{CF}* = 246.4 Hz), 157.82, 151.31, 145.22, 140.22, 131.97 (d, ⁴*J_{CF}* = 3.3 Hz), 130.08 (d, ³*J_{CF}* = 8.2 Hz), 129.27, 124.61, 123.17, 121.06, 120.70, 118.08, 115.90, 115.67 (d, ²*J_{CF} =* 21.7 Hz), 112.74, 69.22, 62.56, 62.37, 55.94, 54.15, 48.85, 48.47, 31.92; HRMS (ESI-Q-TOF): *m*/*z* [M+H]⁺ calcd. for C₂₉H₃₁FN₂O₄: 491.23404; found: 491.2349.

### The preparation of 4-(3-fluorobenzyl)-9-(3-(2-methoxyphenoxy)benzyl)-1-oxa-4,9-diazaspiro[5.5]undecan-3-one (compound 21b)

This compound was synthesized via general procedure 1 affording compound **21b** as a brown oil, in 64% yield.

¹H NMR (400 MHz, CDCl₃): δ (ppm) 7.34-7.28 (m, 1H), 7.21 (t, *J* = 7.8 Hz, 1H), 7.13-7.09 (m, 1H), 7.04-6.88 (m, 8H), 6.79 (dd, *J* = 2.5, 8.1 Hz, 1H), 4.58 (s, 2H), 4.21 (s, 2H), 3.83 (s, 3H), 3.45 (s, 2H), 3.07 (s, 2H), 2.51-2.48 (m, 2H), 2.32-2.26 (m, 2H), 1.82-1.79 (m, 2H), 1.53-1.45 (m, 2H); ¹³C NMR (101 MHz, CDCl₃) *δ* (ppm) 166.85, 163.00 (d, ¹*J_{CF}* = 247.1 Hz), 157.83, 151.31, 145.19, 140.21, 138.73 (d, *³J_{CF} =* 7.1 Hz), 130.31 (d, ³*J_{CF}* = 8.3 Hz), 129.26, 124.62, 123.84 (d, ⁴*J_{CF}* = 2.9 Hz), 123.13, 121.05, 120.74, 118.02, 115.86, 115.15 (d, ²*J_{CF}* = 21.7 Hz), 114.81 (d, ²*J_{CF}* = 20.9 Hz), 112.74, 69.23, 62.56, 62.35, 55.93, 54.42, 49.14 (d, ⁴*J_{CF} =* 1.2 Hz), 48.47, 31.94; HRMS (ESI-Q-TOF): *m*/*z* [M+H]⁺ calcd. for C₂₉H₃₁FN₂O₄: 491.23404; found: 491.2328.

### The preparation of 4-(2-fluorobenzyl)-9-(3-(2-methoxyphenoxy)benzyl)-1-oxa-4,9-diazaspiro[5.5]undecan-3-one (compound 21c)

This compound was synthesized via general procedure 1 affording compound **21c** as a brown oil, in 56% yield.

¹H NMR (400 MHz, CDCl₃): δ (ppm) 7.36-7.32 (m, 1H), 7.31-7.25 (m, 1H), 7.21 (t, *J* = 7.8 Hz, 1H), 7.14-7.10 (m, 2H), 7.08-7.03 (m, 1H), 7.01-6.88 (m, 5H), 6.79 (dd, *J* = 2.5, 7.4 Hz, 1H), 4.66 (s, 2H), 4.19 (s, 2H), 3.83 (s, 3H), 3.45 (s, 2H), 3.13 (s, 2H), 2.51-2.48 (m, 2H), 2.32-2.26 (m, 2H), 1.82-1.78 (m, 2H), 1.54-1.47 (m, 2H); ¹³C NMR (101 MHz, CDCl₃) *δ* (ppm) 166.87, 161.16 (d, ¹*J_{CF}* = 246.4 Hz), 157.82, 151.31, 145.20, 140.24, 130.90 (d, *³J_{CF} =* 4.1 Hz), 129.64 (d*,* ³*J_{CF}* = 8.1 Hz), 129.26, 124.62, 124.55 (d*,* ⁴*J_{CF}* = 3.6 Hz), 123.17, 123.02, 121.06, 120.73, 118.06, 115.87, 115.44 (d, ²*J_{CF} =* 21.6 Hz), 112.75, 69.28, 62.58, 62.41, 55.94, 54.57, 48.51, 43.04 (d, *³J_{CF}* = 3.6 Hz), 31.89; HRMS (ESI-Q-TOF): *m*/*z* [M+H]⁺ calcd. for C₂₉H₃₁FN₂O₄: 491.23404; found: 491.2331.

### The preparation of 4-(4-chlorobenzyl)-9-(3-(2-methoxyphenoxy)benzyl)-1-oxa-4,9-diazaspiro[5.5]undecan-3-one (compound 21d)

This compound was synthesized via general procedure 1 affording compound **21d** as a green oil, in 51% yield.

¹H NMR (400 MHz, CDCl₃): δ (ppm) 7.33-7.29 (m, 2H), 7.23-7.18 (m, 3H), 7.14-7.09 (m, 1H), 7.01-6.88 (m, 5H), 6.80 (dd, *J* = 2.4, 8.1 Hz, 1H), 4.55 (s, 2H), 4.20 (s, 2H), 3.83 (s, 3H), 3.45 (s, 2H), 3.05 (s, 2H), 2.55-2.48 (m, 2H), 2.31-2.26 (m, 2H), 1.80-1.77 (m, 2H), 1.51-1.44 (m, 2H); ¹³C NMR (101 MHz, CDCl₃) *δ* (ppm) 166.81, 157.82, 151.30, 145.20, 140.16, 134.69, 133.73, 129.71, 129.27, 128.94, 124.61, 123.16, 121.05, 120.71, 118.06, 115.90, 112.74, 69.20, 62.53, 62.35, 55.94, 54.32 ,48.94, 48.44, 31.90; HRMS (ESI-Q-TOF): *m*/*z* [M+H]⁺ calcd. for C₂₉H₃₁ClN₂O₄: 507.20449; found: 507.2044.

### The preparation of 4-(3-chlorobenzyl)-9-(3-(2-methoxyphenoxy)benzyl)-1-oxa-4,9-diazaspiro[5.5]undecan-3-one (compound 21e)

This compound was synthesized via general procedure 1 affording compound **21e** as a brown oil, in 55% yield.

¹H NMR (400 MHz, CDCl₃): δ (ppm) 7.28-7.25 (m, 3H), 7.21 (t, *J* = 7.8 Hz, 1H), 7.15-7.09 (m, 2H), 7.01-6.88 (m, 5H), 6.80 (dd, *J* = 1.8, 8.1 Hz, 1H), 4.56 (s, 2H), 4.21 (s, 2H), 3.83 (s, 3H), 3.45 (s, 2H), 3.07 (s, 2H), 2.51-2.49 (m, 2H), 2.32-2.27 (m, 2H), 1.82-1.79 (m, 2H), 1.53-1.46 (m, 2H); ¹³C NMR (101 MHz, CDCl₃) *δ* (ppm) 166.86, 157.83, 151.31, 145.19, 140.13, 138.23, 134.64, 130.07, 129.27, 128.34, 128.06, 126.39, 124.63, 123.16, 121.06, 120.74, 118.04, 115.89, 112.75, 69.24, 62.56, 62.35, 55.94, 54.41, 49.09, 48.47, 31.92; HRMS (ESI-Q-TOF): *m*/*z* [M+H]⁺ calcd. for C₂₉H₃₁ClN₂O₄: 507.20449; found: 507.2047.

### The preparation of 4-(2-chlorobenzyl)-9-(3-(2-methoxyphenoxy)benzyl)-1-oxa-4,9-diazaspiro[5.5]undecan-3-one (compound 21f)

This compound was synthesized via general procedure 1 affording compound **21f** as a brown oil, in 35% yield.

¹H NMR (400 MHz, CDCl₃): δ (ppm) 7.39-7.36 (m, 1H), 7.32-7.30 (m, 1H), 7.27-7.19 (m, 3H), 7.14-7.10 (m, 1H), 7.01-6.88 (m, 5H), 6.80 (dd, *J* = 2.0, 8.0 Hz, 1H), 4.76 (s, 2H), 4.22 (s, 2H), 3.83 (s, 3H), 3.45 (s, 2H), 3.10 (s, 2H), 2.51-2.49 (m, 2H), 2.33-2.28 (m, 2H), 1.85-1.82 (m, 2H), 1.55-1.50 (m, 2H); ¹³C NMR (101 MHz, CDCl₃) *δ* (ppm) 166.94, 157.83, 151.32, 145.20, 140.17, 133.89, 133.58, 129.98, 129.72, 129.28, 129.13, 127.26, 124.63, 123.19, 121.06, 120.74, 118.09, 115.89, 112.75, 69.30, 62.57, 62.43, 55.95, 54.59, 48.51, 46.74, 31.96; HRMS (ESI-Q-TOF): *m*/*z* [M+H]⁺ calcd. for C₂₉H₃₁ClN₂O₄: 507.20449; found: 507.2035.

### The preparation of 4-((9-(3-(2-methoxyphenoxy)benzyl)-3-oxo-1-oxa-4,9-diazaspiro[5.5]undecan-4-yl)methyl)benzonitrile (compound 21g)

This compound was synthesized via general procedure 1 affording compound **21g** as a yellow solid, in 56% yield.

Mp: 47.2-49.0 °C.¹H NMR (400 MHz, CDCl₃): δ (ppm) 7.66-7.63 (m, 2H), 7.39-7.36 (m, 2H), 7.21 (t, *J* = 7.8 Hz, 1H), 7.14-7.10 (m, 1H), 7.01-6.88 (m, 5H), 6.79 (dd, *J* = 2.0, 8.0 Hz, 1H), 4.63 (s, 2H), 4.22 (s, 2H), 3.84 (s, 3H), 3.46 (s, 2H), 3.08 (s, 2H), 2.54-2.51 (m, 2H), 2.32-2.27 (m, 2H), 1.83-1.80 (m, 2H), 1.54-1.47 (m, 2H); ¹³C NMR (101 MHz, CDCl₃) *δ* (ppm) 167.08, 157.81, 151.28, 145.21, 141.69, 140.07, 132.60, 129.29, 128.79, 124.61, 123.18, 121.05, 120.64, 118.48, 118.13, 115.93, 112.75, 111.84, 69.21, 62.52, 62.32, 55.94, 54.99, 49.50, 48.39, 31.93; HRMS (ESI-Q-TOF): *m*/*z* [M+H]⁺ calcd. for C₃₀H₃₁N₃O₄: 498.23871; found: 498.2396.

### The preparation of 3-((9-(3-(2-methoxyphenoxy)benzyl)-3-oxo-1-oxa-4,9-diazaspiro[5.5]undecan-4-yl)methyl)benzonitrile (compound 21h)

This compound was synthesized via general procedure 1 affording compound **21h** as a yellow solid, in 63% yield.

Mp: 31.6-33.4 °C.¹H NMR (400 MHz, CDCl₃): δ (ppm) 7.61-7.51 (m, 3H), 7.46 (t, *J* = 7.6 Hz, 1H), 7.21 (t, *J* = 7.8 Hz, 1H), 7.14-7.10 (m, 1H), 7.01-6.88 (m, 5H), 6.79 (dd, *J* = 1.9, 8.1 Hz, 1H), 4.61 (s, 2H), 4.22 (s, 2H), 3.83 (s, 3H), 3.46 (s, 2H), 3.08 (s, 2H), 2.53-2.50 (m, 2H), 2.32-2.27 (m, 2H), 1.83-1.80 (m, 2H), 1.53-1.46 (m, 2H); ¹³C NMR (101 MHz, CDCl₃) *δ* (ppm) 167.06, 157.82, 151.30, 145.20, 140.12, 137.93, 132.59, 131.65, 131.60, 129.70, 129.28, 124.62, 123.16, 121.06, 120.70, 118.41, 118.08, 115.90, 112.94, 112.75, 69.22, 62.55, 62.32, 55.94, 54.87, 49.16, 48.41, 31.94; HRMS (ESI-Q-TOF): *m*/*z* [M+H]⁺ calcd. for C₃₀H₃₁N₃O₄: 498.23871; found: 498.2395.

### The preparation of 2-((9-(3-(2-methoxyphenoxy)benzyl)-3-oxo-1-oxa-4,9-diazaspiro[5.5]undecan-4-yl)methyl)benzonitrile (compound 21i)

This compound was synthesized via general procedure 1 affording compound **21i** as a yellow solid, in 53% yield.

Mp: 40.5-42.1 °C.¹H NMR (400 MHz, CDCl₃): δ (ppm) 7.67 (d, *J* = 7.7 Hz, 1H), 7.62-7.57 (m, 1H), 7.49 (d, *J* = 7.8 Hz, 1H), 7.43-7.39 (m, 1H), 7.21 (t, *J* = 7.8 Hz, 1H), 7.14-7.10 (m, 1H), 7.01-6.88 (m, 5H), 6.80 (dd, *J* = 2.0, 8.1 Hz, 1H), 4.84 (s, 2H), 4.23 (s, 2H), 3.83 (s, 3H), 3.46 (s, 2H), 3.16 (s, 2H), 2.54-2.51 (m, 2H), 2.35-2.28 (m, 2H), 1.85-1.82 (m, 2H), 1.56-1.51 (m, 2H); ¹³C NMR (101 MHz, CDCl₃) *δ* (ppm) 167.17, 157.84, 151.32, 145.19, 140.08, 133.44, 132.87, 129.30, 128.71, 128.40, 128.32, 124.64, 123.17, 121.07, 120.73, 118.06, 117.45, 115.92, 112.76, 112.34, 69.31, 62.53, 62.36, 55.95, 55.11, 48.44, 47.65, 31.93; HRMS (ESI-Q-TOF): *m*/*z* [M+H]⁺ calcd. for C₃₀H₃₁N₃O₄: 498.23871; found: 498.2385.

The preparation of 9-(3-(2-methoxyphenoxy)benzyl)-4-(pyridin-2-ylmethyl)-1-oxa-4,9-diazaspiro[5.5]undecan-3-one (compound **21j**)

This compound was synthesized via general procedure 1 affording compound **21j** as a colorless oil, in 59% yield.

¹H NMR (400 MHz, CDCl₃): δ (ppm) 8.54-8.52 (m, 1H), 7.69-7.65 (m, 1H), 7.32 (d, *J* = 7.8 Hz, 1H), 7.23-7.19 (m, 2H), 7.14-7.10 (m, 1H), 7.01-6.88 (m, 5H), 6.80 (dd, *J* = 2.4, 8.1 Hz, 1H), 4.70 (s, 2H), 4.20 (s, 2H), 3.83 (s, 3H), 3.46 (s, 2H), 3.28 (s, 2H), 2.52-2.49 (m, 2H), 2.34-2.29 (m, 2H), 1.83-1.80 (m, 2H), 1.58-1.51 (m, 2H); ¹³C NMR (101 MHz, CDCl₃) *δ* (ppm) 166.84, 157.84, 156.31, 151.32, 149.32, 145.18, 140.06, 136.97, 129.29, 124.64, 123.21, 122.72, 122.67, 121.07, 120.74, 118.09, 115.91, 112.75, 69.35, 62.57, 62.43, 55.94, 55.18, 51.61, 48.55, 31.93; HRMS (ESI-Q-TOF): *m*/*z* [M+H]⁺ calcd. for C₂₈H₃₁N₃O₄: 474.23871; found: 474.2379.

### The preparation of tert-butyl 4-amino-4-(hydroxymethyl)piperidine-1-carboxylate (compound 23)

Compound **22** (1.90 g, 7.78 mmol, 1.00 eq) was added to a solution of NaBH₄ (0.71 g, 18.67 mmol, 2.40 eq) in THF (40.0 mL). Then, the reaction mixture was stirred at 0 °C for 5 minutes before adding I₂ (1.97 g, 7.78 mmol, 1.00 eq) in THF (10.0 mL) dropwise. After 10 minutes, the reaction mixture was stirred at 80 °C for further 10 h. Then, the reaction mixture was quenched with methanol (10.0 mL). Afterwards, it was concentrated under reduced pressure affording the crude product. The crude product was put into an aqueous solution of KOH (20%, 100.0 mL) and stirred at room temperature for 2 h. After this, it was extracted with dichloromethane (150.0 mL). The organic layer was collected and and dried over magnesium sulfate. Then, it was concentrated under reduced pressure affording the compound **23** as a white solid (1.75 g, 98% yield). ¹H NMR (400 MHz, CDCl₃): δ (ppm) 3.67-3.63 (m, 2H), 3.36 (s, 2H), 3.30-3.24 (m, 2H), 1.57-1.50 (m, 2H), 1.46 (s, 9H), 1.42-1.37 (m, 2H);

### The preparation of tert-butyl 2-oxo-4-oxa-1,9-diazaspiro[5.5]undecane-9-carboxylate (compound 24)

This compound was synthesized via general procedure 5 affording compound **24** as a white solid, in 59% yield. ¹H NMR (400 MHz, CDCl₃): δ (ppm) 7.01 (br, 1H), 4.17 (s, 2H), 3.66 (s, 2H), 3.49-3.46 (m, 4H), 1.76-1.65 (m, 4H), 1.46 (s, 9H);

### The preparation of 9-(3-(2-methoxybenzyl)benzyl)-4-oxa-1,9-diazaspiro[5.5]undecan-2-one (compound 25)

This compound was synthesized via general procedure 2 affording compound **25** as a yellow solid, in 78% yield.

¹H NMR (400 MHz, CDCl₃): δ (ppm) 7.22 (t, *J* = 7.8 Hz, 1H), 7.15-7.11 (m, 1H), 7.02-6.89 (m, 5H), 6.81 (dd, *J* = 1.9, 8.1 Hz, 1H), 6.62 (br, 1H), 4.15 (s, 2H), 3.83 (s, 3H), 3.62 (s, 2H), 3.49 (s, 2H), 2.50-2.40 (m, 4H), 1.80-1.68 (m, 4H); ¹³C NMR (101 MHz, CDCl₃) *δ* (ppm) 168.73, 157.91, 151.35, 145.11, 139.84, 129.33, 124.71, 123.08, 121.08, 120.82, 117.94, 115.91, 112.78, 71.75, 67.85, 62.58, 55.95, 52.11, 48.81, 34.80;

### The preparation of 1-benzyl-9-(3-(2-methoxyphenoxy)benzyl)-4-oxa-1,9-diazaspiro[5.5]undecan-2-one (compound 26)

This compound was synthesized via general procedure 1 affording compound **26** as a yellow oil, in 40% yield.

¹H NMR (400 MHz, CDCl₃): δ (ppm) 7.31-7.27 (m, 2H), 7.23-7.19 (m, 4H), 7.15-7.11 (m, 1H), 7.01-6.89 (m, 5H), 6.80 (dd, *J* = 1.7, 8.2 Hz, 1H), 4.69 (s, 2H), 4.32 (s, 2H), 3.87 (s, 2H), 3.81 (s, 3H), 3.45 (s, 2H), 2.75-2.73 (m, 2H), 2.01-1.96 (m, 4H), 1.63-1.60 (m, 2H); ¹³C NMR (101 MHz, CDCl₃) *δ* (ppm) 168.36, 157.95, 151.38, 145.03, 139.74, 138.69, 129.30, 128.46, 126.76, 126.38, 124.76, 123.04, 121.09, 120.91, 117.75, 115.93, 112.77, 69.19, 68.32, 62.35, 57.81, 55.92, 49.83, 43.66, 31.30; HRMS (ESI-Q-TOF): *m*/*z* [M+H]⁺ calcd. for C₂₉H₃₂N₂O₄: 473.24346; found: 473.2433.

### The preparation of tert-butyl 2-methyl-3-oxo-1-oxa-4,9-diazaspiro[5.5]undecane-9-carboxylate (compound 27)

Compound **27** was synthesized as a white solid (162.5mg, 52% yield) based on the reported procedure and the spectral data of target compound **27** was consistent with previous record[3].

### The preparation of 9-(3-(2-methoxyphenoxy)benzyl)-2-methyl-1-oxa-4,9-diazaspiro[5.5]undecan-3-one (compound 28)

This compound was synthesized via the same procedure as compound **18** affording compound **28** as a white solid, in 60% yield.

¹H NMR (400 MHz, CDCl₃): δ (ppm) 7.23 (t, *J* = 7.8 Hz, 1H), 7.15-7.10 (m, 1H), 7.03-6.99 (m, 2H), 6.97-6.89 (m, 3H), 6.82 (dd, *J* = 1.9, 8.1 Hz, 1H), 6.15 (br, 1H), 4.19 (q, *J* = 6.8 Hz, 1H), 3.84 (s, 3H), 3.52 (s, 2H), 3.35 (d, *J* = 11.9 Hz, 1H), 3.10 (dd, *J* = 4.8, 11.9 Hz, 1H), 2.63-2.47 (m, 3H), 2.33-2.28 (m, 1H), 2.15-2.12 (m, 1H), 1.73-1.62 (m, 3H), 1.45 (d, *J* = 6.8 Hz, 3H); ¹³C NMR (101 MHz, CDCl₃) *δ* (ppm) 171.92, 157.87, 151.33, 145.17, 139.78, 129.35, 124.67, 123.31, 121.08, 120.77, 118.17, 116.02, 112.78, 68.83, 67.99, 62.54, 55.96, 50.76, 48.74, 48.68, 35.22, 29.40, 18.28.

### The preparation of 4-benzyl-9-(3-(2-methoxyphenoxy)benzyl)-2-methyl-1-oxa-4,9-diazaspiro[5.5]undecan-3-one (compound 29)

This compound was synthesized via general procedure 1 affording compound **29** as a colorless oil, in 50% yield.

¹H NMR (400 MHz, CDCl₃): δ (ppm) 7.35-7.27 (m, 3H), 7.25-7.18 (m, 3H), 7.13-7.09 (m, 1H), 7.00-6.88 (m, 5H), 6.80 (dd, *J* = 1.8, 8.1 Hz, 1H), 4.84 (d, *J* = 14.5 Hz, 1H), 4.30 (d, *J* = 14.5 Hz, 1H), 4.25 (q, *J* = 6.8 Hz, 1H), 3.82 (s, 3H), 3.44 (s, 2H), 3.24 (d, *J* = 12.1 Hz, 1H), 2.90 (d, *J* = 12.1 Hz, 1H), 2.45-2.39 (m, 3H), 2.23-2.17 (m, 1H), 1.99-1.95 (m, 1H), 1.57-1.54 (m, 2H), 1.49 (d, *J* = 6.8 Hz, 3H), 1.40-1.33 (m, 1H); ¹³C NMR (101 MHz, CDCl₃) *δ* (ppm) 169.92, 157.81, 151.31, 145.19, 140.12, 136.40, 129.26, 128.71, 128.32, 127.75, 124.62, 123.20, 121.06, 120.74, 118.06, 115.90, 112.75, 68.75, 68.17, 62.52, 55.94, 54.42, 49.82, 48.62, 48.59, 35.25, 29.52, 19.05; HRMS (ESI-Q-TOF): *m*/*z* [M+H]⁺ calcd. for C₃₀H₃₄N₂O₄: 487.25911; found: 487.2585.

### The preparation of tert-butyl 4-(((2-fluorophenyl)amino)methyl)-4-hydroxypiperidine-1-carboxylate (compound 31a)

This compound was synthesized via general procedure 6 affording compound **31a** as a yellow oil, in 88% yield.

¹H NMR (400 MHz, CDCl₃): δ (ppm) 7.00-6.94 (m, 2H), 6.76 (t, *J* = 8.2 Hz, 1H), 6.67-6.61 (m, 1H), 4.22 (s, 1H), 3.87 (s, 1H), 3.22-3.13 (m, 4H), 2.15 (s, 1H), 1.68-1.53 (m, 4H), 1.46 (s, 9H); ¹³C NMR (101 MHz, CDCl₃) *δ* (ppm) 154.82, 151.76 (d, ¹*J_{CF} =* 238.4 Hz), 136.99 (d, ²*J_{CF} =* 11.2 Hz), 124.60 (d, ⁴*J_{CF}* = 3.3 Hz), 117.25 (d, ³*J_{CF}* = 7.0 Hz), 114.57 (d, ²*J_{CF}* = 18.8 Hz), 112.62 (d, *³J_{CF}* = 2.9 Hz), 79.59, 69.89, 54.35, 39.48, 34.99, 28.45.

### The preparation of tert-butyl (R or S)-4-(((1-(2-fluorophenyl)ethyl)amino)methyl)-4-hydroxypiperidine-1-carboxylate (compound 31b or 31c)

This compound was synthesized via general procedure 6 affording compound **31b** as a colorless oil, in 56% yield.

¹H NMR (400 MHz, CDCl₃): δ (ppm) 7.31-7.27 (m, 1H), 7.25-7.19 (m, 1H), 7.14-7.10 (m, 1H), 7.04-6.99 (m, 1H), 4.05 (q, *J* = 6.7 Hz, 1H), 3.83-3.79 (m, 2H), 3.16-3.15 (m, 2H), 2.39 (dd, *J* = 12.3, 14.9 Hz, 2H), 1.51-1.35 (m, 16H); ¹³C NMR (101 MHz, CDCl₃) *δ* (ppm) 160.79 (d, ¹*J_{CF}* = 245.0 Hz), 154.85, 131.76 (d, ²*J_{CF} =* 13.2 Hz), 128.41 (d, *³J_{CF}* = 8.5 Hz), 127.65 (d, ³*J_{CF}* = 5.0 Hz), 124.35 (d, ⁴*J_{CF}* = 3.5 Hz), 115.61 (d, ²*J_{CF}* = 22.5 Hz), 79.28, 68.16, 57.24, 52.95, 40.05, 35.28 (d, ³*J_{CF}* = 25.9 Hz), 28.46, 22.77.

### The preparation of tert-butyl 4-(2-fluorophenyl)-3-oxo-1-oxa-4,9-diazaspiro[5.5]undecane-9-carboxylate (compound 32a)

This compound was synthesized via general procedure 5 affording compound **32a** as a brown oil, in 79% yield.

¹H NMR (400 MHz, CDCl₃): δ (ppm) 7.36-7.25 (m, 2H), 7.21-7.15 (m, 2H), 4.35 (s, 2H), 3.90-3.86 (m, 2H), 3.54 (s, 2H), 3.22-3.16 (m, 2H), 2.03-2.00 (m, 2H), 1.65-1.58 (m, 2H), 1.47 (s, 9H); ¹³C NMR (101 MHz, CDCl₃) *δ* (ppm) 166.51, 157.67 (d, ¹*J_{CF} =* 250.3 Hz), 154.72, 129.66 (d, *³J_{CF}* = 7.8 Hz), 128.84, 128.66 (d, ²*J_{CF} =* 12.7 Hz), 124.88 (d, ⁴*J_{CF} =* 3.7 Hz), 116.93 (d, ²*J_{CF} =* 19.8 Hz), 79.80, 69.87, 63.02, 58.50, 39.35, 31.75, 28.43.

### The preparation of tert-butyl (R or S)-4-(1-(2-fluorophenyl)ethyl)-3-oxo-1-oxa-4,9-diazaspiro[5.5]undecane-9-carboxylate (compound 32b or 32c)

This compound was synthesized via general procedure 5 affording compound **32b** as a brown oil, in 81% yield.

¹H NMR (400 MHz, CDCl₃): δ (ppm) 7.36-7.30 (m, 2H), 7.18-7.14 (m, 1H), 7.09-7.05 (m, 1H), 6.17 (q, *J* = 7.1 Hz, 1H), 4.19 (s, 2H), 3.72-3.56 (m, 2H), 3.14-3.00 (m, 2H), 3.04 (d, *J* = 12.2 Hz, 1H), 2.70 (d, *J* = 12.2 Hz, 1H), 1.77-1.71 (m, 2H), 1.53 (d, *J* = 7.2 Hz, 3H), 1.46-1.37 (m, 11H); ¹³C NMR (101 MHz, CDCl₃) *δ* (ppm) 165.55, 161.15 (d, ¹*J_{CF}* = 249.3 Hz), 154.63, 129.94 (d, *³J_{CF}* = 8.3 Hz), 129.01 (d, *³J_{CF} =* 4.4 Hz), 126.03 (d, ²*J_{CF} =* 14.0 Hz), 124.12 (d, ⁴*J_{CF}* = 3.6 Hz), 115.89 (d, ²*J_{CF}* = 22.0 Hz), 79.69, 69.44, 62.72, 49.30, 45.98 (d, ³*J_{CF}* = 2.3 Hz), 39.33, 32.38, 28.41, 15.10.

### The preparation of 4-(2-fluorophenyl)-9-(3-(2-methoxyphenoxy)benzyl)-1-oxa-4,9-diazaspiro[5.5]undecan-3-one (compound 33a)

This compound was synthesized via general procedure 2 affording compound **33a** as a yellow oil, in 68% yield.

¹H NMR (400 MHz, CDCl₃): δ (ppm) 7.34-7.23 (m, 3H), 7.21-7.17 (m, 2H), 7.14-7.10 (m, 1H), 7.02-6.89 (m, 5H), 6.81 (dd, *J* = 1.8, 8.1 Hz, 1H), 4.33 (s, 2H), 3.84 (s, 3H), 3.53 (s, 2H), 3.51 (s, 2H), 2.62-2.59 (m, 2H), 2.43-2.38 (m, 2H), 2.02-1.99 (m, 2H), 1.77-1.70 (m, 2H); ¹³C NMR (101 MHz, CDCl₃) *δ* (ppm) 166.78, 157.86, 157.71 (d, ¹*J_{CF}* = 250.7 Hz), 151.34, 145.20, 140.26, 129.54 (d, *³J_{CF} =* 7.8 Hz), 129.28, 128.89, 128.82 (d, ³*J_{CF}* = 9.4 Hz), 124.83 (d, ⁴*J_{CF}* = 3.8 Hz), 124.64, 123.15, 121.07, 120.77, 118.02, 116.89 (d, ²*J_{CF}* = 19.9 Hz), 115.86, 112.76, 69.71, 62.77, 62.60, 58.50, 55.94, 48.65, 31.93; HRMS (ESI-Q-TOF): *m*/*z* [M+H]⁺ calcd. for C₂₈H₂₉FN₂O₄: 477.21839; found: 477.2181.

### The preparation of (S)-4-(1-(2-fluorophenyl)ethyl)-9-(3-(2-methoxyphenoxy)benzyl)-1-oxa-4,9-diazaspiro[5.5]undecan-3-one (compound 33b)

This compound was synthesized via general procedure 2 affording compound **33b** as a colorless oil, in 83% yield.

¹H NMR (400 MHz, CDCl₃): δ (ppm) 7.35-7.28 (m, 2H), 7.20 (t, *J* = 7.8 Hz, 1H), 7.17-7.10 (m, 2H), 7.07-6.99 (m, 2H), 6..96-6.88 (m, 4H), 6.79 (dd, *J* = 2.5, 8.1 Hz, 1H), 6.17 (q, *J* = 7.1 Hz, 1H), 4.17 (s, 2H), 3.83 (s, 3H), 3.42 (s, 2H), 3.03 (d, *J* = 12.2 Hz, 1H), 2.68 (d, *J* = 12.2 Hz, 1H), 2.51-2.48 (m, 1H), 2.33-2.28 (m, 2H), 2.25-2.19 (m, 1H), 1.76-1.72 (m, 2H), 1.55-1.48 (m, 4H), 1.32-1.25 (m, 1H); ¹³C NMR (101 MHz, CDCl₃) *δ* (ppm) 165.74, 161.14 (d, ¹*J_{CF}* = 249.5 Hz), 157.79, 151.30, 145.20, 140.18, 129.84 (d, *³J_{CF}* = 8.5 Hz), 129.25, 128.96 (d, *³J_{CF}* = 3.9 Hz), 126.13 (d, ²*J_{CF}* = 13.9 Hz), 124.61, 124.02 (d, ⁴*J_{CF}* = 3.6 Hz), 123.20, 121.05, 120.72, 118.12, 115.85, 115.83 (d, ²*J_{CF}* = 21.9 Hz), 112.74, 69.26, 62.62, 62.52, 55.93, 49.20, 48.62, 48.54, 45.80 (d, *³J_{CF}* = 1.9 Hz), 32.59, 30.81, 15.06; HRMS (ESI-Q-TOF): *m*/*z* [M+H]⁺ calcd. for C₃₀H₃₃FN₂O₄: 505.24969; found: 505.2493.

### The preparation of (R)-4-(1-(2-fluorophenyl)ethyl)-9-(3-(2-methoxyphenoxy)benzyl)-1-oxa-4,9-diazaspiro[5.5]undecan-3-one (compound 33c)

This compound was synthesized via general procedure 2 affording compound **33c** as a colorless oil, in 82% yield.

¹H NMR (400 MHz, CDCl₃): δ (ppm) 7.35-7.28 (m, 2H), 7.20 (t, *J* = 7.8 Hz, 1H), 7.17-7.10 (m, 2H), 7.07-6.99 (m, 2H), 6..96-6.88 (m, 4H), 6.79 (dd, *J* = 2.5, 8.1 Hz, 1H), 6.17 (q, *J* = 7.1 Hz, 1H), 4.17 (s, 2H), 3.83 (s, 3H), 3.42 (s, 2H), 3.03 (d, *J* = 12.2 Hz, 1H), 2.68 (d, *J* = 12.2 Hz, 1H), 2.51-2.48 (m, 1H), 2.33-2.28 (m, 2H), 2.24-2.19 (m, 1H), 1.76-1.72 (m, 2H), 1.54-1.48 (m, 4H), 1.32-1.26 (m, 1H); ¹³C NMR (101 MHz, CDCl₃) *δ* (ppm) 165.75, 161.15 (d, ¹*J_{CF}* = 249.5 Hz), 157.79, 151.31, 145.20, 140.19, 129.84 (d, *³J_{CF}* = 8.5 Hz), 129.25, 128.96 (d, *³J_{CF}* = 3.8 Hz), 126.14 (d, ²*J_{CF}* = 13.9 Hz), 124.61, 124.02 (d, ⁴*J_{CF}* = 3.6 Hz), 123.21, 121.05, 120.71, 118.13, 115.85, 115.84 (d, ²*J_{CF}* = 21.9 Hz), 112.74, 69.26, 62.62, 62.53, 55.93, 49.21, 48.62, 48.54, 45.81 (d, *³J_{CF}* = 1.9 Hz), 32.60, 30.81, 15.06; HRMS (ESI-Q-TOF): *m*/*z* [M+H]⁺ calcd. for C₃₀H₃₃FN₂O₄: 505.24969; found: 505.2491.

### The preparation of tert-butyl 4-benzyl-3-oxo-1-oxa-4,9-diazaspiro[5.5]undecane-9-carboxylate (compound 34)

This compound was synthesized via general procedure 1 affording compound **34** as a colorless oil, in 75% yield.

¹H NMR (400 MHz, CDCl₃): δ (ppm) 7.37-7.30 (m, 3H), 7.27-7.24 (m, 2H), 4.60 (s, 2H), 4.23 (s, 2H), 3.73-3.71 (m, 2H), 3.11-3.05 (m, 4H), 1.81-1.77 (m, 2H), 1.43 (s, 9H), 1.39-1.32 (m, 2H); ¹³C NMR (101 MHz, CDCl₃) *δ* (ppm) 166.48, 154.61, 136.00, 128.81, 128.34, 127.91, 79.72, 69.39, 62.58, 54.00, 49.48, 38.79, 31.77, 28.40;

### The preparation of 4-benzyl-9-(2-isobutoxybenzyl)-1-oxa-4,9-diazaspiro[5.5]undecan-3-one (compound 36)

This compound was synthesized via general procedure 2 affording compound **36** as a colorless oil, in 82% yield.

¹H NMR (400 MHz, CDCl₃): δ (ppm) 7.35-7.23 (m, 6H), 7.21-7.17 (m, 1H), 6.90-6.86 (m, 1H), 6.82 (d, *J* = 8.2 Hz, 1H), 4.59 (s, 2H), 4.22 (s, 2H), 3.71 (d, *J* = 6.3 Hz, 2H), 3.55 (s, 2H), 3.06 (s, 2H), 2.56-2.53 (m, 2H), 2.41-2.35 (m, 2H), 2.13-2.03 (m, 1H), 1.82-1.79 (m, 2H), 1.54-1.47 (m, 2H), 1.03 (d, *J* = 6.7 Hz, 6H); ¹³C NMR (101 MHz, CDCl₃) *δ* (ppm) 166.78, 157.31, 136.10, 130.44, 128.72, 128.33, 128.03, 127.77, 126.36, 120.05, 111.26, 74.36, 69.26, 62.40, 55.93, 54.08, 49.50, 48.61, 31.98, 28.42, 19.39; HRMS (ESI-Q-TOF): *m*/*z* [M+H]⁺ calcd. for C₂₆H₃₄N₂O₃: 423.26420; found: 423.2631.

### The preparation of 3-((2-methylallyl)oxy)benzaldehyde (compound 38)

Using K₂CO₃ as base, this compound was synthesized via general procedure 1 at 0 °C affording compound **38** as a colorless oil, in 91% yield.

¹H NMR (400 MHz, CDCl₃): δ (ppm) 9.97 (s, 1H), 7.47-7.44 (m, 2H), 7.42-7.40 (m, 1H), 7.22-7.19 (m, 1H), 5.07 (d, *J* = 38.7 Hz, 2H), 4.50 (s, 2H), 1.84 (s, 3H).

The preparation of 3,3-dimethyl-2,3-dihydrobenzofuran-4-carbaldehyde (compound **39**) An oven-dried screw cap test tube was added with a magnetic stir-bar, compound **38** (630.0 mg, 3.58 mmol, 1.00 eq), pivalic acid (730.3 mg, 7.15 mmol, 2.00 eq), silver hexafluoroantimonate (245.7 mg, 0.72 mmol, 0.20 eq), β-alanine (127.4 mg, 1.43 mmol, 0.40 eq) and Cl₄Rh₂ (44.2 mg, 0.07 mmol, 0.02 eq). The tube was then evacuated and back-filled with argon. The evacuation/backfill sequence was repeated with two additional times. The reaction tube was evacuated and backfilled with argon three times. Under counterflow of argon, anhydrous DCM (7.0 mL) was added by syringe. After that, the reaction mixture was placed at 80 °C overnight. Then, it was concentrated under reduced pressure affording the crude product. The crude residue was purified by chromatography on silica gel (3% of ethyl acetate in petroleum ether) to give compound **39** as a red oil (432.0 mg, 69% yield). ¹H NMR (400 MHz, CDCl₃): δ (ppm) 10.19 (s, 1H), 7.40-7.38 (m, 1H), 7.30 (t, *J* = 7.8 Hz, 1H), 7.04-7.02 (m, 1H), 4.27 (s, 2H), 1.53 (s, 6H).

The preparation of 3,3-dimethyl-2,3-dihydrobenzofuran-4-carbaldehyde (compound **42**) This compound was synthesized via general procedure 7 affording compound **42** as a yellow oil, in 66% yield. ¹H NMR (400 MHz, CDCl₃): δ (ppm) 10.04 (s, 1H), 7.31-7.28 (m, 2H), 6.98 (dd, *J* = 2.4, 6.5 Hz, 1H), 3.35 (s, 2H), 1.50 (s, 6H).

The preparation of 2,2-dimethyl-2,3-dihydrobenzofuran-7-carbaldehyde (compound **44**) Compound **43** (370.1 mg, 2.1 mmol, 1.00 eq) was stirred at 200 °C for 4h. Then, it was cooled to room temperature before adding I₂ (106.6 mg, 0.42 mmol, 0.20 eq) in DCM (5.0 mL). Afterwards, it was stirred at room temperature for 1 h. After this, it was concentrated under reduced pressure affording the crude product. The crude residue was purified by chromatography on silica gel (2% of ethyl acetate in petroleum ether) to give compound **44** as an orange solid (121.2 mg, 33% yield). ¹H NMR (400 MHz, CDCl₃): δ (ppm) 10.23 (s, 1H), 7.60-7.58 (m, 1H), 7.35-7.33 (m, 1H), 6.88 (t, *J* = 7.5 Hz, 1H), 3.03 (s, 2H), 1.54 (s, 6H).

### The preparation of 2-((2-methylbut-3-yn-2-yl)oxy)benzaldehyde (compound 46)

An oven-dried screw cap test tube was added with a magnetic stir-bar, compound **45** (390.8 mg, 3.2 mmol, 1.00 eq) and CuCl₂ (4.3 mg, 0.03 mmol, 0.01 eq). The tube was then evacuated and back-filled with argon. The evacuation/backfill sequence was repeated with two additional times. The reaction tube was evacuated and backfilled with argon three times. Under counterflow of argon, anhydrous CH₃CN (6.0 mL) was added by syringe, followed by 3-chloro-3-methyl-1-butyne (344.6 mg, 3.36 mmol, 1.05 eq), DBU (535.9 mg, 3.52 mmol, 1.10 eq). After that, the reaction mixture was placed at 0 °C for 4 h. Then, it was concentrated under reduced pressure affording the crude product. The crude residue was purified by chromatography on silica gel (4% of ethyl acetate in petroleum ether) to give compound **46** as a yellow oil (410.0 mg, 68% yield). ¹H NMR (400 MHz, CDCl₃): δ (ppm) 10.44 (s, 1H), 7.88-7.86 (m, 1H), 7.56-7.52 (m, 2H), 7.16-7.12 (m, 1H), 2.62 (s, 1H), 1.74 (s, 6H).

### The preparation of 2,2-dimethyl-2H-chromene-8-carbaldehyde (compound 47)

Compound **46** (380.0 mg, 2.02 mmol, 1.00 eq) was added into N,N-diethylaniline (2.0 mL) and stirred at 210 °C for 4h. Then, the reaction mixture was washed with brine (10.0 mL) and extracted with ethyl acetate (20.0 mL). The organic layer was collected and dried over magnesium sulfate. After this, it was concentrated under reduced pressure affording the crude product. The crude residue was purified by chromatography on silica gel (1% of ethyl acetate in petroleum ether) to give compound **47** as an orange oil (130.2 mg, 34% yield). ¹H NMR (400 MHz, CDCl₃): δ (ppm) 10.46 (s, 1H), 7.64 (dd, *J =* 1.7, 7.9 Hz, 1H), 7.17 (dd, *J =* 1.7, 7.3 Hz, 1H), 6.89 (t, *J* = 7.6 Hz, 1H), 6.35 (d, *J* = 9.9 Hz, 1H), 5.70 (d, *J* = 9.9 Hz, 1H), 1.50 (s, 6H).

### The preparation of benzofuran-7-carbaldehyde (compound 49)

This compound was synthesized via general procedure 7 affording compound **49** as a colorless oil, in 74% yield. ¹H NMR (400 MHz, CDCl₃): δ (ppm) 10.46 (s, 1H), 7.88 (dd, *J =* 1.2, 7.7 Hz, 1H), 7.81 (dd, *J =* 1.1, 7.5 Hz, 1H), 7.79 (d, *J =* 2.2 Hz, 1H), 7.40 (t, *J* = 7.6 Hz, 1H), 6.88 (d, *J* = 2.2 Hz, 1H).

### The preparation of 2,2,4-trimethylbenzo[d][1,3]dioxole (compound 52)

Compound **51** (372.4 mg, 3.00 mmol, 1.00 eq), 2,2-dimethoxypropane (781.1 mg, 7.50 mmol, 2.50 eq) and p-toluenesulfonic acid monohydrate (5.70 mg, 0.03 mmol, 0.01 eq) were added into toluene (6.0 mL) and the reaction mixture was stirred at 105 °C for 4h. After this, it was concentrated under reduced pressure affording the crude product. The crude residue was purified by chromatography on silica gel (100% petroleum ether) to give compound **52** as a yellow oil (200.1 mg, 41% yield). ¹H NMR (400 MHz, CDCl₃): δ (ppm) 6.68 (t, *J =* 7.7 Hz, 1H), 6.62-6.57 (m, 2H), 2.20 (s, 3H), 1.67 (s, 6H).

The preparation of 4-(bromomethyl)-2,2-dimethylbenzo[d][1,3]dioxole (compound **53**) An oven-dried screw cap test tube was added with a magnetic stir-bar, compound **52** (200.0 mg, 1.22 mmol, 1.00 eq), azobisisobutyronitrile (100.0 mg, 0.61 mmol, 0.50 eq) and NBS (216.8 mg, 1.22 mmol, 1.00 eq). The tube was then evacuated and back-filled with argon. The evacuation/backfill sequence was repeated with two additional times. The reaction tube was evacuated and backfilled with argon three times. Under counterflow of argon, cyclohexane (2.4 mL) was added by syringe. After that, the reaction mixture was placed at 85 °C for 8 h. Then, it was concentrated under reduced pressure affording the crude product. The crude residue was purified by chromatography on silica gel (5% of ethyl acetate in petroleum ether) to give compound **53** as a crude compound which was used for next step without further purification.

The preparation of 4-(chloromethyl)-2,2-difluoro-2,3-dihydrobenzofuran (compound **55**) This compound was synthesized via general procedure 8 affording compound **55** as a colorless oil, in 50% yield. ¹H NMR (400 MHz, CDCl₃): δ (ppm) 7.38 (t, *J* = 8.0 Hz, 1H), 7.27 (dd, *J =* 1.4, 7.7 Hz, 1H), 7.10 (dd, *J =* 1.3, 8.3 Hz, 1H), 3.94 (s, 2H), 1.54 (s, 2H);

### The preparation of 4-benzyl-9-((3,3-dimethyl-2,3-dihydrobenzofuran-4-yl)methyl)-1-oxa-4,9-diazaspiro[5.5]undecan-3-one (compound 56a)

This compound was synthesized via general procedure 9 affording compound **56a** as a yellow oil, in 44% yield.

¹H NMR (400 MHz, CDCl₃): δ (ppm) 7.36-7.29 (m, 3H), 7.26-7.24 (m, 2H), 7.04 (t, *J* = 7.8 Hz, 1H), 6.84 (d, *J* = 7.6 Hz, 1H), 6.69 (dd, *J =* 0.7, 7.9 Hz, 1H), 4.60 (s, 2H), 4.24 (s, 2H), 4.11 (s, 2H), 3.51 (s, 2H), 3.09 (s, 2H), 2.50-2.47 (m, 2H), 2.35-2.30 (m, 2H), 1.83-1.80 (m, 2H), 1.53-1.46 (m, 2H), 1.40 (s, 6H); ¹³C NMR (101 MHz, CDCl₃) *δ* (ppm) 166.78, 159.92, 136.11, 135.30, 133.83, 128.76, 128.30, 127.81, 127.79, 122.35, 108.99, 84.46, 69.33, 62.40, 59.29, 54.06, 49.56, 48.63, 42.97, 32.02, 26.20; HRMS (ESI-Q-TOF): *m*/*z* [M+H]⁺ calcd. for C₂₆H₃₂N₂O₃: 421.24855; found: 421.2485.

### The preparation of 4-benzyl-9-((2,2-dimethyl-2,3-dihydrobenzofuran-4-yl)methyl)-1-oxa-4,9-diazaspiro[5.5]undecan-3-one (compound 56b)

This compound was synthesized via general procedure 9 affording compound **56b** as a yellow oil, in 50% yield.

¹H NMR (400 MHz, CDCl₃): δ (ppm) 7.35-7.28 (m, 3H), 7.26-7.24 (m, 2H), 7.04 (t, *J* = 7.8 Hz, 1H), 6.73 (d, *J* = 7.5 Hz, 1H), 6.63 (d, *J* = 7.9 Hz, 1H), 4.60 (s, 2H), 4.23 (s, 2H), 3.37 (s, 2H), 3.07 (s, 2H), 2.96 (s, 2H), 2.47-2.44 (m, 2H), 2.32-2.27 (m, 2H), 1.82-1.79 (m, 2H), 1.51-1.47 (m, 2H), 1.45 (s, 6H); ¹³C NMR (101 MHz, CDCl₃) *δ* (ppm) 166.74, 158.83, 136.13, 135.44, 128.74, 128.33, 127.79, 127.77, 126.68, 120.77, 108.15, 86.48, 69.31, 62.40, 60.65, 54.02, 49.49, 48.64, 41.87, 32.04, 28.29; HRMS (ESI-Q-TOF): *m*/*z* [M+H]⁺ calcd. for C₂₆H₃₂N₂O₃: 421.24855; found: 421.2474.

### The preparation of 4-benzyl-9-((2,2-dimethyl-2,3-dihydrobenzofuran-7-yl)methyl)-1-oxa-4,9-diazaspiro[5.5]undecan-3-one (compound 56c)

This compound was synthesized via general procedure 9 affording compound **56c** as a yellow oil, in 36% yield.

¹H NMR (400 MHz, CDCl₃): δ (ppm) 7.35-7.28 (m, 3H), 7.25-7.23 (m, 2H), 7.11 (d, *J* = 7.6 Hz, 1H), 7.05 (d, *J* = 7.3 Hz, 1H), 6.79 (t, *J* = 7.5 Hz, 1H), 4.58 (s, 2H), 4.19 (s, 2H), 3.61 (s, 2H), 3.07 (s, 2H), 3.00 (s, 2H), 2.69-2.66 (m, 2H), 2.51-2.46 (m, 2H), 1.84-1.81 (m, 2H), 1.66-1.59 (m, 2H), 1.44 (s, 6H); ¹³C NMR (101 MHz, CDCl₃) *δ* (ppm) 166.60, 157.91, 135.98, 129.57, 128.77, 128.32, 127.85, 127.00, 124.49, 119.98, 117.49, 86.64, 68.91, 62.42, 55.39, 54.03, 49.57, 47.80, 42.98, 31.32, 28.20; HRMS (ESI-Q-TOF): *m*/*z* [M+H]⁺ calcd. for C₂₆H₃₂N₂O₃: 421.24855; found: 421.2475.

### The preparation of 4-benzyl-9-((2,2-dimethyl-2H-chromen-8-yl)methyl)-1-oxa-4,9-diazaspiro[5.5]undecan-3-one (compound 56d)

This compound was synthesized via general procedure 9 affording compound **56d** as a yellow oil, in 70% yield.

¹H NMR (400 MHz, CDCl₃): δ (ppm) 7.35-7.28 (m, 3H), 7.25-7.20 (m, 3H), 6.93 (dd, *J* = 1.6 Hz, *J* = 7.4 Hz, 1H), 6.82 (t, *J* = 7.5 Hz, 1H), 6.31 (d, *J* = 9.8 Hz, 1H), 5.61 (d, *J* = 9.8 Hz, 1H), 4.58 (s, 2H), 4.19 (s, 2H), 4.11 (s, 2H), 3.73 (s, 2H), 3.09 (s, 2H), 2.82-2.80 (m, 2H), 2.63-2.55 (m, 2H), 1.87-1.84 (m, 2H), 1.76-1.69 (m, 2H), 1.41 (s, 6H); ¹³C NMR (101 MHz, CDCl₃) *δ* (ppm) 166.41, 151.38, 135.89, 131.32, 130.59, 128.79, 128.31, 127.90, 126.33, 122.38, 121.78, 121.12, 120.51, 76.68, 68.64, 62.43, 54.34, 53.97, 49.60, 47.71, 30.89, 28.13; HRMS (ESI-Q-TOF): *m*/*z* [M+H]⁺ calcd. for C₂₇H₃₂N₂O₃: 433.24855; found: 433.2476.

### The preparation of 9-(benzofuran-7-ylmethyl)-4-benzyl-1-oxa-4,9-diazaspiro[5.5]undecan-3-one (compound 56e)

This compound was synthesized via general procedure 9 affording compound **56e** as a colorless oil, in 71% yield.

¹H NMR (400 MHz, CDCl₃): δ (ppm) 7.61 (d, *J* = 2.2 Hz, 1H), 7.50 (dd, *J* = 1.5, 7.4 Hz, 1H), 7.34-7.27 (m, 3H), 7.24-7.20 (m, 4H), 6.76 (d, *J* = 2.2 Hz, 1H), 4.58 (s, 2H), 4.21 (s, 2H), 3.82 (s, 2H), 3.05 (s, 2H), 2.58-2.55 (m, 2H), 2.43-2.37 (m, 2H), 1.83-1.80 (m, 2H), 1.54-1.47 (m, 2H); ¹³C NMR (101 MHz, CDCl₃) *δ* (ppm) 166.74, 153.87, 144.79, 136.09, 128.72, 128.35, 127.79, 127.28, 125.34, 122.72, 121.63, 120.12, 106.69, 69.20, 62.41, 56.43, 54.02, 49.46, 48.64, 31.89; HRMS (ESI-Q-TOF): *m*/*z* [M+H]⁺ calcd. for C₂₄H₂₆N₂O₃: 391.20160; found: 391.2015.

### The preparation of 4-benzyl-9-((2,3-dihydrobenzofuran-7-yl)methyl)-1-oxa-4,9-diazaspiro[5.5]undecan-3-one (compound 56f)

This compound was synthesized via general procedure 9 affording compound **56f** as a yellow oil, in 68% yield.

¹H NMR (400 MHz, CDCl₃): δ (ppm) 7.34-7.28 (m, 3H), 7.25-7.22 (m, 2H), 7.13-7.09 (m, 2H), 6.81 (t, *J* = 7.5 Hz, 1H), 4.58 (s, 2H), 4.54 (t, *J* = 8.7 Hz, 2H), 4.20 (s, 2H), 3.59 (s, 2H), 3.20 (t, *J* = 8.7 Hz, 2H), 3.07 (s, 2H), 2.69-2.66 (m, 2H), 2.48-2.43 (m, 2H), 1.84-1.81 (m, 2H), 1.66-1.59 (m, 2H); ¹³C NMR (101 MHz, CDCl₃) *δ* (ppm) 166.57, 158.93, 135.99, 129.72, 128.76, 128.34, 127.84, 126.87, 124.34, 120.40, 117.60, 71.07, 68.90, 62.43, 55.88, 53.97, 49.54, 48.06, 31.25, 29.87; HRMS (ESI-Q-TOF): *m*/*z* [M+H]⁺ calcd. for C₂₄H₂₈N₂O₃: 393.21725; found: 393.2164.

### The preparation of 4-benzyl-9-(chroman-8-ylmethyl)-1-oxa-4,9-diazaspiro[5.5]undecan-3-one (compound 56g)

This compound was synthesized via general procedure 9 affording compound **56g** as a colorless oil, in 67% yield.

¹H NMR (400 MHz, CDCl₃): δ (ppm) 7.35-7.29 (m, 3H), 7.25-7.23 (m, 3H), 7.03 (d, *J* = 7.0 Hz, 1H), 6.83 (t, *J* = 7.5 Hz, 1H), 4.58 (s, 2H), 4.20-4.18 (m, 4H), 3.85 (s, 2H), 3.11 (s, 2H), 2.97-2.95 (m, 2H), 2.79 (t, *J* = 6.5 Hz, 2H), 2.71-2.63 (m, 2H), 2.02-1.96 (m, 2H), 1.92-1.86 (m, 4H); ¹³C NMR (101 MHz, CDCl₃) *δ* (ppm) 166.19, 153.57, 135.79, 130.58, 130.06, 128.83, 128.33, 127.97, 122.49, 120.03, 119.96, 68.31, 66.68, 62.48, 54.65, 53.89, 49.68, 47.43, 30.21, 24.90, 22.17; HRMS (ESI-Q-TOF): *m*/*z* [M+H]⁺ calcd. for C₂₅H₃₀N₂O₃: 407.23290; found: 407.2318.

### The preparation of 9-(benzo[d][1,3]dioxol-4-ylmethyl)-4-benzyl-1-oxa-4,9-diazaspiro[5.5]undecan-3-one (compound 56h)

This compound was synthesized via general procedure 9 affording compound **56h** as a colorless oil, in 71% yield.

¹H NMR (400 MHz, CDCl₃): δ (ppm) 7.34-7.23 (m, 5H), 6.78-6.72 (m, 3H) , 5.92 (s, 2H), 4.58 (s, 2H), 4.21 (s, 2H), 3.50 (s, 2H), 3.05 (s, 2H), 2.56-2.53 (m, 2H), 2.38-2.32 (m, 2H), 1.83-1.80 (m, 2H), 1.55-1.48 (m, 2H); ¹³C NMR (101 MHz, CDCl₃) *δ* (ppm) 166.68, 147.13, 146.21, 136.06, 128.72, 128.35, 127.79, 123.42, 121.31, 118.99, 107.56, 100.65, 69.09, 62.39, 56.04, 54.01, 49.46, 48.38, 31.73; HRMS (ESI-Q-TOF): *m*/*z* [M+H]⁺ calcd. for C₂₃H₂₆N₂O₄: 395.19652; found: 395.1955.

### The preparation of 4-benzyl-9-((2,3-dihydrobenzo[b][1,4]dioxin-5-yl)methyl)-1-oxa-4,9-diazaspiro[5.5]undecan-3-one (compound 56i)

This compound was synthesized via general procedure 9 affording compound **56i** as a colorless oil, in 77% yield.

¹H NMR (400 MHz, CDCl₃): δ (ppm) 7.35-7.23 (m, 5H), 6.91-6.88 (m, 1H), 6.82-6.78 (m, 2H), 4.58 (s, 2H), 4.25 (s, 4H), 4.20 (s, 2H), 3.66 (s, 2H), 3.08 (s, 2H), 2.75-2.73 (m, 2H), 2.53-2.47 (m, 2H), 1.86-1.83 (m, 2H), 1.70-1.65 (m, 2H); ¹³CNMR (101 MHz, CDCl₃) *δ* (ppm) 166.49, 143.51, 142.27, 135.95, 128.78, 128.34, 127.87, 123.44, 120.79, 116.94, 68.78, 64.31, 64.11, 62.43, 54.87, 53.98, 49.56, 47.99, 31.08; HRMS (ESI-Q-TOF): *m*/*z* [M+H]⁺ calcd. for C₂₄H₂₈N₂O₄: 409.21216; found: 409.2116.

### The preparation of 4-benzyl-9-((3,4-dihydro-2H-benzo[b][1,4]dioxepin-6-yl)methyl)-1-oxa-4,9-diazaspiro[5.5]undecan-3-one (compound 56j)

This compound was synthesized via general procedure 9 affording compound **56j** as a yellow oil, in 71% yield.

¹H NMR (400 MHz, CDCl₃): δ (ppm) 7.35-7.23 (m, 5H), 7.04-7.01 (m, 1H), 6.95-6.87 (m, 2H), 4.58 (s, 2H), 4.20 (s, 2H), 4.18-4.15 (m, 4H), 3.71 (s, 2H), 3.08 (s, 2H), 2.75-2.72 (m, 2H), 2.54-2.48 (m, 2H), 2.21-2.16 (m, 2H), 1.86-1.83 (m, 2H), 1.71-1.65 (m, 2H); ¹³CNMR (101 MHz, CDCl₃) *δ* (ppm) 166.47, 151.60, 150.34, 135.94, 128.78, 128.33, 127.87, 125.60, 122.97, 121.45, 70.56, 70.47, 68.75, 62.43, 55.60, 53.94, 49.57, 47.95, 31.71, 31.10; HRMS (ESI-Q-TOF): *m*/*z* [M+H]⁺ calcd. for C₂₅H₃₀N₂O₄: 423.22781; found: 423.2271.

### The preparation of 4-benzyl-9-((2,2-dimethylbenzo[d][1,3]dioxol-4-yl)methyl)-1-oxa-4,9-diazaspiro[5.5]undecan-3-one (compound 56k)

This compound was synthesized via general procedure 2 affording compound **56k** as a colorless oil, in 59% yield.

¹H NMR (400 MHz, CDCl₃): δ (ppm) 7.35-7.28 (m, 3H), 7.25-7.23 (m, 2H), 6.78-6.72 (m, 2H), 6.66 (dd, *J* = 2.3, 6.6 Hz, 1H), 4.59 (s, 2H), 4.20 (s, 2H), 3.56 (s, 2H), 3.07 (s, 2H), 2.65-2.62 (m, 2H), 2.45-2.40 (m, 2H), 1.85-1.81 (m, 2H), 1.65 (s, 6H), 1.62-1.55 (m, 2H); ¹³C NMR (101 MHz, CDCl₃) *δ* (ppm) 166.65, 146.96, 146.38, 136.01, 128.76, 128.33, 127.84, 122.93, 120.87, 118.92, 117.55, 107.60, 68.93, 62.41, 55.27, 54.08, 49.57, 47.81, 31.43, 25.87; HRMS (ESI-Q-TOF): *m*/*z* [M+H]⁺ calcd. for C₂₅H₃₀N₂O₄: 423.22781; found: 423.2281.

### The preparation of 4-benzyl-9-((2,2-difluorobenzo[d][1,3]dioxol-4-yl)methyl)-1-oxa-4,9-diazaspiro[5.5]undecan-3-one (compound 56I)

This compound was synthesized via general procedure 2 affording compound **56I** as a colorless oil, in 73% yield.

¹H NMR (400 MHz, CDCl₃): δ (ppm) 7.35-7.29 (m, 3H), 7.26-7.23 (m, 2H), 7.05-6.99 (m, 2H), 6.96-6.93 (m, 1H), 4.59 (s, 2H), 4.21 (s, 2H), 3.55 (s, 2H), 3.07 (s, 2H), 2.50-2.48 (m, 2H), 2.39-2.33 (m, 2H), 1.84-1.80 (m, 2H), 1.53-1.46 (m, 2H); ¹³C NMR (101 MHz, CDCl₃) *δ* (ppm) 166.69, 143.46, 142.50, 136.08, 131.47 (t, ¹*J_{CF}* = 254.5 Hz), 128.75, 128.36, 127.82, 124.85, 123.28, 120.81, 108.17, 69.06, 62.39, 55.42, 53.97, 49.48, 48.39, 31.89; HRMS (ESI-Q-TOF): *m*/*z* [M+H]⁺ calcd. for C₂₃H₂₄F₂N₂O₄: 431.17767; found: 431.1767.

### The preparation of tert-butyl 4-(2-fluorobenzyl)-3-oxo-1-oxa-4,9-diazaspiro[5.5]undecane-9-carboxylate (compound 57b)

This compound was synthesized via general procedure 1 affording compound **57b** as a brown oil, in 97% yield. ¹H NMR (400 MHz, CDCl₃): δ (ppm) 7.38-7.34 (m, 1H), 7.32-7.27 (m, 1H), 7.16-7.12 (m, 1H), 7.09-7.04 (m, 1H), 4.67 (s, 2H), 4.20 (s, 2H), 3.76-3.72 (m, 2H), 3.15 (s, 2H), 3.11-3.05 (m, 2H), 1.81-1.78 (m, 2H), 1.44 (s, 9H), 1.43-1.36 (m, 2H); ¹³C NMR (101 MHz, CDCl₃) *δ* (ppm) 166.61, 161.17 (d, ¹*J_{CF}* = 246.3 Hz), 154.64, 131.00 (d, ³*J_{CF}* = 3.7 Hz), 129.76 (d, ³*J_{CF}* = 8.1 Hz), 124.63 (d, ⁴*J_{CF}* = 3.6 Hz), 123.00 (d, ²*J_{CF} =* 14.8 Hz), 115.50 (d, ²*J_{CF}* = 21.8 Hz), 79.74, 69.44, 62.62, 54.58, 43.09 (d*,* ³*J_{CF}* = 3.6 Hz), 39.09, 31.71, 28.41.

### The preparation of tert-butyl 3-oxo-4-(pyridin-2-ylmethyl)-1-oxa-4,9-diazaspiro[5.5]undecane-9-carboxylate (compound 57c)

This compound was synthesized via general procedure 1 affording compound **57c** as a colorless oil, in 97% yield.

¹H NMR (400 MHz, CDCl₃): δ (ppm) 8.55-8.53 (m, 1H), 7.70-7.66 (m, 1H), 7.34 (d, *J* = 7.8 Hz, 1H), 7.24-7.21 (m, 1H), 4.70 (s, 2H), 4.22 (s, 2H), 3.74-3.71 (m, 2H), 3.32 (s, 2H), 3.15-3.09 (m, 2H), 1.82-1.79 (m, 2H), 1.44 (s, 9H), 1.47-1.40 (m, 2H);

### The preparation of 9-((2,2-dimethyl-2,3-dihydrobenzofuran-4-yl)methyl)-4-(2-fluorophenyl)-1-oxa-4,9-diazaspiro[5.5]undecan-3-one (compound 58a)

This compound was synthesized via general procedure 9 affording compound **58a** as a yellow oil, in 27% yield.

¹H NMR (400 MHz, CDCl₃): δ (ppm) 7.35-7.29 (m, 1H), 7.28-7.24 (m, 1H), 7.21-7.14 (m, 2H), 7.08 (t, *J* = 7.8 Hz, 1H), 6.80 (d, *J* = 7.6 Hz, 1H), 6.66 (d, *J* = 7.9 Hz, 1H), 4.34 (s, 2H), 3.55 (s, 2H), 3.50 (s, 2H), 3.02 (s, 2H), 2.70-2.68 (m, 2H), 2.49-2.44 (m, 2H), 2.04-2.01 (m, 2H), 1.83-1.77 (m, 2H), 1.47 (s, 6H); ¹³C NMR (101 MHz, CDCl₃) *δ* (ppm) 166.71, 158.94, 157.68 (d, ¹*J_{CF}* = 251.2 Hz), 134.33, 129.60 (d, ³*J_{CF} =* 8.0 Hz), 128.82, 128.69, 127.94, 126.95, 124.84 (d, ⁴*J_{CF}* = 3.8 Hz), 121.07, 116.90 (d, ²*J_{CF} =* 19.9 Hz), 108.50, 86.56, 69.59, 62.82, 60.30, 58.41, 48.52, 41.94, 31.64, 28.28; HRMS (ESI-Q-TOF): *m*/*z* [M+H]⁺ calcd. for C₂₅H₂₉FN₂O₃: 425.22348; found: 425.2227.

### The preparation of 9-((2,2-dimethyl-2,3-dihydrobenzofuran-4-yl)methyl)-4-(2-fluorobenzyl)-1-oxa-4,9-diazaspiro[5.5]undecan-3-one (compound 58b)

This compound was synthesized via general procedure 9 affording compound **58b** as a yellow oil, in 21% yield.

¹H NMR (400 MHz, CDCl₃): δ (ppm) 7.37-7.33 (m, 1H), 7.31-7.25 (m, 1H), 7.14-7.10 (m, 1H), 7.08-7.03 (m, 2H), 6.75 (d, *J* = 7.6 Hz, 1H), 6.63 (d, *J* = 7.9 Hz, 1H), 4.66 (s, 2H), 4.20 (s, 2H), 3.38 (s, 2H), 3.14 (s, 2H), 2.98 (s, 2H), 2.50-2.47 (m, 2H), 2.32-2.27 (m, 2H), 1.83-1.79 (m, 2H), 1.53-1.49 (m, 2H), 1.46 (s, 6H); ¹³C NMR (101 MHz, CDCl₃) *δ* (ppm) 166.88, 161.17 (d, ¹*J_{CF}* = 246.3 Hz), 158.84, 135.47, 130.91 (d, ³*J_{CF} =* 4.1 Hz), 129.63 (d, ³*J_{CF}* = 8.1 Hz), 127.79, 126.69, 124.56 (d, ⁴*J_{CF}* = 3.5 Hz), 123.12 (d, ²*J_{CF} =* 14.9 Hz), 120.78, 115.44 (d, ²*J_{CF}* = 21.9 Hz), 108.17, 86.50, 69.36, 62.42, 60.66, 54.56, 48.65, 43.06 (d, ³*J_{CF}* = 3.6 Hz), 41.88, 32.02, 28.29; HRMS (ESI-Q-TOF): *m*/*z* [M+H]⁺ calcd. for C₂₆H₃₁FN₂O₃: 439.23913; found: 439.2800.

### The preparation of 9-((2,2-dimethyl-2,3-dihydrobenzofuran-4-yl)methyl)-4-(pyridin-2-ylmethyl)-1-oxa-4,9-diazaspiro[5.5]undecan-3-one (compound 58c)

This compound was synthesized via general procedure 9 affording compound **58c** as a colorless oil, in 15% yield.

¹H NMR (400 MHz, CDCl₃): δ (ppm) 8.54-8.53 (m, 1H), 7.69-7.64 (m, 1H), 7.33 (d, *J* = 7.8 Hz, 1H), 7.23-7.19 (m, 1H), 7.05 (t, *J =* 7.7 Hz, 1H), 6.76 (d, *J* = 7.4 Hz, 1H), 6.64 (d, *J* = 7.9 Hz, 1H), 4.70 (s, 2H), 4.22 (s, 2H), 3.39 (s, 2H), 3.30 (s, 2H), 2.98 (s, 2H), 2.50-2.48 (m, 2H), 2.35-2.31 (m, 2H), 1.84-1.81 (m, 2H), 1.58-1.49 (m, 2H), 1.46 (s, 6H); ¹³C NMR (151 MHz, CDCl₃) *δ* (ppm) 166.82, 158.87, 156.33, 149.32, 136.97, 135.53, 127.83, 126.75, 122.72, 122.69, 120.83, 108.19, 86.53, 69.45, 62.45, 60.63, 55.16, 51.65, 48.68, 41.89, 32.06, 28.28; HRMS (ESI-Q-TOF): *m*/*z* [M+H]⁺ calcd. for C₂₅H₃₁N₃O₃: 422.24380; found: 422.2825.

### The preparation of tert-butyl (S)-4-(((1-(2,4-difluorophenyl)ethyl)amino)methyl)-4-hydroxypiperidine-1-carboxylate (compound 59)

This compound was synthesized via general procedure 6 affording compound **59** as a yellow oil, in 91% yield.

¹H NMR (400 MHz, CDCl₃): δ (ppm) 7.30-7.24 (m, 2H), 6.88-6.83 (m, 1H), 6.81-6.75 (m, 1H), 4.03 (q, *J* = 6.7 Hz, 1H), 3.83-3.81 (m, 2H), 3.16-3.14 (m, 2H), 2.38 (dd, *J* = 12.2, 28.8 Hz, 2H), 1.55-1.35 (m, 16H); ¹³C NMR (101 MHz, CDCl₃) *δ* (ppm) 162.12 (dd, ¹*J_{CF}* = 253.2 Hz, ³*J_{CF} =* 10.1 Hz), 161.83 (dd, ¹*J_{CF}* = 250.2 Hz, ³*J_{CF} =* 12.6 Hz), 154.84, 128.42 (t, ³*J_{CF}* = 8.2 Hz), 127.68 (dd, ²*J_{CF}* = 13.4 Hz, ⁴*J_{CF}* = 3.8 Hz), 111.43 (dd, ²*J_{CF}* = 21.1 Hz, ⁴*J_{CF}* = 3.6 Hz), 103.95 (t, ²*J_{CF}* = 25.8 Hz), 79.33, 68.21, 57.21, 52.30, 39.98, 35.28 *(d,* ³*J_{CF}* = 25.1 Hz), 28.46, 22.83.

### The preparation of tert-butyl (S)-4-(1-(2,4-difluorophenyl)ethyl)-3-oxo-1-oxa-4,9-diazaspiro[5.5]undecane-9-carboxylate (compound 60)

This compound was synthesized via general procedure 5 affording compound **60** as a brown oil, in 28% yield.

¹H NMR (400 MHz, CDCl₃): δ (ppm) 7.33-7.29 (m, 1H), 6.97-6.81 (m, 2H), 6.10 (q, *J* = 7.1 Hz, 1H), 4.19 (s, 2H), 3.77-3.69 (m, 2H), 3.12-3.00 (m, 2H), 3.03 (d, *J* = 12.2 Hz, 1H), 2.69 (d, *J* = 12.2 Hz, 1H), 1.79-1.71 (m, 2H), 1.52 (d, *J* = 7.2 Hz, 3H), 1.46-1.37 (m, 11H); ¹³C NMR (101 MHz, CDCl₃) *δ* (ppm) 165.97, 162.05 (dd, ¹*J_{CF}* = 253.21 Hz, ³*J_{CF} =* 10.1 Hz), 159.38 (dd, ¹*J_{CF}* = 250.2 Hz, ³*J_{CF} =* 10.9 Hz), 154.86, 129.89 (t, ³*J_{CF}* = 7.6 Hz), 128.85 (dd, ²*J_{CF} = 9.3* Hz, ⁴*J_{CF}* = 3.3 Hz), 111.23 (dd, ²*J_{CF} =* 21.6 Hz, ⁴*J_{CF}* = 3.6 Hz), 104.45 (t, ²*J_{CF}* = 25.2 Hz), 79.89, 69.40, 62.62, 49.53, 45.90 (d, ³*J_{CF} =* 1.8 Hz), 39.19, 32.19, 28.41, 15.18.

### The preparation of 4-(chloromethyl)-2,2-dimethyl-2,3-dihydrobenzofuran (compound 62)

This compound was synthesized via general procedure 8 affording compound **62** as a colorless oil, in 52% yield.

¹H NMR (400 MHz, CDCl₃): δ (ppm) 7.10 (t, *J* = 7.8 Hz, 1H), 6.82 (d, *J* = 7.5 Hz, 1H), 6.71 (d, *J* = 8.0 Hz, 1H), 4.50 (s, 2H), 1.50 (s, 6H); ¹³C NMR (101 MHz, CDCl₃) *δ* (ppm) 159.24, 134.03, 128.50, 126.58, 120.41, 109.92, 86.98, 44.31, 41.21, 28.33.

### The preparation of (R)-9-((2,2-dimethyl-2,3-dihydrobenzofuran-4-yl)methyl)-4-(1-(2-fluorophenyl)ethyl)-1-oxa-4,9-diazaspiro[5.5]undecan-3-one (compound 63a)

This compound was synthesized via general procedure 2 affording compound **63a** as a colorless oil, in 74% yield.

¹H NMR (400 MHz, CDCl₃): δ (ppm) 7.36-7.28 (m, 2H), 7.17-7.13 (m, 1H), 7.07-7.02 (m, 2H), 6.73 (d, *J* = 7.5 Hz, 1H), 6.62 (d, *J* = 7.9 Hz, 1H), 6.17 (q, *J* = 7.1 Hz, 1H), 4.19 (s, 2H), 3.35 (s, 2H), 3.03 (d, *J* = 12.2 Hz, 1H), 2.96 (s, 2H), 2.70 (d, *J* = 12.2 Hz, 1H), 2.50-2.47 (m, 1H), 2.34-2.29 (m, 2H), 2.26-2.20 (m, 1H), 1.79-1.72 (m, 2H), 1.55-1.49 (m, 4H), 1.45 (s, 6H), 1.35-1.29 (m, 1H); ¹³C NMR (101 MHz, CDCl₃) *δ* (ppm) 165.76, 161.17 (d, ¹*J_{CF}* = 249.6 Hz), 158.81, 135.42, 129.80 (d, ³*J_{CF}* = 8.5 Hz), 128.99 (d, ³*J_{CF}* = 4.2 Hz), 127.78, 126.68, 126.16 (d, ²*J_{CF}* = 13.8 Hz), 124.02 (d, ⁴*J_{CF}* = 3.5 Hz), 120.78, 115.83 (d, ²*J_{CF}* = 21.9 Hz), 108.12, 86.47, 69.34, 62.53, 60.62, 49.20, 48.76, 48.67, 45.86 (d, ³*J_{CF} =* 1.9 Hz), 41.86, 32.61, 31.07, 28.29, 15.08; HRMS (ESI-Q-TOF): *m*/*z* [M+H]⁺ calcd. for C₂₇H₃₃FN₂O₃: 453.25478; found: 453.2535.

### The preparation of (S)-9-((2,2-dimethyl-2,3-dihydrobenzofuran-4-yl)methyl)-4-(1-(2-fluorophenyl)ethyl)-1-oxa-4,9-diazaspiro[5.5]undecan-3-one (compound 63b)

This compound was synthesized via general procedure 2 affording compound **63b** as a colorless oil, in 72% yield.

¹H NMR (400 MHz, CDCl₃): δ (ppm) 7.36-7.28 (m, 2H), 7.17-7.13 (m, 1H), 7.07-7.03 (m, 2H), 6.74 (d, *J* = 7.5 Hz, 1H), 6.63 (d, *J* = 7.9 Hz, 1H), 6.17 (q, *J* = 7.1 Hz, 1H), 4.19 (s, 2H), 3.36 (s, 2H), 3.03 (d, *J* = 12.2 Hz, 1H), 2.97 (s, 2H), 2.70 (d, *J* = 12.2 Hz, 1H), 2.52-2.49 (m, 1H), 2.35-2.30 (m, 2H), 2.27-2.22 (m, 1H), 1.79-1.73 (m, 2H), 1.61-1.49 (m, 4H), 1.45 (s, 6H), 1.35-1.30 (m, 1H); ¹³C NMR (101 MHz, CDCl₃) *δ* (ppm) 165.76, 161.17 (d, ¹*J_{CF}* = 249.4 Hz), 158.83, 135.30, 129.82 (d, ³*J_{CF}* = 8.3 Hz), 128.99 (d, ³*J_{CF}* = 3.9 Hz), 127.83, 126.75, 126.14 (d, ²*J_{CF}* = 13.8 Hz), 124.04 (d, ⁴*J_{CF}* = 3.6 Hz), 120.85, 115.84 (d, ²*J_{CF}* = 21.8 Hz), 108.21, 86.50, 69.30, 62.54, 60.55, 49.21, 48.72, 48.63, 45.88 (d, ³*J_{CF} =* 1.8 Hz), 41.87, 32.51, 31.01, 28.29, 15.09; HRMS (ESI-Q-TOF): *m*/*z* [M+H]⁺ calcd. for C₂₇H₃₃FN₂O₃: 453.25478; found: 453.2546.

### The preparation of (S)-4-(1-(2,4-difluorophenyl)ethyl)-9-((2,2-dimethyl-2,3-dihydrobenzofuran-4-yl)methyl)-1-oxa-4,9-diazaspiro[5.5]undecan-3-one (compound 63c)

This compound was synthesized via general procedure 2 affording compound **63c** as a colorless oil, in 50% yield.

¹H NMR (400 MHz, CDCl₃): δ (ppm) 7.35-7.29 (m, 1H), 7.05 (t, *J* = 7.8 Hz, 1H), 6.91-6.87 (m, 1H), 6.84-6.79 (m, 1H), 6.74 (d, *J* = 7.5 Hz, 1H), 6.63 (d, *J* = 7.8 Hz, 1H), 6.10 (q, *J* = 7.1 Hz, 1H), 4.18 (s, 2H), 3.36 (s, 2H), 3.03 (d, *J* = 12.1 Hz, 1H), 2.97 (s, 2H), 2.69 (d, *J* = 12.1 Hz, 1H), 2.51-2.48 (m, 1H), 2.35-2.29 (m, 2H), 2.27-2.22 (m, 1H), 1.80-1.72 (m, 2H), 1.56-1.50 (m, 4H), 1.46 (s, 6H), 1.35-1.31 (m, 1H); ¹³C NMR (101 MHz, CDCl₃) *δ* (ppm) 165.85, 162.71 (dd, ¹*J_{CF}* = 250.2 Hz, ³*J_{CF}* = 12.5 Hz), 161.25 (dd, ¹*J_{CF}* = 252.3 Hz, ³*J_{CF} =* 11.9 Hz), 158.82, 135.35, 129.88 (dd, ²*J_{CF}* = 8.5 Hz, ⁴*J_{CF} =* 6.9 Hz), 127.81, 126.69, 122.31 (t, ³*J_{CF} =* 9.0 Hz), 120.79, 111.09 (dd, ²*J_{CF} =* 19.8 Hz, ⁴*J_{CF}* = 5.0 Hz), 108.16, 104.36 (t, ²*J_{CF}* = 25.4 Hz), 86.49, 69.33, 62.51, 60.61, 49.33, 48.69 *(d,* ³*J_{CF}* = 7.6 Hz), 45.64, 41.87, 32.53, 31.17, 28.29, 28.28, 15.18; HRMS (ESI-Q-TOF): *m*/*z* [M+H]⁺ calcd. for C₂₇H₃₂F₂N₂O₃: 471.24535; found: 471.2451.

The preparation of 1-(2-bromo-4-chlorophenyl)-2-methylpropan-2-ol (compound **66a**) An oven-dried screw cap test tube was added with a magnetic stir-bar, compound **64** (1500.0 mg, 5.69 mmol, 1.00 eq). The tube was then evacuated and back-filled with argon. The evacuation/backfill sequence was repeated with two additional times. The reaction tube was evacuated and backfilled with argon three times. Under counterflow of argon, anhydrous ether (6.0 mL) was added by syringe. Then, the reaction mixture was stirred at 0 ^{o}C for 5 minutes, followed by the addition of MeMgBr (5.7 mL, 3.0 M in ether) dropwise. After this, the reaction mixture was stirred at 0 ^{o}C for 2 h. Afterwards, a saturated aqueous ammonium chloride solution (1.5 mL) was added to quench the reaction. Then, it was concentrated under reduced pressure affording the crude product. The crude residue was purified by chromatography on silica gel (20% of ethyl acetate in petroleum ether) to give compound **66a** as a colorless oil (1223.0 mg, 82% yield). ¹H NMR (400 MHz, CDCl₃): δ (ppm) 7.58 (d, *J* = 2.2 Hz, 1H), 7.31 (d, *J* = 8.3 Hz, 1H), 7.24 (dd, *J* = 2.1, 8.3 Hz, 1H), 2.97 (s, 2H), 1.27 (s, 6H); ¹³C NMR (101 MHz, CDCl₃) *δ* (ppm) 136.29, 133.03, 132.91, 132.47, 127.31, 126.04, 71.77, 47.25, 29.49.

The preparation of 1-(2-bromo-4-fluorophenyl)-2-methylpropan-2-ol (compound **66b**) An oven-dried screw cap test tube was added with a magnetic stir-bar, Mg (108.9 mg, 4.48 mmol, 1.20 eq) and I₂ (189.5 mg, 0.75 mmol, 0.20 eq). The tube was then evacuated and back-filled with argon. The evacuation/backfill sequence was repeated with two additional times. The reaction tube was evacuated and backfilled with argon three times. Under counterflow of argon, anhydrous ether (4.0 mL) was added by syringe. Then, the reaction mixture was stirred at 0 ^{o}C for 5 minutes, followed by the addition of compound **65** (1.0 g, 3.73 mmol, 1.00 eq) dropwise. After this, the reaction mixture was stirred at room temperature overnight. Afterwards, acetone (260.1 mg, 4.48 mmol, 1.20 eq) was added and stirred at 0 ^{o}C for 1 h. A saturated aqueous ammonium chloride solution (1.2 mL) was added to quench the reaction. Then, it was concentrated under reduced pressure affording the crude product. The crude residue was purified by chromatography on silica gel (20% of ethyl acetate in petroleum ether) to give compound **66b** as a colorless oil (522.1 mg, 57% yield).

¹H NMR (400 MHz, CDCl₃): δ (ppm) 7.36-7.30 (m, 2H), 7.02-6.97 (m, 1H), 2.97 (s, 2H), 1.27 (s, 6H); ¹³C NMR (101 MHz, CDCl₃) *δ* (ppm) 161.03 (d, ¹*J_{CF}* = 249.4 Hz), 133.58 (d, ⁴*J_{CF}* = 3.5 Hz), 133.01 (d, ³*J_{CF} =* 8.1 Hz), 125.57 (d, ³*J_{CF}* = 9.2 Hz), 119.98 (d, ²*J_{CF} =* 24.0 Hz), 114.29 (d, ²*J_{CF} =* 20.6 Hz), 71.75, 47.03, 29.45.

### The preparation of 6-chloro-2,2-dimethyl-2,3-dihydrobenzofuran-4-carbaldehyde (compound 68a)

This compound was synthesized via general procedure 7 affording compound **68a** as a yellow oil, in 31% yield.

¹H NMR (400 MHz, CDCl₃): δ (ppm) 9.96 (s, 1H), 7.26 (d, *J* = 1.7 Hz, 1H), 6.95 (d, *J* = 1.7 Hz, 1H), 3.30 (s, 2H), 1.49 (s, 6H); ¹³C NMR (101 MHz, CDCl₃) *δ* (ppm) 191.29, 160.85, 134.19, 127.08, 125.80, 125.60, 123.29, 89.51, 42.11, 28.24.

### The preparation of 6-fluoro-2,2-dimethyl-2,3-dihydrobenzofuran-4-carbaldehyde (compound 68b)

This compound was synthesized via general procedure 7 affording compound **68b** as a yellow oil, in 36% yield.

¹H NMR (400 MHz, CDCl₃): δ (ppm) 9.96 (d, *J* = 0.5 Hz, 1H), 6.98 (dd, *J* = 2.3, 8.9 Hz, 1H), 6.69 (dd, *J* = 2.3, 8.9 Hz, 1H), 3.29 (d, *J* = 1.6 Hz, 2H), 1.50 (s, 6H); ¹³C NMR (101 MHz, CDCl₃) *δ* (ppm) 191.22 (d, ⁴*J_{CF} =* 2.6 Hz), 163.29 (d, ¹*J_{CF}* = 245.2 Hz), 161.14 (d, ³*J_{CF} =* 12.1 Hz), 132.94 (d, ³*J_{CF} =* 8.2 Hz), 124.26 (d, ⁴*J_{CF} =* 2.3 Hz), 109.22 (d, ²*J_{CF}* = 22.9 Hz), 103.26 (d, ²*J_{CF}* = 26.4 Hz), 89.73, 41.88, 28.26.

### The preparation of 2-(3,5-dibromopyridin-4-yl)propan-2-ol (compound 70)

An oven-dried screw cap test tube was added with a magnetic stir-bar, compound **69** (1254.6 mg, 5.00 mmol, 1.00 eq). The tube was then evacuated and back-filled with argon. The evacuation/backfill sequence was repeated with two additional times. The reaction tube was evacuated and backfilled with argon three times. Under counterflow of argon, anhydrous THF (10.0 mL) was added by syringe. Then, the reaction mixture was stirred at -40 ^{o}C for 5 minutes, followed by the addition of LDA (10.0 mL, 2.0 M) dropwise. After this, the reaction mixture was stirred at -40 ^{o}C for 30 minutes. Afterwards, acetone (348.5 mg, 6.00 mmol, 1.20 eq) was added and stirred at -40 ^{o}C for 2 h. A saturated aqueous ammonium chloride solution (1.5 mL) was added to quench the reaction. Then, it was concentrated under reduced pressure affording the crude product. The crude residue was purified by chromatography on silica gel (15% of ethyl acetate in petroleum ether) to give compound **70** as a yellow oil (310.0 mg, 20% yield). ¹H NMR (400 MHz, CDCl₃): δ (ppm) 8.62 (s, 2H), 3.31 (s, 2H), 1.39 (s, 6H).

### The preparation of 4-bromo-2,2-dimethyl-2,3-dihydrofuro[2,3-c]pyridine (compound 71)

To an oven-dried screw cap test tube equipped with a magnetic stir-bar was added compound **70** (300.0 mg, 0.97 mmol, 1.00 eq), CuI (18.5 mg, 0.10 mmol, 0.10 eq), 8-hydroxyquinoline (21.1 mg, 0.15 mmol, 0.15 eq) and Cs₂CO₃ (632.7 mg, 1.94 mmol, 2.00 eq). The tube was then evacuated and back-filled with argon. The reaction tube was evacuated and backfilled with argon three times. Under a counterflow of argon, anhydrous toluene (2.0 mL) was added via a syringe. After that, the reaction mixture was heated at 120 ^{o}C for 8 hours. Then, it was concentrated under reduced pressure affording the crude product. The crude residue was purified by chromatography on silica gel (20% of ethyl acetate in petroleum ether) to give compound **71** as a colorless oil (89.2 mg, 40% yield).

¹H NMR (400 MHz, CDCl₃): δ (ppm) 8.18 (s, 1H), 8.01 (s, 1H), 3.03 (s, 2H), 1.52 (s, 6H); ¹³C NMR (101 MHz, CDCl₃) *δ* (ppm) 156.15, 142.53, 137.28, 130.09, 117.33, 87.80, 43.51, 28.20.

### The preparation of 2,2-dimethyl-2,3-dihydrofuro[2,3-c]pyridine-4-carbaldehyde (compound 72)

This compound was synthesized via general procedure 7 affording compound **72** as a yellow oil, in 62% yield.

¹H NMR (400 MHz, CDCl₃): δ (ppm) 10.15 (s, 1H), 8.56 (s, 1H), 8.30 (s, 1H), 3.36 (s, 2H), 1.52 (s, 6H); ¹³C NMR (101 MHz, CDCl₃) *δ* (ppm) 191.46, 156.97, 145.35, 136.55, 136.13, 128.78, 89.69, 42.42, 28.21.

The preparation of (S)-9-((6-chloro-2,2-dimethyl-2,3-dihydrobenzofuran-4-yl)methyl)-4-(1-(2-fluorophenyl)ethyl)-1-oxa-4,9-diazaspiro[5.5]undecan-3-one (compound **73a**) This compound was synthesized via general procedure 9 affording compound **73a** as a colorless oil, in 16% yield.

¹H NMR (400 MHz, CDCl₃): δ (ppm) 7.36-7.29 (m, 2H), 7.18-7.14 (m, 1H), 7.08-7.03 (m, 1H), 6.76 (d, *J* = 1.7 Hz, 1H), 6.61 (d, *J* = 1.8 Hz, 1H), 6.17 (q, *J* = 7.1 Hz, 1H), 4.18 (s, 2H), 3.30 (s, 2H), 3.04 (d, *J* = 12.2 Hz, 1H), 2.90 (s, 2H), 2.70 (d, *J* = 12.2 Hz, 1H), 2.50-2.47 (m, 1H), 2.33-2.28 (m, 2H), 2.26-2.21 (m, 1H), 1.80-1.73 (m, 2H), 1.56-1.49 (m, 4H), 1.44 (s, 6H), 1.34-1.25 (m, 1H); ¹³C NMR (101 MHz, CDCl₃) *δ* (ppm) 165.72, 161.17 (d, ¹*J_{CF}* = 249.5 Hz), 159.56, 136.40, 133.16, 129.84 (d, ³*J_{CF}* = 8.3 Hz), 128.99 (d, ³*J_{CF} =* 4.1 Hz), 126.13 *(d,* ²*J_{CF} =* 13.8 Hz), 125.26, 124.06 (d, ⁴*J_{CF} =* 3.6 Hz), 120.45, 115.84 (d, ²*J_{CF}* = 21.9 Hz), 108.83, 87.80, 69.22, 62.52, 60.13, 49.24, 48.72, 48.66, 45.88 (d, ³*J_{CF} =* 1.9 Hz), 41.33, 32.51, 31.04, 28.21, 15.09; HRMS (ESI-Q-TOF): *m*/*z* [M+H]⁺ calcd. for C₂₇H₃₂ClFN₂O₃: 487.21580; found: 487.2161.

The preparation of (S)-9-((6-fluoro-2,2-dimethyl-2,3-dihydrobenzofuran-4-yl)methyl)-4-(1-(2-fluorophenyl)ethyl)-1-oxa-4,9-diazaspiro[5.5]undecan-3-one (compound **73b)** This compound was synthesized via general procedure 9 affording compound **73b** as a colorless oil, in 7% yield.

¹H NMR (400 MHz, CDCl₃): δ (ppm) 7.36-7.29 (m, 2H), 7.18-7.14 (m, 1H), 7.08-7.03 (m, 1H), 6.49 (dd, *J =* 2.2, 10.2 Hz, 1H), 6.33 (dd, *J =* 2.2, 9.3 Hz, 1H), 6.17 (q, *J* = 7.1 Hz, 1H), 4.19 (s, 2H), 3.30 (s, 2H), 3.04 (d, *J* = 12.2 Hz, 1H), 2.89 (s, 2H), 2.70 (d, *J* = 12.2 Hz, 1H), 2.50-2.48 (m, 1H), 2.33-2.28 (m, 2H), 2.26-2.21 (m, 1H), 1.80-1.70 (m, 2H), 1.61-1.48 (m, 4H), 1.45 (s, 6H), 1.34-1.27 (m, 1H); ¹³C NMR (101 MHz, CDCl₃) *δ* (ppm) 165.73, 163.24 (d, ¹*J_{CF}* = 242.3 Hz), 161.18 (d, ¹*J_{CF}* = 249.4 Hz), 159.66 (d, ³*J_{CF} =* 13.4 Hz), 136.26 (d, ³*J_{CF}* = 8.8 Hz), 129.84 (d, ³*J_{CF}* = 8.3 Hz), 129.00 (d, ³*J_{CF}* = 3.8 Hz), 126.14 *(d,* ²*J_{CF} =* 13.7 Hz), 124.05 (d, ⁴*J_{CF}* = 3.6 Hz), 121.81 (d, ⁴*J_{CF}* = 2.8 Hz), 115.84 (d, ²*J_{CF}* = 21.9 Hz), 106.78 (d, ²*J_{CF} =* 22.8 Hz), 96.48 (d, ²*J_{CF}* = 26.2 Hz), 88.03, 69.24, 62.53, 60.18, 49.27, 48.72, 48.66, 45.89 (d, ³*J_{CF}* = 2.4 Hz), 41.17, 32.56, 31.05, 28.24, 15.09; ¹⁹F NMR (377 MHz, CDCl₃) *δ* (ppm) -114.87 (m, 1F), -114.40 (m, 1F); HRMS (ESI-Q-TOF): *m*/*z* [M+H]⁺ calcd. for C₂₇H₃₂F₂N₂O₃: 471.24535; found: 471.2451.

The preparation of (*S*)-9-((2,2-dimethyl-2,3-dihydrofuro[2,3-c]pyridin-4-yl)methyl)-4-(1-(2-fluorophenyl)ethyl)-1-oxa-4,9-diazaspiro[5.5]undecan-3-one (compound **73c)** This compound was synthesized via general procedure 9 affording compound **73c** as a colorless oil, in 17% yield.

¹H NMR (400 MHz, CDCl₃): δ (ppm) 8.00 (s, 2H), 7.37-7.29 (m, 2H), 7.18-7.14 (m, 1H), 7.08-7.03 (m, 1H), 6.17 (q, *J* = 7.1 Hz, 1H), 4.18 (s, 2H), 3.39 (s, 2H), 3.04 (d, *J =* 12.2 Hz, 1H), 2.98 (s, 2H), 2.71 (d, *J* = 12.2 Hz, 1H), 2.48-2.45 (m, 1H), 2.36-2.22 (m, 3H), 1.80-1.71 (m, 2H), 1.55-1.50 (m, 4H), 1.48(s, 6H), 1.33-1.26 (m, 1H); ¹³C NMR (101 MHz, CDCl₃) *δ* (ppm) 165.71, 161.17 (d, ¹*J_{CF}* = 249.4 Hz), 155.97, 142.26, 135.95, 130.90, 130.52, 129.85 (d, ³*J_{CF}* = 8.5 Hz), 129.01 (d, ³*J_{CF}* = 4.1 Hz), 126.13 (d, ²*J_{CF} =* 13.8 Hz), 124.06 (d, ⁴*J_{CF}* = 3.6 Hz), 115.84 (d, ²*J_{CF}* = 21.9 Hz), 87.76, 69.23, 62.53, 58.10, 49.16, 48.80, 48.68, 45.90 (d, ³*J_{CF} = 1.8* Hz), 41.45, 32.57, 31.08, 28.14, 15.09; HRMS (ESI-Q-TOF): *m*/*z* [M+H]⁺ calcd. for C₂₆H₃₂FN₃O₃: 454.25002; found: 454.2496.

### The preparation of ethyl 4-bromobenzofuran-2-carboxylate (compound 75)

Compound **75** was synthesized based on the reported procedure. Compound **74** (1.81 g, 9.00 mmol, 1.00 eq) and K2CO3 (4.98 g, 36.0 mmol, 4.00 eq) were added into DMF (10.0 mL). Then, the reaction mixture was stirred at 0 ^{o}C for 5 minutes before adding ethyl bromoacetate (1.65 g, 9.90 mmol, 1.10 eq) dropwise. After 20 minutes, the reaction mixture was stirred at 120 ^{o}C for further 10 h. Then, it was washed with brine (50.0 mL) and extracted with ethyl acetate (150.0 mL). The organic layer was collected and dried over magnesium sulfate. Then, it was concentrated under reduced pressure affording the compound **75** as a white solid (1.34g, 55% yield). The spectral data of target compound **75** were consistent with previous record[12].

### The preparation of 4-bromo-2-(chloromethyl)benzofuran (compound 76)

This compound was synthesized via general procedure 8 affording compound **76** as a white solid, in 63% yield.

¹H NMR (400 MHz, CDCl₃): δ (ppm) 7.44-7.42 (m, 1H), 7.41-7.38 (m, 1H), 7.18 (t, *J* = 8.1 Hz, 1H), 6.80 (s, 1H), 4.69 (s, 2H); ¹³C NMR (101 MHz, CDCl₃) *δ* (ppm) 154.96, 153.14, 129.49, 126.11, 125.99, 114.23, 110.51, 106.33, 37.39.

### The preparation of 4-bromo-2-methylene-2,3-dihydrobenzofuran (compound 77)

A LiAlH₄ solution (1.0 M in THF, 3.80 mL) was added dropwise to a suspension of CrCl₃ (1.20 g, 7.60 mmol, 2.50 eq) in THF (8.0 mL) at 0 °C and the reaction mixture was stirred at the same temperature for 15 minutes. Then, anhydrous DMF (12.0 mL) and isopropanol (1.30 mL) were added, followed by the dropwise addition of a solution of compound **76** (746.0 mg, 3.04 mmol, 1.00 eq) in DMF (3.0 mL) at 0 °C. Afterwards, the reaction mixture was stirred at room temperature for 18 hours. It was washed with brine (20.0 mL) and extracted with petroleum ether (40.0 mL). The organic layer was collected and dried over magnesium sulfate. Then, it was concentrated under reduced pressure affording the crude product. The crude residue was purified by chromatography on silica gel (100% petroleum ether) to give compound **77** as a colorless oil (370.2 mg, 58% yield).

¹H NMR (400 MHz, CDCl₃): δ (ppm) 7.09-7.02 (m, 2H), 6.81-6.79 (m, 1H), 4.71 (q, *J* = 2.5 Hz, 1H), 4.34 (q, *J* = 2.5 Hz, 1H), 3.87 (t, *J* = 2.5 Hz, 2H); ¹³C NMR (101 MHz, CDCl₃) *δ* (ppm) 159.31, 158.60, 129.58, 127.15, 124.74, 118.61, 108.10, 85.87, 34.40.

### The preparation of 4-bromo-3H-spiro[benzofuran-2,1'-cyclopropane] (compound 78)

A diethylzinc solution (1.1 M in toluene, 3.90 mL) was added dropwise to a solution of compound **77** (360.0 g, 1.71 mmol, 1.00 eq) in anhydrous DCE (7.0 mL) at 0 °C, followed by chloroiodomethane (1504.3 mg, 8.53 mmol, 5.00 eq). Afterwards, the reaction mixture was stirred at 0 °C for 1 hour and at 60 °C for 2.5 hours. A saturated aqueous ammonium chloride solution (5.0 mL) was added. Then, it was washed with brine (20.0 mL) and extracted with DCM (40.0 mL). The organic layer was collected and dried over magnesium sulfate. Then, it was concentrated under reduced pressure affording the crude product. The crude residue was purified by chromatography on silica gel (100% petroleum ether) to give compound **78** as a white solid (125.2 mg, 33% yield).

¹H NMR (400 MHz, CDCl₃): δ (ppm) 7.02-6.97 (m, 2H), 6.67 (dd, *J* = 2.1, 6.7 Hz, 1H), 3.31 (s, 1H), 1.21-1.17 (m, 2H), 0.76-0.73 (m, 2H); ¹³C NMR (101 MHz, CDCl₃) *δ* (ppm) 160.05, 129.30, 128.19, 123.39, 118.99, 108.19, 67.26, 37.79, 12.48.

### The preparation of 3H-spiro[benzofuran-2,1'-cyclopropane]-4-carbaldehyde (compound 79)

This compound was synthesized via general procedure 7 affording compound **79** as a colorless oil, in 77% yield.

¹H NMR (400 MHz, CDCl₃): δ (ppm) 10.05 (s, 1H), 7.35-7.30 (m, 2H), 6.99 (dd, *J* = 2.3, 6.7 Hz, 1H), 3.64 (s, 1H), 1.23-1.19 (m, 2H), 0.78-0.75 (m, 2H); ¹³C NMR (101 MHz, CDCl₃) *δ* (ppm) 192.57, 160.81, 132.76, 128.48, 127.83, 124.53, 114.78, 68.78, 36.32, 12.49.

### The preparation of 4-(chloromethyl)-3H-spiro[benzofuran-2,1'-cyclopropane] (compound 80)

This compound was synthesized via general procedure 8 affording compound **80** as a yellow solid, in 91% yield.

¹H NMR (400 MHz, CDCl₃): δ (ppm) 7.12 (t, *J* = 7.8 Hz, 1H), 6.87 (d, *J* = 7.6 Hz, 1H), 6.73 (d, *J* = 8.1 Hz, 1H), 4.52 (s, 2H), 3.38 (s, 2H), 1.22-1.19 (m, 2H), 0.76-0.73 (m, 2H); ¹³C NMR (101 MHz, CDCl₃) *δ* (ppm) 160.05, 133.68, 128.39, 126.38, 120.90, 109.73, 67.69, 44.18, 34.88, 12.54.

### The preparation of (S)-9-((3H-spiro[benzofuran-2,1'-cyclopropan]-4-yl)methyl)-4-(1-(2-fluorophenyl)ethyl)-1-oxa-4,9-diazaspiro[5.5]undecan-3-one (compound 81)

This compound was synthesized via general procedure 2 affording compound **81** as a colorless oil, in 74% yield.

¹H NMR (400 MHz, CDCl₃): δ (ppm) 7.36-7.28 (m, 2H), 7.17-7.13 (m, 1H), 7.07-7.02 (m, 2H), 6.78 (d, *J* = 7.6 Hz, 1H), 6.64 (d, *J* = 7.9 Hz, 1H), 6.17 (q, *J* = 7.1 Hz, 1H), 4.18 (s, 2H), 3.38 (s, 2H), 3.27 (s, 2H), 3.03 (d, *J* = 12.2 Hz, 1H), 2.69 (d, *J* = 12.2 Hz, 1H), 2.51-2.48 (m, 1H), 2.34-2.29 (m, 2H), 2.27-2.21 (m, 1H), 1.79-1.72 (m, 2H), 1.55-1.48 (m, 4H), 1.18-1.50 (m, 2H), 0.71-0.68 (m, 2H); ¹³C NMR (101 MHz, CDCl₃) *δ* (ppm) 165.76, 161.16 (d, ¹*J_{CF}* = 249.4 Hz), 159.63, 135.06, 129.81 (d, ³*J_{CF} =* 8.5 Hz), 128.98 (d, ³*J_{CF} =* 4.1 Hz), 127.68, 126.44, 126.15 (d, ²*J_{CF} =* 13.8 Hz), 124.03 (d, ⁴*J_{CF}* = 3.6 Hz), 121.30, 115.83 (d, ²*J_{CF} =* 21.8 Hz), 107.99, 69.31, 67.41, 62.52, 60.66, 49.21, 48.78, 48.69, 45.86 (d, ³*J_{CF} =* 1.8 Hz), 35.53, 32.59, 31.04, 15.08, 12.52; HRMS (ESI-Q-TOF): *m*/*z* [M+H]⁺ calcd. for C₂₇H₃₁FN₂O₃: 451.23913; found: 451.2383.

### CCR8 calcium mobilization assay

Human glioblastoma U87 cells that stably express human CCR8 (U87.CD4.CCR8) were used to measure CCR8-dependent Ca²⁺ responses. Cells (20,000 cells/well) were seeded in gelatin-coated black-walled polystyrene 96-well plates and incubated overnight at 37°C and 5% CO₂. The next day, conditioned culture medium was removed and replaced with a fluorescent calcium indicator (calcium 6, Molecular Devices; 80µL/well) after which plates were incubated for 2h at 37°C in the dark. Transient intracellular Ca²⁺ responses were recorded based on calcium 6 fluorescence using the FLIPR (Fluorescent and Luminescent Imaging Plate Reader, Molecular Devices). During the protocol calcium-6 loaded cells were challenged with serial dilutions of test compound or a fixed concentration of recombinant CCL1 (50ng/mL) to evaluate their agonist activity. For each well the fluorescent readout was normalized by dividing over a baseline value obtained immediately before compound or CCL1 addition. Also background responses from cells without compound or CCL1 stimulation (vehicle control) were subtracted from all other responses. For each assay plate the CCL1-induced response (at 50ng/mL) was set as 100% and all other responses were calculated relative to this. For the most active compounds (i.e., >90% activity at 50 µM) EC₅₀ values were calculated based on dose-dependent activation curves using non-linear regression in Graphpad.

Certain compounds of the accompanying examples were found to generate EC₅₀ values in this assay of 10 µM or better. Most of these compounds generated values of less than 1 µM. The majority of these compounds generated values of even less than 0.1 µM.

### References

[1] T. Šumanovac, M. Alešković, M. Šekutor, M. Matković, T. Baron, K. Mlinarić-Majerski, C. Bohne, N. Basarić, Photoelimination of nitrogen from adamantane and pentacycloundecane (PCU) diazirines: A spectroscopic study and supramolecular control, Photochem. Photobiol. Sci. 18 (2019) 1806-1822. https://doi.org/10.1039/c9pp00124g.
[2] A. V. Shevtsov, V. V. Kuznetsov, S.I. Molotov, K.A. Lyssenko, N.N. Makhova, Synthesis of 4-aroyl-1,2,4-triazolidin-3-ones via ring extension in reactions of 1,2-di- and 1,2,3,3-tetraalkyldiaziridines with aroyl isocyanates, Russ. Chem. Bull. 55 (2006) 554-558. https://doi.org/10.1007/s11172-006-0291-2.
[3] M. Garcia, M. Virgili, M. Alonso, C. Alegret, J. Farran, B. Fernández, M. Bordas, R. Pascual, J. Burgueño, A. Vidal-Torres, A.R. Fernández De Henestrosa, E. Ayet, M. Merlos, J.M. Vela, C.R. Plata-Salamán, C. Almansa, Discovery of EST73502, a Dual µ-Opioid Receptor Agonist and σ1Receptor Antagonist Clinical Candidate for the Treatment of Pain, J. Med. Chem. 63 (2020) 15508-15526. https://doi.org/10.1021/acs.jmedchem.0c01127.
[4] K. Geoghegan, J.W. Bode, Bespoke SnAP reagents for the synthesis of c-substituted spirocyclic and bicyclic saturated N-heterocycles, Org. Lett. 17 (2015) 1934-1937. https://doi.org/10.1021/acs.orglett.5b00618.
[5] M. Garcia, M. Virgili, M. Alonso, C. Alegret, B. Fernández, A. Port, R. Pascual, X. Monroy, A. Vidal-Torres, M.T. Serafini, J.M. Vela, C. Almansa, 4-Aryl-1-oxa-4,9-diazaspiro[5.5]undecane Derivatives as Dual µ-Opioid Receptor Agonists and σ1 Receptor Antagonists for the Treatment of Pain, J. Med. Chem. 63 (2020) 2434-2454. https://doi.org/10.1021/acs.jmedchem.9b01256.
[6] Z. Guan, S. Chen, Y. Huang, H. Yao, Rhodium(III)-Catalyzed Intramolecular Olefin Hydroarylation of Aromatic Aldehydes Using a Transient Directing Group, Org. Lett. 21 (2019) 3959-3962. https://doi.org/10.1021/acs.orglett.9b01101.
[7] X. Wang, Y. Lu, H.X. Dai, J.Q. Yu, Pd(II)-catalyzed hydroxyl-directed C-H activation/C-O cyclization: Expedient construction of dihydrobenzofurans, J. Am. Chem. Soc. 132 (2010) 12203-12205. https://doi.org/10.1021/ja105366u.
[8] K.M. Kim, E.K. Ryu, Unexpected iodine catalyzed cyclization for synthesis of 2,2-dialkyl-2,3-dihydrobenzofurans, Heterocycles 41 (1995) 219-223. https://doi.org/10.3987/com-94-6937.
[9] D. Leibig, A.H.E. Müller, H. Frey, Anionic Polymerization of Vinylcatechol Derivatives: Reversal of the Monomer Gradient Directed by the Position of the Catechol Moiety in the Copolymerization with Styrene, Macromolecules 49 (2016) 4792-4801. https://doi.org/10.1021/acs.macromol.6b00831.
[10] W. Xu, J. Ma, X. Yuan, J. Dai, J. Xie, C. Zhu, Synergistic Catalysis for the Umpolung Trifluoromethylthiolation of Tertiary Ethers, Angew. Chemie 130 (2018) 10514-10518. https://doi.org/10.1002/ange.201805927.
[11] J.C. Hood, M. V. Anokhin, D.A. Klumpp, Friedel-Crafts Reactions with N-Heterocyclic Alcohols, J. Org. Chem. 88 (2023) 10483-10493. https://doi.org/10.1021/acs.joc.3c00403.
[12] Y.Y. Shao, Y. Yin, B.P. Lian, J.F. Leng, Y.Z. Xia, L.Y. Kong, Synthesis and biological evaluation of novel shikonin-benzo[b]furan derivatives as tubulin polymerization inhibitors targeting the colchicine binding site, Eur. J. Med. Chem. 190 (2020).
[13] S. Cuisiat, N. Bourdiol, V. Lacharme, A. Newman-Tancredi, F. Colpaert, B. Vacher, Towards a new generation of potential antipsychotic agents combining D 2 and 5-HT1A receptor activities, J. Med. Chem. 50 (2007) 865-876. https://doi.org/10.1021/jm061180b.

## Claims

1. A compound of formula **I:** 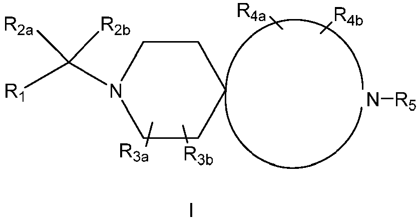
wherein R₁ is selected from: 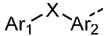 or Ar₃--;
Ar₁ and Ar₂ are heteroaryl and aryl groups, wherein said heteroaryl or aryl groups are optionally substituted with one or more substituents selected from the group consisting of halogen, C1-7 alkyl, C2-7 alkenyl, C2-7 alkynyl, halo C1-7 alkyl, nitro, hydroxyl, sulfhydryl, amino, C1-7 alkoxy,
X is O, NH, S, or CH₂;
Ar₃ is a heteroaryl or aryl group, wherein said heteroaryl or aryl group is optionally substituted with one or more substituents selected from the group consisting of halogen, C1-7 alkyl, C1-7 alkoxy, halo C1-7 alkyl, nitro, hydroxyl, amino, and
Ar₃ is optionally fused with a saturated or partly unsaturated heterocyclic ring with at least one oxygen in said heterocyclic ring; wherein said heterocyclic ring is optionally substituted with one or more substituents selected from the group consisting of halogen, C1-7 alkyl and is optionally spirofused with a C3-10 cycloalkyl;
R₂ₐ and R_{2b} are independently selected from H and C1-7 alkyl;
R₃ₐ and R_{3b} are independently selected from the group consisting of hydrogen, C1-7 alkyl and a carbonyl group; 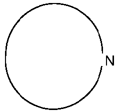
schematically represents a saturated or partly unsaturated heterocyclic ring with at least one nitrogen in said heterocyclic ring; optionally, this heterocyclic ring might also contain one or more oxygen atoms;
R₄ₐ and R_{4b} are independently selected from the group consisting of hydrogen, C1-7 alkyl and a carbonyl group;
R₅ is selected from the group consisting of H, C1-7 alkyl and (CR₇ₐR_{7b})ₙ-Ar₄;
wherein n is 0-2;
wherein R₇ₐ and R_{7b} are independently selected from H and C1-7 alkyl;
wherein Ar₄ is a heteroaryl and aryl group, and wherein said heteroaryl or aryl groups are optionally substituted with one or more substituents selected from the group consisting of halogen, C1-7 alkyl, , halo C1-7 alkyl, , cyano, , C1-7 alkoxy, cyano,;
and/or a pharmaceutical acceptable addition salt thereof and/or a stereoisomer thereof and/or a solvate thereof.

2. The compound according to claim 1,
- wherein Ar₁ and Ar₂ are heteroaryl and aryl groups, wherein said heteroaryl or aryl groups are optionally substituted with one or more substituents selected from the group consisting of halogen and C1-7 alkoxy,
- wherein X is O;
- wherein Ar₃ is a heteroaryl or aryl group, wherein said heteroaryl or aryl group is optionally substituted with one or more substituents selected from the group consisting of halogen and C1-7 alkoxy,
- R₅ is selected from the group consisting of H, C1-7 alkyl and (CR₇ₐR_{7b})ₙ-Ar₄;
wherein n is 0-2;
wherein R₇ₐ and R_{7b} are independently selected from H and C1-7 alkyl;
wherein Ar₄ is a heteroaryl and aryl group, and wherein said heteroaryl or aryl groups are optionally substituted with one or more halogen or cyano substituents.

3. The compound according to claim 1 or 2, wherein Ar₁, Ar₂, Ar₃ and Ar₄ are each independently selected from the group consisting of an unsubstituted heteroaryl and an aryl group, wherein said aryl group is optionally substituted with one or more substituents selected from the group consisting of halogen and C1-7 alkoxy.

4. The compound according to any one of claims 1 to 3, wherein X is O.

5. The compound according to any of claims 1 to 4, wherein R₂ₐ and R_{2b} are both H.

6. The compound according to any of claims 1 to 5, wherein R₄ₐ and R_{4b} are independently selected from the group consisting of hydrogen, a methyl and a carbonyl group.

7. The compound according to any of claims 1 to6, wherein R₄ₐ is hydrogen and R_{4b} is a carbonyl group.

8. The compound according to any of claims 1 to 7, wherein 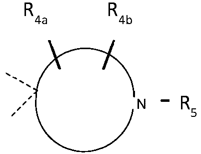 is preferentially

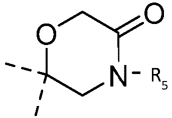

9. The compound according to any of claims 1 to 8, wherein R₅ is (CR₇ₐR_{7b})ₙ-Ar₄, wherein n is 1 or 2, R₇ₐ is hydrogen and R_{7b} is methyl and in which Ar₄ is a phenyl group substituted with a halogen.

10. The compound according to any of claims 1 to 9, wherein R₅ is (CR₇ₐR_{7b})ₙ-Ar₄, wherein n is 1, R₇ₐ is hydrogen and R_{7b} is methyl and in which Ar₄ is a phenyl group substituted with a halogen.

11. A compound according to any one of the claims 1 to 10, for use as a medicament.

12. A compound according to any one of the claims 1 to 10, for use in the treatment or prevention of a CCR8 mediated disease.

13. The compound for use according to claim12 wherein the CCR8 mediated disease is an autoimmune disease such as graft-versus-host disease (GVHD), multiple sclerosis, inflammatory bowel disease, Crohn's disease, rheumatoid arthritis, psoriasis and diabetes.

14. The compound for use to any of claims 11 to 13, wherein the compound is selected from the group consisting of:
3-benzyl-8-(3-(2-methoxyphenoxy)benzyl)-1-oxa-3,8-diazaspiro[4.5]decan-2-one 8-(3-(2-methoxyphenoxy)benzyl)-3-phenethyl-1-oxa-3,8-diazaspiro[4.5]decan-2-one 3-benzyl-8-(3-(2-methoxyphenoxy)benzyl)-1,3,8-triazaspiro[4.5]decane-2,4-dione 8-(3-(2-methoxyphenoxy)benzyl)-3-phenethyl-1,3,8-triazaspiro[4.5]decane-2,4-dione 1-benzyl-8-(3-(2-methoxyphenoxy)benzyl)-1,8-diazaspiro[4.5]decan-2-one
8-(3-(2-methoxyphenoxy)benzyl)-1-phenethyl-1,8-diazaspiro[4.5]decan-2-one 2-benzyl-8-(3-(2-methoxyphenoxy)benzyl)-2,8-diazaspiro[4.5]decan-1-one 8-(3-(2-methoxyphenoxy)benzyl)-2-phenethyl-2,8-diazaspiro[4.5]decan-1-one 1-benzyl-8-(3-(2-methoxyphenoxy)benzyl)-1,8-diazaspiro[4.5]decane 8-(3-(2-methoxyphenoxy)benzyl)-1-phenethyl-1,8-diazaspiro[4.5]decane 2-benzyl-8-(3-(2-methoxyphenoxy)benzyl)-2,8-diazaspiro[4.5]decane 8-(3-(2-methoxyphenoxy)benzyl)-2-phenethyl-2,8-diazaspiro[4.5]decane 8-(3-(2-methoxyphenoxy)benzyl)-4-phenyl-1,4,8-triazaspiro[4.5]decan-2-one 4-benzyl-8-(3-(2-methoxyphenoxy)benzyl)-1,4,8-triazaspiro[4.5]decan-2-one 8-(3-(2-methoxyphenoxy)benzyl)-4-phenethyl-1,4,8-triazaspiro[4.5]decan-2-one 8-(3-(2-methoxyphenoxy)benzyl)-4-phenyl-1,2,4,8-tetraazaspiro[4.5]decan-3-one 4-benzyl-8-(3-(2-methoxyphenoxy)benzyl)-1,2,4,8-tetraazaspiro[4.5]decan-3-one 1-benzyl-8-(2-isobutoxybenzyl)-1,8-diazaspiro[4.5]decan-2-one
4-benzyl-9-(3-(2-methoxyphenoxy)benzyl)-1-oxa-4,9-diazaspiro[5.5]undecan-3-one 9-(3-(2-methoxyphenoxy)benzyl)-4-phenethyl-1-oxa-4,9-diazaspiro[5.5]undecan-3-one
4-benzyl-9-(3-(2-methoxyphenoxy)benzyl)-1-oxa-4,9-diazaspiro[5.5]undecane 9-(3-(2-methoxyphenoxy)benzyl)-4-phenethyl-1-oxa-4,9-diazaspiro[5.5]undecane 4-(4-fluorobenzyl)-9-(3-(2-methoxyphenoxy)benzyl)-1-oxa-4,9-diazaspiro[5.5] undecan-3-one
4-(3-fluorobenzyl)-9-(3-(2-methoxyphenoxy)benzyl)-1-oxa-4,9-diazaspiro[5.5] undecan-3-one
4-(2-fluorobenzyl)-9-(3-(2-methoxyphenoxy)benzyl)-1-oxa-4,9-diazaspiro[5.5] undecan-3-one
4-(4-chlorobenzyl)-9-(3-(2-methoxyphenoxy)benzyl)-1-oxa-4,9-diazaspiro[5.5] undecan-3-one
4-(3-chlorobenzyl)-9-(3-(2-methoxyphenoxy)benzyl)-1-oxa-4,9-diazaspiro[5.5] undecan-3-one
4-(2-chlorobenzyl)-9-(3-(2-methoxyphenoxy)benzyl)-1-oxa-4,9-diazaspiro[5.5] undecan-3-one
4-((9-(3-(2-methoxyphenoxy)benzyl)-3-oxo-1-oxa-4,9-diazaspiro[5.5]undecan-4-yl)methyl)benzonitrile
3-((9-(3-(2-methoxyphenoxy)benzyl)-3-oxo-1-oxa-4,9-diazaspiro[5.5]undecan-4-yl)methyl)benzonitrile
2-((9-(3-(2-methoxyphenoxy)benzyl)-3-oxo-1-oxa-4,9-diazaspiro[5.5]undecan-4-yl)methyl)benzonitrile
9-(3-(2-methoxyphenoxy)benzyl)-4-(pyridin-2-ylmethyl)-1-oxa-4,9-diazaspiro[5.5] undecan-3-one
1-benzyl-9-(3-(2-methoxyphenoxy)benzyl)-4-oxa-1,9-diazaspiro[5.5]undecan-2-one 4-benzyl-9-(3-(2-methoxyphenoxy)benzyl)-2-methyl-1-oxa-4,9-diazaspiro[5.5] undecan-3-one
4-(2-fluorophenyl)-9-(3-(2-methoxyphenoxy)benzyl)-1-oxa-4,9-diazaspiro[5.5] undecan-3-one
(*S*)-4-(1-(2-fluorophenyl)ethyl)-9-(3-(2-methoxyphenoxy)benzyl)-1-oxa-4,9-diazaspiro[5.5]undecan-3-one
*(R)-4-(1-(2-fluorophenyl)ethyl)-9-(3-(2-methoxyphenoxy)benzyl)-1-oxa-4,9-*diazaspiro[5.5]undecan-3-one
4-benzyl-9-((3,3-dimethyl-2,3-dihydrobenzofuran-4-yl)methyl)-1-oxa-4,9-diazaspiro[5.5]undecan-3-one
4-benzyl-9-((2,2-dimethyl-2,3-dihydrobenzofuran-4-yl)methyl)-1-oxa-4,9-diazaspiro[5.5]undecan-3-one
4-benzyl-9-((2,2-dimethyl-2,3-dihydrobenzofuran-7-yl)methyl)-1-oxa-4,9-diazaspiro[5.5]undecan-3-one
4-benzyl-9-((2,2-dimethyl-2*H*-chromen-8-yl)methyl)-1-oxa-4,9-diazaspiro[5.5] undecan-3-one
9-(benzofuran-7-ylmethyl)-4-benzyl-1-oxa-4,9-diazaspiro[5.5]undecan-3-one 4-benzyl-9-((2,3-dihydrobenzofuran-7-yl)methyl)-1-oxa-4,9-diazaspiro[5.5]undecan-3-one
4-benzyl-9-(chroman-8-ylmethyl)-1-oxa-4,9-diazaspiro[5.5]undecan-3-one 9-(benzo[d][1,3]dioxol-4-ylmethyl)-4-benzyl-1-oxa-4,9-diazaspiro[5.5]undecan-3-one 4-benzyl-9-((2,3-dihydrobenzo[b][1,4]dioxin-5-yl)methyl)-1-oxa-4,9-diazaspiro[5.5]undecan-3-one
4-benzyl-9-((3,4-dihydro-2*H*-benzo[b][1,4]dioxepin-6-yl)methyl)-1-oxa-4,9-diazaspiro[5.5]undecan-3-one
4-benzyl-9-((2,2-dimethylbenzo[d][1,3]dioxol-4-yl)methyl)-1-oxa-4,9-diazaspiro[5.5]undecan-3-one
4-benzyl-9-((2,2-difluorobenzo[d][1,3]dioxol-4-yl)methyl)-1-oxa-4,9-diazaspiro[5.5]undecan-3-one
9-((2,2-dimethyl-2,3-dihydrobenzofuran-4-yl)methyl)-4-(2-fluorophenyl)-1-oxa-4,9-diazaspiro[5.5]undecan-3-one
9-((2,2-dimethyl-2,3-dihydrobenzofuran-4-yl)methyl)-4-(2-fluorobenzyl)-1-oxa-4,9-diazaspiro[5.5]undecan-3-one
9-((2,2-dimethyl-2,3-dihydrobenzofuran-4-yl)methyl)-4-(pyridin-2-ylmethyl)-1-oxa-4,9-diazaspiro[5.5]undecan-3-one
(*R*)-9-((2,2-dimethyl-2,3-dihydrobenzofuran-4-yl)methyl)-4-(1-(2-fluorophenyl)ethyl)-1-oxa-4,9-diazaspiro[5.5]undecan-3-one
(*S*)-9-((2,2-dimethyl-2,3-dihydrobenzofuran-4-yl)methyl)-4-(1-(2-fluorophenyl)ethyl)-1-oxa-4,9-diazaspiro[5.5]undecan-3-one
(*S*)-4-(1-(2,4-difluorophenyl)ethyl)-9-((2,2-dimethyl-2,3-dihydrobenzofuran-4-yl)methyl)-1-oxa-4,9-diazaspiro[5.5]undecan-3-one
(*S*)-9-((6-chloro-2,2-dimethyl-2,3-dihydrobenzofuran-4-yl)methyl)-4-(1-(2-fluorophenyl)ethyl)-1-oxa-4,9-diazaspiro[5.5]undecan-3-one
(*S*)-9-((6-fluoro-2,2-dimethyl-2,3-dihydrobenzofuran-4-yl)methyl)-4-(1-(2-fluorophenyl)ethyl)-1-oxa-4,9-diazaspiro[5.5]undecan-3-one
(*S*)-9-((2,2-dimethyl-2,3-dihydrofuro[2,3-c]pyridin-4-yl)methyl)-4-(1-(2-fluorophenyl)ethyl)-1-oxa-4,9-diazaspiro[5.5]undecan-3-one
(*S*)-9-((3*H*-spiro[benzofuran-2,1'-cyclopropan]-4-yl)methyl)-4-(1-(2-fluorophenyl)ethyl)-1-oxa-4,9-diazaspiro[5.5]undecan-3-one

15. A pharmaceutical composition comprising a compound according to any one of the claims 1 to 10, and a pharmaceutically acceptable carrier.
